# EUROPEAN PATENT SPECIFICATION

(11) **EP 3 003 356 B1**
(45) Date of publication and mention of the grant of the patent: **03.07.2019**
(21) Application number: 14733888.3
(22) Date of filing: 30.05.2014
(51) Int. Cl.: A61K 38/20, A61P 25/28

(54) **IL-1 ANTAGONISTS FOR USE IN TREATING ALZHEIMER'S DISEASE**
IL-1-ANTAGONISTEN ZUR VERWENDUNG IN DER BEHANDLUNG VON MORBUS ALZHEIMER
ANTAGONISTES DE L'IL-1 POUR L'ULTILISATION DANS LE TRAITEMENT DE LA MALADIE D'ALZHEIMER

(30) Priority: 31.05.2013 US 201361829713 P
(43) Date of publication of application: 13.04.2016
(73) Proprietor: Regeneron Pharmaceuticals, Inc., Tarrytown, NY 10591 (US)
(72) Inventor: CROLL, Susan, D., Putnam Valley NY-10579 (US); WIEGAND, Stanley, Hopewell Junction, NY 12533 (US)
(74) Representative: J A Kemp
(86) International application number: PCT/US2014/040160
(87) International publication number: WO 2014/194166

(56) References cited:
- US-A1- 2002 131 954
- US-A1- 2003 143 697
- US-A1- 2006 051 381
- US-A1- 2009 156 492
- MITROULIS I ET AL: "Targeting IL-1beta in disease; the expanding role of NLRP3 inflammasome", EUROPEAN JOURNAL OF INTERNAL MEDICINE, ELSEVIER, AMSTERDAM, NL, vol. 21, no. 3, 1 June 2010 (2010-06-01), pages 157-163, XP027053775, ISSN: 0953-6205 [retrieved on 2010-05-18]
- HAL M. HOFFMAN ET AL: "Efficacy and safety of rilonacept (interleukin-1 trap) in patients with cryopyrin-associated periodic syndromes: Results from two sequential placebo-controlled studies", ARTHRITIS & RHEUMATISM, vol. 58, no. 8, 30 July 2008 (2008-07-30), pages 2443-2452, XP055014926, ISSN: 0004-3591, DOI: 10.1002/art.23687
- DANIELA GALIMBERTI ET AL: "Progress in Alzheimerâ s disease", JOURNAL OF NEUROLOGY, STEINKOPFF-VERLAG, DA, vol. 259, no. 2, 25 June 2011 (2011-06-25) , pages 201-211, XP035009898, ISSN: 1432-1459, DOI: 10.1007/S00415-011-6145-3

## Description

### FIELD OF THE INVENTION

The invention relates to an interleukin-1 (IL-1) antagonist for use in methods to treat or to slow the progression of a disease characterized in part by beta amyloid (Aβ) expression or activity, or by aberrant deposition of beta amyloid in a subject, such as in Alzheimer's disease, and more specifically, the pathologies associated with such a disease, including for example, behavioral changes or cognitive dysfunction associated with Alzheimer's disease.

### STATEMENT OF RELATED ART

The proinflammatory cytokine interleukin-1 (IL-1) is an important player in inflammatory processes throughout the body, including in the central nervous system (CNS). IL-1 is found in two distinct isoforms, IL-1α and IL-1β, although IL-1β is considered the primary active isoform. Upregulation of IL-1β is part of the response to a range of CNS insults, including infections, stroke, and traumatic injuries (Allan, S. M., Tyrrell, P. J., & Rothwell, N. J. (2005), Nature Reviews Immunology, 5, 629-640). This neuroinflammatory response is characterized by activation of resident glial cells (microglia and astrocytes), infiltration of peripheral immune cells, and the expression of inflammatory mediators, such as cytokines and chemokines (Shaftel, S. S., Griffin, W. S. T., & O'Banion, M. K. (2008), Journal of Neuroinflammation, 5:7).

IL-1-mediated neuroinflammation may also play a role in the pathogenesis of neurodegenerative diseases. For example, elevated levels of IL-1 are reported in the brain tissue of patients with Alzheimer's disease (AD). AD (Griffin, W. S., Stanley, L. C., Ling, C., White, L., MacLeod, V., Perrot, L. J., Araoz, C. (1989), Proceedings of the National Academy of Sciences of the United States of America, 86, 7611-7615) and in rodent models of the disease (Benzing, W. C., Wujek, J. R., Ward, E. K., Shaffer, D., Ashe, K. H., Younkin, S. G., & Brunden, K. R. (1999), Neurobiology of Aging, 20(6), 581-589). The degree of neuroinflammation and IL-1 expression has been shown to correlate with the level of pathology in AD patients (Sheng, J. G., Ito, K., Skinner, R. D., Mrak, R. E., Rovnaghi, C. R., Van Eldik, L. J., & Griffin, W. S. (1996), Neurobiology of Aging, 17(5), 761-766).

Although the research to date may support a link between AD and IL-1, it is unclear what role IL-1 may play. IL-1 modulates actions that contribute to AD pathology, including the synthesis and processing of the Aβ precursor protein and the activity of acetylcholinesterase (Mrak, R. E. &Griffin, W. S. (2001), Neurobiology of Aging, 22(6), 903-908). Chronic IL-1 expression has been associated with demyelination (Ferrari, C. C., Depino, A. M., Prada, F., Muraro, N., Camptbell, S., Podhajcer, O., Pitossi, F. J. (2004), American Journal of Pathology, 165(5), 1827-1837), breakdown of the blood-brain barrier, and neutrophil recruitment (Ferrari et al, *supra*; Shaftel, S. S., Carlson, T. J., Olschowka, J. A., Kyrkanides, S., Matousek, S. B., & O'Banion, M. K. (2007), Journal of Neuroscience, 27(35), 9301-9309). IL-1 also activates microglia, which in turn produce pro-inflammatory cytokines such as IL-1, IL-6, and tumor necrosis factor alpha (TNFα). Neuronal insults, such as the accumulation of Aβ, may therefore induce a self-propagating cycle of cytokine activation in which levels of IL-1 constantly rise, leading to neuronal damage and further plaque deposition (Griffin, W. S. T., Sheng, J. G., Royston, M. C., Gentelman, S. M., McKenzie, J. E., Graham, D. I.,Mrak, R. E. (1998), Brain Pathology, 8, 65-72).

Prior research has explored the use of anti-inflammatory drugs as a therapeutic strategy against AD. Epidemiological studies have demonstrated a reduced risk of developing the disease in long-term non-steroidal anti-inflammatory drug (NSAID) users (Szekely, C. A., Breitner, J. C. S., Fitzpatrick, A. L., Rea, T. D., Psaty, B. M., Kuller, L. H., & Zandi, P. P. (2008), Neurology, 70(1), 17-24). In transgenic animal models, chronic NSAID administration has been somewhat effective in preventing or delaying the onset of amyloid deposition, dystrophic neurite formation and inflammation (Lim, G. P., Yang, F., Chu, T., Chen, P., Beech, W., Teter, B., Cole, G. M. (2000), Journal of Neuroscience, 20(15), 5709-5714). However, randomized clinical trials have failed to consistently support the therapeutic effectiveness of NSAIDs against AD (Scharf, S., Mander, A., Ugoni, A., Vajda, F., & Christophidis, N. (1999), Neurology, 53(1), 197-201; Aisen, P. S., Schafer, K. A., Grundman, M., Pfeiffer, E., Sano, M., Davis, K. L., Thai, L. J. (2003), Journal of the American Medical Association, 289(21), 2819-2826).

Given the small number of approved therapies to treat, or to slow down the progression of a disease characterized in part by beta amyloid expression, activity, or aberrant deposition, such as AD, there is a need to identify and explore the use of other agents for treating these diseases, such as the IL-1 antagonists as described herein.

US2006/0051381 A1 is concerned with cytokine antagonists for neurological and neuropsychiatric disorders.

### BRIEF SUMMARY OF THE INVENTION

The invention provides an IL-1 antagonist for use in a method for treating, or delaying the onset, or the progression of a disease characterized in part by beta amyloid expression, activity, or deposition in a subject in need thereof, the method comprising administering to the subject a therapeutically effective amount of an IL-1 antagonist as a first therapeutic agent, wherein the IL-1 antagonist is an IL-1 trap, wherein the IL-1 trap is a fusion protein comprising the amino acid sequence of SEQ ID NO: 10. An IL-1 antagonist is a compound capable of blocking or inhibiting at least one biological activity of IL-1. An IL-1 antagonist may take the form of an antibody, a soluble receptor, or a fusion protein capable of trapping IL-1, such as an IL-1 trap as described herein. The IL-1 antagonist employed in the invention is an IL-1 trap which is a fusion protein comprising the amino acid sequence of SEQ ID NO: 10. In one embodiment, the subject is a human patient suffering from Alzheimer's Disease (AD). The IL-1 trap may be administered alone, or in conjunction with one or more therapeutic agents that are useful for treating AD, or for slowing the progression of the disease, or for ameliorating at least one symptom associated with the disease, including, but not limited to behavioral changes associated with AD, or the cognitive decline or dysfunction observed in patients with AD.

**[Deleted]**

**[Deleted]**

**[Deleted]**

**[Deleted]**

The IL-1 antagonist is a fusion protein comprising an IL-1 binding portion of the extracellular domain of IL-1 Receptor Accessory protein (IL-1RAcP), an IL-1 binding portion of the extracellular domain of IL-1R1, and a multimerizing component. In particular, the IL-1 antagonist employed in the invention is an IL-1 trap is a fusion protein comprising the amino acid sequence of SEQ ID NO: 10.

In one embodiment, the IL-1 antagonist is an IL-1-specific fusion protein comprising two IL-1 receptor components and a multimerizing component, for example, an IL-1 trap as described in U.S. patent Nos. 6,927,044; 6,472,179; 7,459,426; 8,414,876; 7,361,350; 8,114,394; 7,820,154 and 7,632,490.

In one embodiment, the IL-1 trap is shown in SEQ ID NO: 10.

The subject being treated is most preferably a human suffering from a disease associated with beta-amyloid deposition and/or activity in the brain, such as Alzheimer's disease. Other subjects that may benefit from such therapy include subjects suffering from multi-infarct dementia, cognitive impairment, Down's syndrome and cerebral amyloid angiopathy. In certain embodiments the Alzheimer's disease may be prodromal, preclinical or clinical stage AD. In certain embodiments the cerebral amyloid angiopathy may be preclinical or clinical stage cerebral amyloid angiopathy.

The IL-1 trap is a fusion protein comprising the amino acid sequence of SEQ ID NO: 10.

**[Deleted]**

In certain embodiments, the administration of the IL-1 trap is subcutaneous, intramuscular, intranasal, intraarterial, intravenous, intrathecal, intraventricular, intracerebral, topical, transdermal administration or oral.

In one embodiment, a therapeutically effective amount of the IL-1 trap to be administered is between 1 mg/kg to 750 mg/kg.

In one embodiment, a therapeutically effective amount of the IL-1 trap to be administered is between 10 mg/kg to 500 mg/kg.

In one embodiment, a therapeutically effective amount of the IL-1 trap to be administered is between 50 mg/kg to 150 mg/kg.

In certain embodiments the IL-1 antagonist provided is for use in the method further comprising administering to a subject in need thereof a therapeutically effective amount of one or more other therapeutic agents, wherein the disease is lessened in severity or duration, or wherein the onset or progression of the disease is delayed.

Symptoms associated with the disease may include memory loss, depression, anxiety, dementia, irritability, confusion, inattention, mood swings, and aggressive and/or apathetic behavior.

In certain embodiments the other therapeutic agent(s) is/are administered by any route selected from subcutaneous, intramuscular, intranasal, intraarterial, intravenous, intrathecal, intraventricular, intracerebral, topical, transdermal administration or oral.

In one embodiment, the other therapeutic agent is an acetylcholinesterase inhibitor or a glutamate pathway modifier.

In one embodiment, the acetylcholinesterase inhibitor is selected from the group consisting of ARICEPT® (donepezil HCI), EXELON® (rivastigmine tartrate), and RAZADYNE® (galantamine HBr).

In one embodiment, the glutamate pathway modifier is Namenda (memantine).

In one embodiment, the other therapeutic agent(s) is/are selected from the group consisting of a different IL-1 antagonist, an anti-inflammatory agent, an antibody specific for tau, an antibody specific for beta amyloid and a microtubule stabilizer.

In one embodiment, the other therapeutic agent(s) is/are a different IL-1 antagonist selected from the group consisting of an IL-1 alpha or IL-1 beta antibody, a soluble IL-1 receptor, a different IL-1 trap, anakinra (KINERET®) and canakinumab.

In one embodiment, the anti-inflammatory agent is aspirin or a different NSAID.

In one embodiment, the antibody specific for beta amyloid is selected from the group consisting of solanezumab, gantenerumab, and bapineuzumab.

In one embodiment, the microtubule stabilizer is epothilone.

Also disclosed is a method of improving cognitive impairment in a mammal having beta amyloid deposits in brain tissue, the method comprising administering to the subject a therapeutically effective amount of an IL-1 antagonist as a first therapeutic agent, wherein the IL-1 antagonist is selected from the group consisting of an antibody specific for IL-1 alpha or IL-1 beta, or an antigen binding fragment thereof, a soluble IL-1 receptor, and an IL-1 fusion protein (IL-1 trap).

Also disclosed a method of improving cognitive impairment in a mammal having beta amyloid deposits in brain tissue, the method comprising administering to the subject a therapeutically effective amount of an IL-1 antagonist as a first therapeutic agent, wherein the IL-1 antagonist is an IL-1 fusion protein (IL-1 trap).

In one embodiment, the mammal demonstrates an improvement in cognitive function(s) without the necessity of a change in the beta amyloid plaque burden in the brain.

Also disclosed is a method of improving cognitive impairment in a mammal having beta amyloid deposits in brain tissue, the method comprising administering a composition comprising an IL-1 trap of the invention as a first therapeutic agent, either alone, or in combination with one or more other therapeutic agents useful for treating the disease or at least one symptom of the disease. In one instance, the method provides for improvement of cognitive impairment in a subject having beta amyloid deposits in the brain, without necessarily altering the amount (increase or decrease) of beta amyloid in the brain. The improvement of cognitive impairment in a subject may be an improvement in learning performance, or an improvement in memory performance, or a decrease in memory loss, or a decrease in learning impairment.

The cognitive impairment may be associated with Alzheimer's disease. The treatment may result in slowing the progression of any one or more cognitive or non-cognitive behavioral changes in the subject, including but not limited to memory loss, inability to learn, depression, anxiety, dementia, irritability, confusion, inattention, mood swings, diminished general locomotor and/or exploratory activity and aggressive and/or apathetic behavior. Other subjects that may benefit from therapy with an IL-1 trap of the invention in combination with one or more other therapeutic agents include subjects suffering from multi-infarct dementia, cognitive impairment, Down's syndrome and cerebral amyloid angiopathy. The Alzheimer's disease may be prodromal, preclinical or clinical stage AD. The cerebral amyloid angiopathy may be preclinical or clinical stage cerebral amyloid angiopathy.

The IL-1 trap is the fusion protein comprising SEQ ID NO: 10. The IL-1 trap may be as shown in SEQ ID NO: 10.

In certain embodiments, subjects being treated may suffer from chronic neuroinflammation, which may contribute to the neurodegeneration and/or associated cognitive or non-cognitive dysfunction observed in patients with Alzheimer's disease, or any of the other neurodegenerative conditions described herein. In certain embodiments, the subjects being treated with the IL-1 trap of the invention may have improved cognitive or non-cognitive behavioral symptoms following treatment, but will exhibit no change in the amount of beta-amyloid deposited in the brain. In certain embodiments, the subjects being treated with the IL-1 trap will demonstrate a diminished immune response triggered by the amyloid plaque burden. The reduction in immune response may be shown by a reduction in the number, activated phenotype and/or the size of peri-plaque microglia.

Administration of the IL-1 antagonist (IL-1 trap) may be by any means known to the art, for example, subcutaneous, intramuscular, intranasal, intraarterial, intravenous, intracerebral, intraventricular, intrathecal, topical, transvaginal, transdermal, transanal administration or oral routes of administration.

In one embodiment, a therapeutically effective amount of the IL-1 antagonist (IL-1 trap) to be administered to a subject in need thereof ranges from 1 mg/kg to 750 mg/kg, or 10 mg/kg to 500 mg/kg, or more preferably from 50 mg/kg to 150 mg/kg. In one embodiment, the IL-1 trap is for use in a method where it is administered on a weekly basis.

In one embodiment, a therapeutically effective amount of the IL-1 antagonist (IL-1 trap) to be administered to a subject in need thereof ranges from 10 mg to 500 mg, or 100 mg to 320 mg. In one embodiment, a therapeutically effective amount of the IL-1 antagonist (IL-1 trap) to be administered to a subject in need thereof is 100 mg, or 160 mg, or 320 mg. In certain embodiments the IL-1 trap is for use in a method as where it is administered on a weekly basis.

In certain embodiments the IL-1 antagonist is for use in therapeutic methods where the subject is treated with a combination of an IL-1 trap and one or more other (second or third, etc.) therapeutic agents. The other therapeutic agents may be a second IL-1 antagonist, such as, for example, anakinra (KINERET®) or canakinumab, or a second different IL-1 trap, or a recombinant, nonglycosylated form of the human IL-1 receptor antagonist (IL1Ra), or an anti-IL-18 drug such as IL-18BP or a derivative, an IL-18 Trap, anti-IL-18, anti-IL-18R1, or anti-IL-18Racp. Other co-therapies may include an acetylcholinesterase inhibitor (e.g. ARICEPT® (donepezil HCl), EXELON® (rivastigmine tartrate), RAZADYNE® (galantamine HBr)), or a glutamate pathway modifier, such as, Namenda (memantine HCl). Other co-therapies include aspirin or other NSAIDs, or other inflammatory inhibitors such as inhibitors of caspase-1, p38, IKK1/2, CTLA-4lg, anti-IL-6 or anti-IL6Ra, etc. Other co-therapies include an antibody specific for tau or an antibody specific for beta amyloid (such as solanezumab, gantenerumab, or bapineuzumab), as well as a microtubule stabilizer (such as epothilone B).

The IL-1 antagonist may be for use in a therapeutic method of treating a disease characterized by deposition of beta-amyloid in a subject, or ameliorating at least one symptom of a disease characterized by aberrant deposition of beta-amyloid in a subject, such as Alzheimer's disease, by administering a pharmaceutical composition comprising an IL-1 trap and a pharmaceutically acceptable carrier, in a dose range of about 1 mg/kg to about 300 mg/kg, preferably about 50 mg/kg to about 150 mg/kg alone, or in combination with a second therapeutic agent useful for treating the disease.

In one embodiment, the IL-1 antagonist may be for use in a method for delaying the onset of, or slowing the progression of the disease, comprising administering to a subject in need thereof a pharmaceutical composition comprising an IL-1 antagonist and a pharmaceutically acceptable carrier, in a dose range of about 10 mg/kg to about 300 mg/kg, or about 50 mg/kg to about 150 mg/kg on a weekly basis for a treatment period of between 1 week, 1 month, to one year or more. In certain embodiments, the treatment could last for decades.

In certain embodiments, the IL-1 antagonist to be used as a second therapeutic agent is an antibody specific for either IL-1 alpha or IL-1 beta.

In certain embodiments the IL-1 antagonist to be used as a second therapeutic agent is a soluble IL-1 receptor that blocks or inhibits the activity of either or both IL-1 alpha and/or IL-1 beta.

In certain embodiments, the IL-1 antagonist to be used as a second therapeutic agent is anakinra or canakinumab.

In certain embodiments, the IL-1 antagonist to be used as a first or second therapeutic agent is an IL-1 trap as described herein.

In one particular embodiment, the IL-1 antagonist to be used as a first therapeutic agent is the fusion protein (IL-1 trap) as shown in SEQ ID NO: 10. In one embodiment, the IL-1 trap is shown in SEQ ID NO: 10.

In certain embodiments, the IL-1 antagonist may be administered twice a week, or weekly, or monthly, or bi-monthly, or less frequently depending on the results achieved.

In certain embodiments, the doses may be adjusted if it is determined that the patient may need chronic life-long therapy with the IL-1 trap alone, or in conjunction with a second therapeutic agent useful for treating the disease. In certain embodiments, the methods for treating a disease characterized in part by beta amyloid activity or deposition in brain tissue of a patient comprises administering to a subject in need thereof a pharmaceutical composition comprising an IL-1 trap at doses of about 100 mg, or about 160 mg, or about 320 mg and a pharmaceutically acceptable carrier. In certain embodiments the IL-1 trap is administered on a weekly basis. In certain embodiments, the IL-1 trap may be administered on a bi-weekly basis, a monthly basis, or a bi-monthly basis, or less frequently as determined by a patient's response to therapy.

In certain embodiments, the pharmaceutical composition may contain a second or third therapeutically effective amount of another agent useful for treating the disease (a co-formulation). The second or third other therapeutic agent(s) may be a second IL-1 antagonist, such as, for example, anakinra (KINERET®) or canakinumab, a different IL-1 trap, a recombinant, nonglycosylated form of the human IL-1 receptor antagonist (IL1Ra), or an anti-IL-18 drug such as IL-18BP or a derivative, an IL-18 Trap, anti-IL-18, anti-IL-18R1, or anti-IL-18Racp. Other co-therapies include acetylcholinesterase inhibitors (e.g. ARICEPT® (donepezil HCl), EXELON® (rivastigmine tartrate), RAZADYNE® (galantamine HBr)), or glutamate pathway modifiers, such as, Namenda (memantine HCl). Other co-therapies include aspirin or other NSAIDs, or other inflammatory inhibitors such as inhibitors of caspase-1, p38, IKK1/2, CTLA-4lg, anti-IL-6 or anti-IL6Ra, etc. Other co-therapies include an antibody specific for tau or an antibody specific for beta amyloid (such as solanezumab, gantenerumab, or bapineuzumab), as well as a microtubule stabilizer (such as epothilone B).

In one embodiment, the first and second other therapeutic agent may be administered simultaneously in one pharmaceutical formulation, or may be administered sequentially in different pharmaceutical compositions.

In one embodiment, the disease that is to be treated with a pharmaceutical composition containing an IL-1 trap of the invention is Alzheimer's disease. In certain embodiments, the treatment with the pharmaceutical composition results in preventing the onset of, slowing the progression of, or ameliorating/improving any one or more cognitive or non-cognitive behavioral changes in the subject suffering from Alzheimer's disease, including but not limited to memory loss, inability to learn, depression, anxiety, dementia, inattention, irritability, confusion, mood swings and aggressive and/or apathetic behavior.

**[Deleted]**

**[Deleted]**

**[Deleted]**

**[Deleted]**

Other objects and advantages will become apparent from a review of the ensuing detailed description.

### BRIEF DESCRIPTION OF THE FIGURES

Figure 1a shows the results of the water maze acquisition test.
Figure 1b shows the results of the water maze retention test (Time in Goal Quadrant).
Figure 1c shows the results of the water maze retention test (Platform crosses).

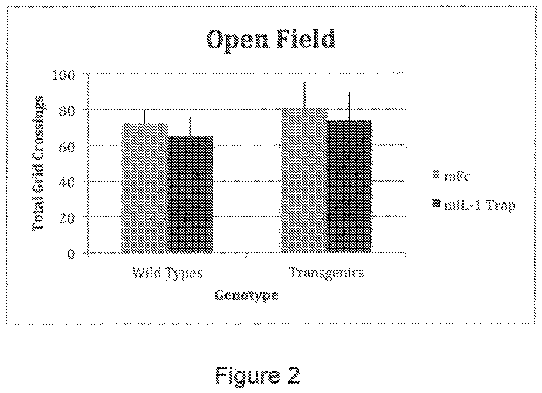

Figure 2 shows the results of the open field test.
Figure 3 shows increased plaque burden in transgenic animals.
Figure 4a shows the median number of microglia-like cells per plaque.
Figure 4b shows the mean size of lba-1-immunoreactive microglial-like cells in transgenic mice stratified by proximity to plaques.
Figure 4c shows the mean difference in microglial size per animal in microglial in normal tissue (at least 30 microns from the nearest plaque) versus contacting amyloid plaques.
Figure 5 shows the mean dorsal hippocampal volume at sacrifice for the four groups of animals.

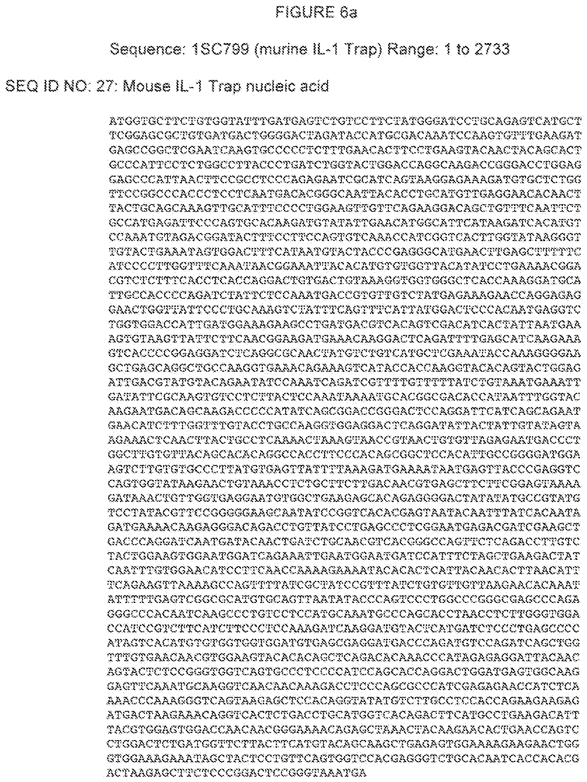

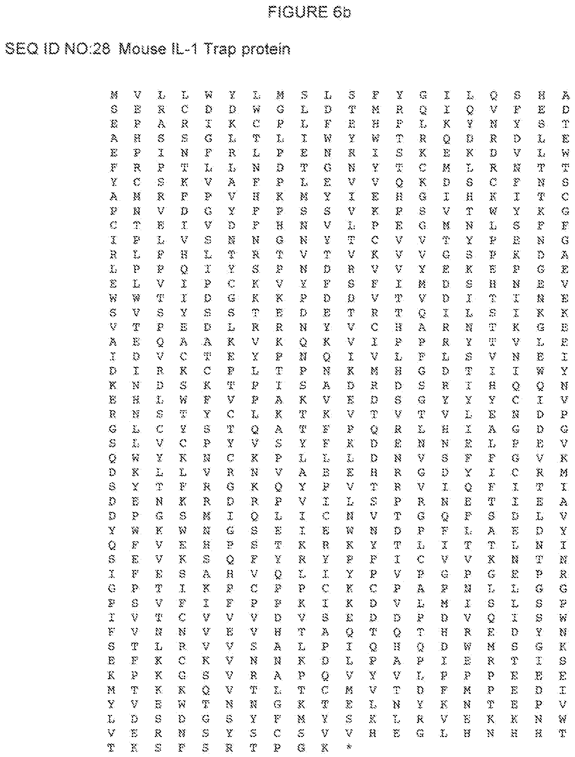

Figure 6a shows the nucleic acid sequence (SEQ ID NO: 27) of the mouse IL-1 trap and Figure 6b shows the amino acid sequence (SEQ ID NO: 28) of the mouse IL-1 trap as utilized in the studies described herein.

### DETAILED DESCRIPTION

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only the appended claims.

As used in this specification and the appended claims, the singular forms "a", "an", and "the" include plural references unless the context clearly dictates otherwise. Thus for example, a reference to "a method" includes one or more methods, and/or steps of the type described herein and/or which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All patents, applications and non-patent publications mentioned in this specification are incorporated herein by reference in their entireties.

### General Description

Alzheimer's disease (AD) is a degenerative brain disorder characterized clinically by both cognitive and non-cognitive behavioral changes, including progressive memory deficits, depression, anxiety, dementia, irritability, mood swings, inattention, aggressive and/or apathetic behavior, confusion, gradual physical deterioration and, ultimately, death. Histologically, the disease is characterized by neuritic plaques, composed primarily of beta amyloid (Aβ) peptide. The plaques are found primarily in the association cortex, limbic system and basal ganglia. Beta amyloid peptide is the cleavage product of beta amyloid precursor protein (β APP or APP). APP is a type I transmembrane glycoprotein that contains a large ectopic N-terminal domain, a transmembrane domain, and a small cytoplasmic C-terminal tail. Alternative splicing of the transcript of the single APP gene on chromosome 21 results in several isoforms that differ in the number of amino acids.

It is believed that Aβ may play a role in the neuropathology of Alzheimer's disease. For example, familial forms of the disease have been linked to mutations in APP and the presenilin genes (Tanzi et al., 1996, Neurobiol. Dis. 3:159-168; Hardy, 1996, Ann. Med. 28:255-258). Furthermore, diseased-linked mutations in these genes result in increased production of the 42-amino acid form of Aβ, the predominant form found in amyloid plaques.

The proinflammatory cytokine interleukin-1 (IL-1) is as an important player in inflammatory processes throughout the body, including in the central nervous system (CNS). IL-1 is found in two distinct isoforms, IL-1α and IL-1β, although IL-1β is considered the primary active isoform. Upregulation of IL-1β is part of the response to a range of CNS insults, including infections, stroke, and traumatic injuries (Allan, S. M., Tyrrell, P. J., & Rothwell, N. J. (2005). Nature Reviews Immunology, 5, 629-640). This neuroinflammatory response is characterized by activation of resident glial cells (microglia and astrocytes), infiltration of peripheral immune cells, and the expression of inflammatory mediators, such as cytokines and chemokines (Shaftel, S. S., Griffin, W. S. T., & O'Banion, M. K. (2008), Journal of Neuroinflammation, 5:7).

IL-1-mediated neuroinflammation may also play a role in the pathogenesis of neurodegenerative diseases (Griffin, W. S., Stanley, L. C., Ling, C., White, L., MacLeod, V., Perrot, L. J., Araoz, C. (1989), Proceedings of the National Academy of Sciences of the United States of America, 86, 7611-7615; Benzing, W. C., Wujek, J. R., Ward, E. K., Shaffer, D., Ashe, K. H., Younkin, S. G., & Brunden, K. R. (1999), Neurobiology of Aging, 20(6), 581-589; Sheng, J. G., Ito, K., Skinner, R. D., Mrak, R. E., Rovnaghi, C. R., Van Eldik, L. J., & Griffin, W. S. (1996), Neurobiology of Aging, 17(5), 761-766).

Although the research to date may support a link between AD and IL-1, it is unclear what role IL-1 plays. IL-1 modulates actions that may contribute to AD pathology, including the synthesis and processing of the Aβ precursor protein and the activity of acetylcholinesterase (Mrak, R. E. &Griffin, W. S. (2001), Neurobiology of Aging, 22(6), 903-908). Chronic IL-1 expression has been associated with demyelination (Ferrari, C. C., Depino, A. M., Prada, F., Muraro, N., Camptbell, S., Podhajcer, O., Pitossi, F. J. (2004), American Journal of Pathology, 165(5), 1827-1837), breakdown of the blood-brain barrier, and neutrophil recruitment (Ferrari et al, *supra*; Shaftel, S. S., Carlson, T. J., Olschowka, J. A., Kyrkanides, S., Matousek, S. B., & O'Banion, M. K. (2007a), Journal of Neuroscience, 27(35), 9301-9309). IL-1 also activates microglia, which in turn produce pro-inflammatory cytokines such as IL-1, IL-6, and tumor necrosis factor alpha (TNFα). Neuronal insults, such as the accumulation of Aβ, may therefore induce a self-propagating cycle of cytokine activation in which levels of IL-1 constantly rise, leading to neuronal damage and further plaque deposition (Griffin, W. S. T., Sheng, J. G., Royston, M. C., Gentelman, S. M., McKenzie, J. E., Graham, D. I.,Mrak, R. E. (1998), Brain Pathology, 8, 65-72).

On the other hand, some have argued that IL-1 plays a beneficial role in AD. Sustained IL-1β overexpression for 4 weeks reduces amyloid plaque expression in swAPP-PS1 mice, a mouse model of Alzheimer's-like pathology that uses the Swedish pedigree mutation in the amyloid precursor protein and the high Alzheimer's risk polymorphism in presenilin-1 (Shaftel, S. S., Kyrkanides, S., Olschowka, J. A., Miller, J. H., Johnson, R. E., & O'Banion, M. K. (2007b), Journal of Clinical Investigation, 117(6), 1595-1604). In the AD brain, microglia expressing IL-1 surround amyloid plaque deposits, suggesting an attempt at phagocytic removal of the plaques (Griffin, W. S., Stanley, L. C., Ling, C., White, L., MacLeod, V., Perrot, L. J., Araoz, C. (1989), Proceedings of the National Academy of Sciences of the United States of America, 86, 7611-7615). In early stages of the disease, microglial activation does seem to delay the progression of AD-like pathology (EI Khoury, J., & Luster, A. D. (2008), Trends in Pharmacological Sciences, 29, 626-632; Simard, A. R., Soulet, D., Gowing, G., Julien, J., & Rivest, S. (2006), Neuron, 49, 489-502). However, amyloid plaque burden eventually increases, despite continued microglial activation. One explanation is that the microglia become defective and lose their Aβ-clearing effectiveness. The expression of microglial Aβ receptors and Aβ-degrading enzymes start to decrease around 8 months of age in swAPP-PS1 mice, resulting in reduced Aβ uptake and clearance (Hickman, S. E., Allison, E. K., & EI Khoury, J. (2008), Neurobiology of Disease, 28, 8354-8360). The microglia, however, maintain production of IL-1β and TNFα.

Hippocampally-mediated memory processes may be impaired by the overexpression of IL-1 (Moore, A. H., Wu, M., Shaftel, S., Graham, K. A., & O'Banion, M. K. (2009), Neuroscience, 164, 1484-1495; Tanaka, S., Ide, M., Shibutani, T., Ohtaki, H., Numazawa, S., Shioda, S., & Yoshida, T. (2006), Journal of Neuroscience Research, 83, 557-566; Depino, A. M., Alonso, M., Ferrari, C., del Ray, A., Anthony, D., Besedovsky, H., Pitossi, F. (2004), Hippocampus, 14, 526-535).

Although prior research has explored the use of anti-inflammatory drugs as a therapeutic strategy against AD, randomized clinical trials have failed to consistently support the therapeutic effectiveness of NSAIDs against AD (Scharf, S., Mander, A., Ugoni, A., Vajda, F., & Christophidis, N. (1999), Neurology, 53(1), 197-201; Aisen, P. S., Schafer, K. A., Grundman, M., Pfeiffer, E., Sano, M., Davis, K. L., Thai, L. J. (2003), Journal of the American Medical Association, 289(21), 2819-2826).

To date, there have been no studies examining the effects of chronic, systemic IL-1 inhibition on AD-like behavior and pathology. The current studies, described herein, utilized a mouse IL-1 Trap (mIL-1 Trap), an immunoadhesin consisting of a forced IL-1 receptor 1 homodimer fused to a mouse Fc fragment. This trap binds IL-1 at a high affinity, preventing it from binding to its endogenous receptor, and therefore serves as an antagonist of IL-1 signaling. After five months of subcutaneous administration of the mIL-1 Trap in the swAPP-PS1 mutant mouse model of Alzheimer's-like pathology, the effect of IL-1 inhibition on spatial memory, open field activity, amyloid plaque burden, microglial activation, and overall inflammation was examined.

### Definitions

By the term "blocker", "inhibitor", or "antagonist" is meant a substance that retards or prevents a chemical or physiological reaction or response. Common blockers or inhibitors include but are not limited to antisense molecules, antibodies, antagonists and their derivatives. More specifically, an example of an IL-1 blocker or inhibitor is an IL-1 antagonist including, but not limited to, an antibody (human or humanized), or an antigen binding portion thereof, to IL-1 alpha and/or IL-1 beta, a soluble IL-1 receptor that blocks or inhibits the activity of either IL-1 alpha or IL-1 beta or both, or an IL-1 trap as described herein, which binds and inhibits IL-1 activity. The IL-1 trap of the invention is a fusion protein comprising the amino acid sequence of SEQ ID NO: 10.

By the term "therapeutically effective dose" is meant a dose that produces the desired effect for which it is administered. The exact dose will depend on the purpose of the treatment, and will be ascertainable by one skilled in the art using known techniques (see, for example, Lloyd (1999) The Art, Science and Technology of Pharmaceutical Compounding).

By the term "substantially identical" is meant a protein sequence having at least 95% identity to an amino acid sequence selected from the group consisting of the amino acid sequences SEQ ID NOs: 2, 4, 6, 8, 10, 12, 14, 16, 18, 20, 22, 24, 26 and 28, and capable of binding IL-1 and inhibiting the biological activity of IL-1.

The term "identity" or "homology" is construed to mean the percentage of amino acid residues in the candidate sequence that are identical with the residue of a corresponding sequence to which it is compared, after aligning the sequences and introducing gaps, if necessary to achieve the maximum percent identity for the entire sequence, and not considering any conservative substitutions as part of the sequence identity. Neither N- or C-terminal extensions nor insertions will be construed as reducing identity or homology. Methods and computer programs for the alignment are well known in the art. Sequence identity may be measured using sequence analysis software (e.g., Sequence Analysis Software Package, Genetics Computer Group, University of Wisconsin Biotechnology Center, 1710 University Ave., Madison, Wis. 53705). This software matches similar sequences by assigning degrees of homology to various substitutions, deletions, and other modifications.

The term "treating" (or "treat" or "treatment") refers to processes involving a slowing, interrupting, inhibiting, arresting, controlling, stopping, reducing, ameliorating, or reversing the progression, duration, or severity of an existing symptom, disorder, condition, or disease, but does not necessarily involve a total elimination of all disease-related symptoms, conditions, or disorders through use of the IL-1 trap as described herein. Furthermore, "treating", "treatment" or "treat" refers to an approach for obtaining beneficial or desired results including clinical results, which include, but are not limited to, one or more of the following: inhibiting, delaying or preventing the onset of, or the progression of, a disease associated with beta amyloid activity, or characterized by aberrant deposition of beta amyloid in a subject, such as in Alzheimer's disease; or inhibiting, preventing, or ameliorating at least one symptom associated with a disease associated with beta amyloid activity, or characterized by aberrant deposition of beta amyloid in a subject, such as in Alzheimer's disease, wherein the symptoms include, but are not limited to, cognitive impairment, memory loss, depression, anxiety, dementia, irritability, confusion, mood swings, aggressive and/or apathetic behavior. "Treatment" or "treating", as used herein, also refers to increasing the quality of life of those suffering from the disease, decreasing the dose of other medications required to treat the disease and/or prolonging survival of patients.

"Delaying the onset of" Alzheimer's disease or a symptom thereof means to defer, hinder, slow, retard, stabilize, and/or postpone development of the disease, or a symptom associated with, or resulting from the disease. This delay can be of varying lengths of time, depending on the history of the disease and/or individual being treated. As is evident to one skilled in the art, a sufficient or significant delay can, in effect, encompass prevention, in that the individual does not develop the disease. A method that "delays" development of Alzheimer's disease is a method that reduces probability of disease development in a given time frame and/or reduces extent of the disease in a given time frame, when compared to not using the method. Such comparisons are typically based on clinical studies, using a statistically significant number of subjects.

"Development" of Alzheimer's disease means the onset and/or progression of Alzheimer's disease within an individual. Alzheimer's disease development can be detectable using standard clinical techniques. However, development also refers to disease progression that may be initially undetectable. For purposes of this invention, progression refers to the biological course of the disease state, in this case, as determined by a standard neurological examination, patient interview, or may be determined by more specialized testing. A variety of these diagnostic tests include, but are not limited to, neuroimaging, detecting alterations of levels of specific proteins in the serum or cerebrospinal fluid (e.g., amyloid peptides and Tau), computerized tomography (CT), positron emission tomography (PET), and magnetic resonance imaging (MRI). "Development" includes occurrence, recurrence, and onset. As used herein "onset" or "occurrence" of Alzheimer's disease includes initial onset and and/or recurrence.

The term "Alzheimer's Disease" or "AD" generally refers to a clinical entity that typically presents with a characteristic progressive amnestic disorder with subsequent appearance of other cognitive, behavioral and neuropsychiatric changes that impair social function and activities of daily living. The initial presentation can be atypical, with non-amnestic focal cortical cognitive symptoms. Disease onset and/or progression can now be assessed through the use of validated and disease-specific biomarkers. Laboratory and neuroimaging biomarkers are highly correlated with neuropathological lesions of AD. These biomarkers can be divided into pathophysiological and topographical markers. Pathophysiological markers correspond to the two etiological degenerative processes that characterize Alzheimer's pathology: the amyloidosis path to neuritic plaques and the tauopathy path to neurofibrillary tangles. They include CSF measurements of concentrations of amyloid beta, total tau, and phosphotau, amyloid PET scanning with Pittsburgh compound B or other radioligands (florbetaben, ¹⁸F-AV-45, etc.). Topographical markers are used to assess the less specific and downstream brain changes that correlate with the regional distribution of AD pathology and include medial temporal lobe atrophy (as measured by MRI) and reduced glucose metabolism in temporo-parietal regions on fluorodeoxyglucose PET. These MRI and PET markers have been shown to predict the development of AD dementia in mild cognitive impairment (MCI) cohorts and to correlate with disease severity. Patients with clinical AD suffer from moderate to severe cognitive and memory impairments that meet the diagnostic criteria of AD and impact work and relationships (including, potentially activities of daily living) and these symptoms are usually accompanied by positive findings on a biomarker tests as described above.

"Prodromal Alzheimer's disease", also referred to as "predementia stage of AD" refers to the early symptomatic predementia phase of AD in which 1) clinical symptoms including episodic memory loss of the hippocampal type are present, but not sufficiently severe to affect instrumental activities of daily living and do not warrant a diagnosis of dementia; and in which 2) biomarker evidence from CSF or imaging is supportive of the presence of AD pathological changes.

"Preclinical Alzheimer's disease", which includes both "asymptomatic at-risk state for AD" and "presymptomatic AD" refer to the long asymptomatic stage between the earliest pathogenic events/brain lesions of AD and the first appearance of specific cognitive changes. The "asymptomatic at-risk" state for AD is identified *in vivo* by evidence of amyloidosis in the brain (with retention of specific PET amyloid tracers) or in the CSF (with changes in amyloid beta, tau, and phosphotau concentrations). "Presymptomatic AD" applies to individuals who will develop AD and this can only be ascertained in families that are affected by rare autosomal dominant monogenic mutations (monogenic AD).

"Cerebral amyloid angiopathy" (CAA), also known as congophilic angiopathy is a form of angiopathy in which amyloid deposits form in the walls of the blood vessels of the central nervous system. The term congophilic is used because the presence of the abnormal aggregations of amyloid can be demonstrated by microscopic examination of brain tissue after application of a special stain called Congo red. The amyloid material is only found in the brain and as such the disease is not related to other forms of amyloidosis. CAA has been identified as occurring either sporadically (generally in elderly populations) or in familial forms such as Flemish, Iowa, and Dutch types. Sporadic forms of CAA have been further characterized into two types based on deposition of amyloid β-protein (Aβ) in cortical capillaries. In all cases, it is defined by the deposition of Aβ in the leptomeningal and cerebral vessel walls. Amyloid deposition predisposes these blood vessels to failure, increasing the risk of a hemorrhagic stroke. Since this can be caused by the same amyloid protein that is associated with Alzheimer's dementia, such brain hemorrhages are more common in people who suffer from Alzheimer's, however they can also occur in those who have no history of dementia. The hemorrhage within the brain is usually confined to a particular lobe and this is slightly different compared to brain hemorrhages that occur as a consequence of high blood pressure (hypertension), a more common cause of a hemorrhagic stroke (or cerebral hemorrhage).

### IL-1- Specific Antagonists

The invention provides IL-1 antagonists for the treatment of diseases characterized by aberrant deposition of beta amyloid in a subject, such as in patients suffering from Alzheimer's disease (AD). In particular, the present invention provides an IL-1 antagonist for use in a method for treating, or delaying the onset, or the progression of a disease characterized in part by beta amyloid expression, activity, or deposition in a subject in need thereof, the method comprising administering to the subject a therapeutically effective amount of an IL-1 antagonist as a first therapeutic agent, wherein the IL-1 antagonist is an IL-1 trap, wherein the IL-1 trap is a fusion protein comprising the amino acid sequence of SEQ ID NO: 10. In certain embodiments the IL-1 antagonist may be for use in a method wherein further IL-1 antagonists are also administered. In certain embodiments the further IL-1 antagonists may include an antibody (or an antigen binding fragment thereof) specific for IL-1 alpha or IL-1 beta, or a soluble receptor that blocks or inhibits the activity of IL-1 alpha or IL-1 beta or both. In certain embodiments, the further IL-1 antagonist may be anakinra or canakinumab. The IL-1 antagonist provided though is an IL-1-specific fusion protein antagonist (sometimes referred to as an "IL-1 trap"), which is useful for treating such conditions and which is a fusion protein comprising the amino acid sequence of SEQ ID NO: 10. IL-1 traps are multimers of fusion proteins containing IL-1 receptor components and a multimerizing component capable of interacting with the multimerizing component present in another fusion protein to form a higher order structure, such as a dimer. Cytokine traps include two distinct receptor components that bind a single cytokine, resulting in the generation of antagonists with dramatically increased affinity over that offered by single component reagents. In fact, the cytokine traps that are described herein are among the most potent cytokine blockers ever described. Briefly, the cytokine traps called IL-1 traps are comprised of the extracellular domain of human IL-1R Type I (IL-1RI) or Type II (IL-1RII) followed by the extracellular domain of human IL-1 Receptor Accessory protein (IL-1RAcP), followed by a multimerizing component. In one embodiment, the multimerizing component is an immunoglobulin-derived domain, such as, for example, the Fc region of human IgG, including part of the hinge region, the CH2 and CH3 domains. An immunoglobulin-derived domain may be selected from any of the major classes of immunoglobulins, including IgA, IgD, IgE, IgG and IgM, and any subclass or isotype, e.g. IgG1, IgG2, IgG3 and IgG4; IgA-1 and IgA-2. For a more detailed description of the IL-1 traps, see WO00/18932, US6,927,044; US7,459,426. Preferred IL-1-specific fusion proteins have the amino acid sequence shown in SEQ ID NO: 10.

In certain embodiments, the IL-1 antagonist provided may be for use in a method that comprises administering a further IL-1 antagonist that comprises an antibody fragment capable of binding IL-1α, IL-1β, IL-1R1 and/or IL-1RAcp, or a fragment thereof. The preferred embodiment would be an antagonist of IL-1β. One embodiment of an IL-1 antagonist comprises one or more antibody fragments, for example, single chain Fv (scFv), is described in U.S. 6,472,179. In all of the IL-1 antagonist embodiments comprising one or more antibody-derived components specific for IL-1 or an IL-1 receptor, the components may be arranged in a variety of configurations, e.g., a IL-1 receptor component(s)-scFv(s)-multimerizing component; IL-1 receptor component(s)-multimerizing component-scFv(s); scFv(s)-IL-1 receptor component(s)-multimerizing component, ScFv-ScFv-Fc, etc., so long as the molecule or multimer is capable of inhibiting the biological activity of IL-1.

### Anti-IL-1 Human Antibodies and Antibody Fragments

In another embodiment of the IL-1 antagonist provided is for use in a method comprising administering a further IL-1 antagonist, where the further IL-1 antagonist is an IL-1 antibody, examples of which are anti-IL-1 antibodies are disclosed in US 4,935,343; US 5,681,933; WO 95/01997; EP 0267611, US 6,419,944; WO 02/16436 and WO 01/53353. The further IL-1 antagonist may include an antibody or antibody fragment specific for an IL-1 ligand (e.g., IL-1α or IL-1β) and/or an IL-1 receptor (e.g., IL-1R1 and/or IL-1RAcp). Antibody fragments include any fragment having the required target specificity, e.g. antibody fragments either produced by the modification of whole antibodies (e.g. enzymatic digestion), or those synthesized *de novo* using recombinant DNA methodologies (scFv, single domain antibodies or dAbs, single variable domain antibodies) or those identified using human phase display libraries (see, for example, McCafferty et al. (1990) Nature 348:552-554). Alternatively, antibodies can be isolated from mice producing human or human-mouse chimeric antibodies using standard immunization and antibody isolation methods, including but not limited to making hybridomas, or using B cell screening technologies, such as SLAM. Immunoglobulin binding domains also include, but are not limited to, the variable regions of the heavy (V_{H}) or the light (V_{L}) chains of immunoglobulins. Or by immunizing people and isolating antigen positive B cells and cloning the cDNAs encoding the heavy and light chain and coexpressing them in a cell, such as CHO.

The term "antibody" as used herein refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant regions, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. Within each IgG class, there are different isotypes (eg. IgG₁, IgG₂, IgG₃, IgG₄). Typically, the antigen-binding region of an antibody will be the most critical in determining specificity and affinity of binding.

An exemplary immunoglobulin (antibody) structural unit comprises a tetramer. Each tetramer is composed of two identical pairs of polypeptide chains, each pair having one light chain (about 25 kD) and one heavy chain (about 50-70 kD). The N-terminus of each chain defines a variable region of about 100-110 or more amino acids primarily responsible for antigen recognition. The terms "variable light chain" (V_{L}) and variable heavy chain (V_{H}) refer to these light and heavy chains respectively.

Antibodies exist as intact immunoglobulins, or as a number of well-characterized fragments produced by digestion with various peptidases. For example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region. While various antibody fragments are defined in terms of the digestion of an intact antibody, one of skill will appreciate that such fragments may be synthesized *de novo* either chemically or by using recombinant DNA methodology.

Methods for preparing antibodies are known to the art. See, for example, Kohler & Milstein (1975) Nature 256:495-497; Harlow & Lane (1988) Antibodies: a Laboratory Manual, Cold Spring Harbor Lab., Cold Spring Harbor, NY). The genes encoding the heavy and light chains of an antibody of interest can be cloned from a cell, e.g., the genes encoding a monoclonal antibody can be cloned from a hybridoma and used to produce a recombinant monoclonal antibody. Monoclonal antibodies can be humanized using standard cloning of the CDR regions into a human scaffold. Gene libraries encoding human heavy and light chains of monoclonal antibodies can also be made from hybridoma or plasma cells. Random combinations of the heavy and light chain gene products generate a large pool of antibodies with different antigenic specificity. Techniques for the production of single chain antibodies or recombinant antibodies (US 4,946,778; US 4,816,567) can be adapted to produce antibodies used in the fusion proteins and methods of the instant invention. Also, transgenic mice, or other organisms such as other mammals, may be used to express human, human-mouse chimeric, or humanized antibodies. Alternatively, phage display technology can be used to identify human antibodies and heteromeric Fab fragments that specifically bind to selected antigens.

### Antibody Screening and Selection

Screening and selection of preferred antibodies can be conducted by a variety of methods known to the art. Initial screening for the presence of monoclonal antibodies specific to a target antigen may be conducted through the use of ELISA-based methods, for example. A secondary screen is preferably conducted to identify and select a desired monoclonal antibody for use in construction of the multi-specific fusion proteins of the invention. Secondary screening may be conducted with any suitable method known to the art. One preferred method, termed "Biosensor Modification-Assisted Profiling" ("BiaMAP") is described in co-pending USSN 60/423,017 filed 01 Nov 2002. BiaMAP allows rapid identification of hybridoma clones producing monoclonal antibodies with desired characteristics. More specifically, monoclonal antibodies are sorted into distinct epitope-related groups based on evaluation of antibody:antigen interactions. Antibodies capable of blocking either a ligand or a receptor may be identified by a cell based assay, such as a luciferase assay utilizing a luciferase gene under the control of an NFKB driven promoter. Stimulation of the IL-1 receptors by IL-1 ligands leads to a signal through NFKB thus increasing luciferase levels in the cell. Blocking antibodies are identified as those antibodies that blocked IL-1 induction of luciferase activity.

### Treatment Population

The IL-1 antagonist provided is for use in therapeutic methods which are useful for treating individuals affected with a disease or condition characterized by aberrant deposition and/or activity of beta amyloid in a subject. Diseases or conditions for which the current IL-1 antagonists may be used include Alzheimer's disease, multi-infarct dementia, cognitive impairment and cerebral amyloid angiopathy (CAA). The stages of Alzheimer's disease (AD) or cerebral amyloid angiopathy (CAA) for which the treatments may be effective include any of the following: prodromal AD or CAA, preclinical AD or CAA and clinical stage AD or CAA, as described previously.

### Therapeutic Administration and Formulations

The invention provides therapeutic compositions comprising the IL-1 antagonist (IL-1 trap) of the present invention. The administration of therapeutic compositions in accordance with the invention will be administered via a suitable route including, but not limited to, intravenously, subcutaneously, intramuscularly, intrathecally, intracerebrally, intraventricularly, intranasally, with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

The dose of the IL-1 antagonist, e.g. IL-1 trap, may vary depending upon the age and the size of a subject to be administered, target disease, conditions, route of administration, and the like. When an antibody is used for treating Alzheimer's disease, it is advantageous to intravenously administer the antibody of the present invention normally at a single dose of about 1 to about 750 mg/kg body weight, more preferably about 5 to about 300, about 10 to about 200, or about 50 to about 150 mg/kg body weight. Depending on the severity of the condition, the frequency and the duration of the treatment can be adjusted. In certain instances, the IL-1 trap can be administered as an initial dose of at least about 0.1 mg to about 800 mg, about 1 to about 500 mg, about 5 to about 300 mg, or about 10 to about 200 mg, to about 100 mg, or to about 50 mg. In certain instances, the initial dose may be followed by administration of a second or a plurality of subsequent doses of the antibody or antigen-binding fragment thereof in an amount that can be approximately the same or less than that of the initial dose, wherein the subsequent doses are separated by at least 1 day to 3 days; at least one week, at least 2 weeks; at least 3 weeks; at least 4 weeks; at least 5 weeks; at least 6 weeks; at least 7 weeks; at least 8 weeks; at least 9 weeks; at least 10 weeks; at least 12 weeks; or at least 14 weeks. The IL-1 trap of the invention may be administered to a subject using any of the above described dosing regimens throughout the life of the patient.

Various delivery systems are known and can be used to administer the pharmaceutical composition of the invention, *e.g*., encapsulation in liposomes, microparticles, microcapsules, recombinant cells capable of expressing the mutant viruses, receptor mediated endocytosis (see, *e.g*., Wu et al. (1987) J. Biol. Chem. 262:4429-4432). Methods of introduction include, but are not limited to, intradermal, transdermal, intramuscular, intraperitoneal, intravenous, subcutaneous, intranasal, epidural, intrathecal, intraventricular, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

The pharmaceutical composition can be also delivered in a vesicle, in particular a liposome (see, for example, Langer (1990) Science 249:1527-1533).

In certain situations, the pharmaceutical composition can be delivered in a controlled release system. In one embodiment, a pump may be used. In another embodiment, polymeric materials can be used. In yet another embodiment, a controlled release system can be placed in proximity of the composition's target, thus requiring only a fraction of the systemic dose.

The injectable preparations may include dosage forms for intravenous, subcutaneous, intracutaneous and intramuscular injections, drip infusions, etc. These injectable preparations may be prepared by methods publicly known. For example, the injectable preparations may be prepared, *e.g*., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (*e.g*., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, *e.g*., sesame oil, soybean oil, etc., which may be used in combination with a solubilizing agent such as benzyl benzoate, benzyl alcohol, etc. The injection thus prepared is preferably filled in an appropriate ampoule.

A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but certainly are not limited to AUTOPEN™ (Owen Mumford, Inc., Woodstock, UK), DISETRONIC™ pen (Disetronic Medical Systems, Burghdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (sanofi-aventis, Frankfurt, Germany), to name only a few. Examples of disposable pen delivery devices having applications in subcutaneous delivery of a pharmaceutical composition of the present invention include, but certainly are not limited to the SOLOSTAR™ pen (sanofi-aventis), the FLEXPEN™ (Novo Nordisk), and the KWIKPEN™ (Eli Lilly), the SURECLICK™ Autoinjector (Amgen, Thousands Oaks, CA), the PENLET™ (Haselmeier, Stuttgart, Germany), the EPIPEN (Dey, L.P.) and the HUMIRA™ Pen (Abbott Labs, Abbott Park, IL), to name only a few.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc. The amount of the aforesaid antibody contained is generally about 5 to about 750 mg per dosage form in a unit dose; especially in the form of injection, it is preferred that the aforesaid antibody is contained in about 5 to about 100 mg and in about 10 to about 250 mg for the other dosage forms.

### Combination Therapies

In numerous embodiments, the IL-1 antagonists of the present invention may be administered in combination with one or more additional compounds or therapies. Combination therapy may be simultaneous or sequential. The IL-1 antagonists of the invention may be combined with other IL-1 antagonists, such as antibodies specific for IL-1 alpha or IL-1 beta (or antigen binding fragments thereof), a soluble IL-1 receptor, or other different IL-1 traps. The IL-1 traps of the invention may also be be combined with, for example, ARICEPT® (donepezil HCl), EXELON® (rivastigmine tartrate), RAZADYNE® (galantamine HBr), steroids, anakinra (KINARET®, Amgen) or canakinumab. The IL-1 traps of the invention may also be combined with anti-IL-18 drugs, such as for example, IL-18BP or a derivative, an IL-18 Trap, anti-IL-18, anti-IL-18R1, or anti-IL-18Racp. Other co-therapies include aspirin or other NSAIDs, steroids such as prednisolone, other inflammatory inhibitors such as inhibitors of caspase-1, p38, IKK1/2, CTLA-4lg, anti-IL-6 or anti-IL6Ra, an antibody specific for tau, an antibody specific for beta amyloid (such as solanezumab, gantenerumab, or bapineuzumab) or a microtubule stabilizers (such as epothilone B).

### Administration Regimens

According to certain embodiments of the present invention, multiple doses of the IL-1 trap may be administered to a subject over a defined time course. The methods comprise sequentially administering to a subject multiple doses of the IL-1 trap of the invention. As used herein, "sequentially administering" means that each dose of the IL-1 trap is administered to the subject at a different point in time, *e.g*., on different days separated by a predetermined interval (*e.g*., hours, days, weeks or months). The present invention includes the IL-1 antagonist provided for use in methods, which comprise sequentially administering to the patient a single initial dose of the IL-1 trap, followed by one or more secondary doses of the IL-1 trap, and optionally followed by one or more tertiary doses of the IL-1 trap.

The terms "initial dose," "secondary doses," and "tertiary doses," refer to the temporal sequence of administration of an IL-1 trap of the invention. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the IL-1 trap, but generally may differ from one another in terms of frequency of administration. In certain embodiments, however, the amount of the IL-1 trap contained in the initial, secondary and/or tertiary doses varies from one another (*e.g*., adjusted up or down as appropriate) during the course of treatment. In certain embodiments, two or more (*e.g*., 2, 3, 4, or 5) doses are administered at the beginning of the treatment regimen as "loading doses" followed by subsequent doses that are administered on a less frequent basis (*e.g*., "maintenance doses").

In one exemplary embodiment of the present invention, each secondary and/or tertiary dose is administered ½ to 26 (*e.g*., ½, 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of the IL-1 trap, which is administered to a patient prior to the administration of the very next dose in the sequence with no intervening doses.

The methods may comprise administering to a patient any number of secondary and/or tertiary doses of the IL-1 trap. For example, in certain embodiments, only a single secondary dose is administered to the patient. In other instances, two or more (*e.g*., 2, 3, 4, 5, 6, 7, 8, or more) secondary doses are administered to the patient. Likewise, in certain instances, only a single tertiary dose is administered to the patient. In other instances, two or more (*e.g*., 2, 3, 4, 5, 6, 7, 8, or more) tertiary doses are administered to the patient.

In instances involving multiple secondary doses, each secondary dose may be administered at the same frequency as the other secondary doses. For example, each secondary dose may be administered to the patient 1 to 2 weeks after the immediately preceding dose. Similarly, in instances involving multiple tertiary doses, each tertiary dose may be administered at the same frequency as the other tertiary doses. For example, each tertiary dose may be administered to the patient 2 to 4 weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

### Pharmaceutical Compositions

The present invention also provides pharmaceutical compositions comprising the IL-1 antagonist. Such compositions comprise a therapeutically effective amount of an active agent, and a pharmaceutically acceptable carrier. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin.

In one embodiment, the composition is formulated in accordance with routine procedures as a pharmaceutical composition adapted for intravenous administration to human beings. Where necessary, the composition may also include a solubilizing agent and a local anesthetic such as lidocaine to ease pain at the site of the injection. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The active agents of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the active agent of the invention which will be effective in the treatment of Alzheimer's disease can be determined by standard clinical techniques based on the present description. In addition, *in vitro* assays may optionally be employed to help identify optimal dosage ranges. The precise dose to be employed in the formulation will also depend on the route of administration, and the seriousness of the condition, and should be decided according to the judgment of the practitioner and each subject's circumstances. However, suitable dosage ranges for intravenous administration are generally about 20 micrograms to 2 grams of active compound per kilogram body weight. Suitable dosage ranges for intra-nasal administration are generally about 0.01 pg/kg body weight to 1 mg/kg body weight. Effective doses may be extrapolated from dose-response curves derived from *in vitro* or animal model test systems.

For systemic administration, a therapeutically effective dose can be estimated initially from *in vitro* assays. For example, a dose can be formulated in animal models to achieve a circulating concentration range that includes the IC₅₀ as determined in cell culture. Such information can be used to more accurately determine useful doses in humans. Initial dosages can also be estimated from *in vivo* data, e.g., animal models, using techniques that are well known in the art. One having ordinary skill in the art could readily optimize administration to humans based on animal data.

Dosage amount and interval may be adjusted individually to provide plasma levels of the compounds that are sufficient to maintain therapeutic effect. In cases of local administration or selective uptake, the effective local concentration of the compounds may not be related to plasma concentration. One having skill in the art will be able to optimize therapeutically effective local dosages without undue experimentation.

The amount of compound administered will, of course, be dependent on the subject being treated, on the subject's weight, the severity of the affliction, the manner of administration, and the judgment of the prescribing physician. The therapy may be repeated intermittently while symptoms are detectable or even when they are not detectable. The therapy may be provided alone or in combination with other drugs.

### Kits

Also disclosed is an article of manufacturing comprising packaging material and a pharmaceutical agent contained within the packaging material, wherein the pharmaceutical agent comprises at least one IL-1-specific fusion protein of the invention and wherein the packaging material comprises a label or package insert which indicates that the IL-1-specific fusion protein can be used for treating a disease characterized by aberrant deposition of beta amyloid, such as Alzheimer's disease.

Other features of the invention will become apparent in the course of the following descriptions of exemplary embodiments which are given for illustration of the invention and are not intended to be limiting thereof.

### EXAMPLES

The following example is put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### EXAMPLE 1: Effect of an IL-1 Antagonist (IL-1 Trap) in a Mouse Model of Alzheimer's-Like Pathology

### Methods

### Animals and Injections:

Subjects were 40 male mice split into two cohorts of 20. Within each cohort, ten mice were wild type (WT) animals and ten were tandem transgenic (Tg) for both the Swedish pedigree mutation in the amyloid precursor protein (SwAPP) and the high Alzheimer's risk polymorphism in presenilin-1 (PS-1). Both the wild type controls and the transgenic mice were of the C57BI/6 background. The mice were housed in a temperature-stabilized facility on a 12:12 light:dark cycle (lights on 07:00), with food and water available *ad libitum.*

Starting at approximately 8 months of age, animals were administered biweekly injections of mouse IL-1 Trap or control mFc subcutaneously. Five Tg animals and five WT animals of each cohort received mIL-1 Trap at a dose of 10 mg/kg. The rest of the mice received mouse Fc (mFc) at the same dose and volume. Mice were weighed weekly in order to establish dosage and evaluate animal health. The injections continued for 5 months. Three mice died before completing the behavioral testing phase (one WT animal receiving mFc, one TG animal receiving mFc and one TG animal receiving mIL-1 Trap.)

### Behavioral testing:

*Morris Water Maze:* During the fifth month of injections, the Morris Water Maze Test was conducted to evaluate spatial learning and memory. A pool measuring 105 cm in diameter and 35cm in depth was filled with water made opaque with non-toxic white paint. The pool was then divided conceptually into four quadrants, one of which contained an escape platform hidden 2.5 cm beneath the surface. Animals were placed in a different, pseudo-randomly selected quadrant at the start of each trial, and the latency to escape the maze onto the hidden platform was measured. There were two trial blocks per day for five days, each consisting of three one-minute trials. Between trials, animals were held with a towel for approximately 30 seconds before being placed back in the water. After each block, they were placed in a holding cage lined with towels until dry and then returned to their home cages. The testing was conducted at the same time each day with a 3-hour interval between the first and second daily blocks. Any animal not locating the platform within one minute was assigned a latency of 60 seconds and guided to the platform by hand before being removed from the water. Normal animals were expected to demonstrate a decrease in latency to escape across trials in the water maze, indicating acquisition of the location of the platform over time.

One hour after the final block of water maze acquisition testing, animals were returned to the maze for a 30 second probe trial with the platform removed to test their retention of the platform's location. Retention was assessed via a measure of the amount of time animals spent in the quadrant that formerly housed the platform, termed the "goal quadrant." The number of times the animals swam over the spot where the platform was located was also counted. Animals with normal retention are expected to spend more time in the "goal quadrant" than in other quadrants and to make more "platform crosses", thus demonstrating retention of the spatial location of the escape platform.

For each block, the animals' median latency to escape the maze across the three trials was recorded and these median latencies were used for statistical analysis. In addition, animals' swim speeds were estimated by dividing latency to escape by the number of maze quadrants crossed per trial to calculate mean quadrant crossing time. The median quadrant crossing time for each trial block was used as a covariate in the statistical analysis to account for potential differences in motor speed.

### Open Field:

During the fifth month, the open field test was conducted in order to explore some of the non-cognitive behavioral symptoms of Alzheimer's-like disease and whether IL-1 inhibition has an effect on those symptoms. Specifically, open field can be used to measure general locomotor and exploratory activity (Walsh, R. N. & Cummins, R. A. (1976), Psychological Bulletin, 83(3), 482-504). Animals were placed into a white, box-like apparatus measuring 48 x 48 x 24 cm. The inside floor was divided into nine grids, each measuring 16 x 16 cm. Animals were placed in the center grid and allowed to freely explore the apparatus for a six minute trial. The number of total grid crossings was recorded as a measure of general locomotor and exploratory behavior.

### Tissue Collection and Processing:

After 5 months of treatment, the animals were sacrificed and brains were prepared for histological analysis. All animals were overdosed with a pentobarbital-based euthanasia solution and perfusion fixed. Cold heparinized isotonic (0.9%) saline was run through the body to exsanguinate the animal and animals were then perfused with 4% paraformaldehyde first in acetate, and then borate buffer (100ml each). Upon completion of the perfusion, brains were removed and placed in 30% buffered sucrose for 3-7 days. The fixed brains were sectioned coronally at 50µm and stored in cryoprotectant (Watson, R. E., Wiegand, S. J., Clough, R. W., & Hoffman, G. E. (1986), Peptides, 7(1), 155-159) at -20°C until they were stained.

### Histology:

Brain tissue from the animals was used for histological analysis. Sections were stained in a 1:12 series with Congo Red to detect the presence of amyloid plaques and with cresyl violet for Nissl bodies in separate sections for visualization of all cells. Immunostaining was conducted to visualize the microglial marker Iba-1 (Millipore rabbit polyclonal anti-lba-1, 1:500). For the second cohort of animals, a double-stain was conducted for both plaques and microglia in order to evaluate parameters of microglia at various distances from plaques.

### Image Analysis:

Amyloid plaque burden was analyzed by contrast analysis using the ImageJ image analysis software program (NIH). A minimum of 2 bilateral sections of hippocampus in a 1:12 series were selected and images were captured in 10x using the PictureFrame program. In each picture, the red color of the stain was isolated and the background faded using Adobe Photoshop to achieve contrast. Identical processing parameters were used for all sections. The entire image was then converted to black and white and imported to Image J. For each image, a set percentage of the background was removed from all images and the image was converted to binary. The percentage of area stained, number of separate plaques, and average size per plaque were recorded for each animal.

The level of overall inflammation was assessed by subjective ratings. An experienced histologist blind to the experimental conditions examined cresyl violet-stained hippocampal sections at a magnification of 40x. Each animal was given a rating of "none," "mild," "moderate," or "marked," based on the presence of inflammatory cell profiles (microglia, immune cells), with "none" signifying no inflammatory cells and "severe" being the highest amount of inflammation observed. The presence of microglial-invested deposits was also noted. For each animal showing plaque-like deposits, 5 random deposits were selected from the hippocampus and the surrounding microglia-like cells were counted. The median number of microglial profiles per plaque was recorded for each animal. Finally, double stains for congo red-positive plaques and Iba-1-immunoreactive microglia were used to measure the sizes of microglia contacting plaques (contact), within 20 microns of a plaque (adjacent), or greater than 30 microns from a plaque (distant).

### Statistical Analysis

For water maze acquisition, the median latency to escape to platform was recorded for each animal for each trial block. A three-way mixed Factorial analysis of variance (ANOVA) was conducted using treatment and genotype as the between groups independent variables and block as a repeated measure. For retention, the proportion of time spent in the goal quadrant was recorded for each animal along with the number of platform crosses. Two-way ANOVAs (genotype x treatment) were conducted with each of these as dependent variables.

To evaluate locomotor activity and exploratory behavior, the number of grid crossings in the open field test was counted for each animal. A two-way factorial ANOVA was conducted using treatment and genotype as the between groups independent variables.

Two-way ANOVAs (genotype x treatment) were conducted for each of the following measures: amyloid plaque total area, plaque number, hippocampal volume, microglial size, and average plaque size. For these measures, values for individual hemispheres were averaged to calculate a mean for each animal. Finally, regression analyses were conducted to assess the predictive relationship between memory performance and plaque pathology for each treatment group (mFc and mIL-1 Trap).

A non-parametric log-linear analysis for qualitative variables was conducted to analyze the overall inflammation ratings. An independent groups t-test was conducted to compare number of microglia-like cells surrounding hippocampal deposits in mFc-treated transgenic mice versus mIL-1-treated trap transgenic mice.

Data are presented as mean across all animals within a treatment/genotype group and standard error of the mean (SEM). Statistical significance was set at an alpha level of 0.05.

### Results

### General Health:

Mice were weighed and evaluated weekly to assess their general health. Out of the original 40 animals, 37 survived to sacrifice. In the first cohort, one transgenic animal in the mFc-treated group died before the behavioral testing stage. From the second cohort, one transgenic animal in the mIL-1 Trap group died before the behavioral testing stage and one wild type animal in the mIL-1 Trap group died after completing some behavioral testing. At the final weighing before perfusion, the transgenic mice weighed about 27% less than the wild type mice (*F*_{(1,33)}=55.46, *p<.001).* Although the transgenic mice weighed less, they were actually closer to the normal body weight for adult male mice, and appeared grossly healthy. The mIL-1 Trap had no negative impact on body weights (*F*_{(1,33)} =0.00, *p*=.994) and the mice all seemed healthy throughout the study.

### Water Maze:

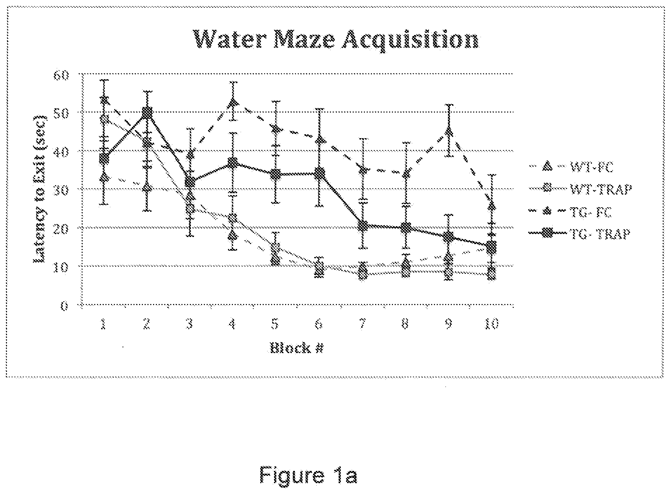

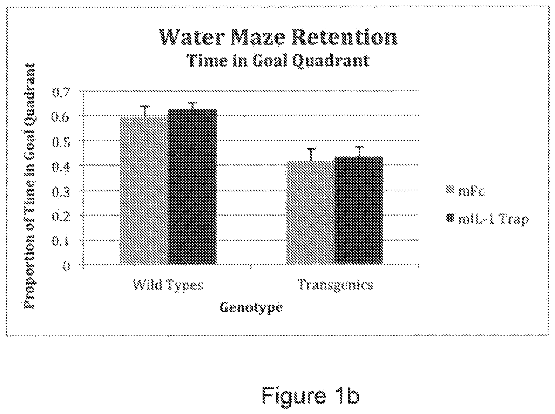

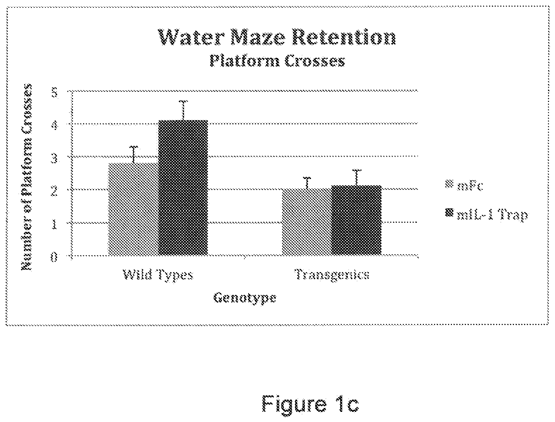

As expected, a three-way mixed Factorial ANOVA (genotype x treatment x trial block) showed that the wild type animals performed significantly better overall than the swAPP/PS-1 double transgenic animals in water maze acquisition (*F*_{(1,33)}=29.71, *p*<.001). The mIL-1 Trap had no significant overall effect on water maze performance (*F*_{(1,33)}=2.76, *p*=0.11), but there was a significant interaction between the two factors (*F*_{(1,33)}=4.61, *p*=.039; Figure 1), such that the trap selectively improved performance in the transgenic animals. An analysis of cohort effects revealed a significant cohort by genotype interaction, such that the transgenic animals of the second cohort performed worse overall than those in the first cohort. However, there was no cohort by treatment effect and no cohort by genotype by treatment effect, indicating that the trap affected the mice similarly across both cohorts.

We also assessed whether differences in swim speed could be influencing these results. Slower swim speeds have been reported in transgenic AD mice (Ying, T., Xu, Y., Scearce-Levie, K., Ptacek, L. J., & Fu, Y.H. (2010), Neurogenetics, 11(1), 41-52), which can interfere with an animal's latency to swim to the platform. A three-way mixed Factorial ANOVA (genotype x treatment x trial block) revealed no significant differences in quadrant crossing time across genotype (F_{(1,33)}=.61, p=.442) or treatment (F_{(1,33)}=1.13, p=.296), and no genotype by treatment interaction (F_{(1,33)}=.47, p=.50). In addition, we did not see any significant general locomotor differences in the open field test (see Open Field section), suggesting that motor differences cannot account for the acquisition results observed.

On the retention portion of the water maze, wild type animals spent significantly more time in the goal quadrant (*F*_{(1,33)}=18.83, *p*<.001) and made significantly more platform crosses (*F*_{(1,33)}=8.86, *p*=0.005) than transgenic animals, confirming the memory impairment in the transgenics. There was no significant effect of IL-1 Trap treatment on proportion of time spent in the goal quadrant (*F*_{(1,33)}=.313, *p*=.58) and there was no interaction between the factors (F_{(1,33)}=.04, p=.84) (Figure 1b). There was also no significant effect of treatment on number of platform crosses (F_{(1,33)}=2.29, p=.14) and no interaction between treatment and genotype (F_{(1,33)}=.1.63, p=.21) (Figure 1c).

### Open Field

A two-way ANOVA (genotype x treatment) was conducted to examine differences in locomotor activity and exploratory behavior. Overall, there was no difference in total number of grid crossings between wild type and transgenic animals (F_{(1,33)}=.699, p=.409), and no difference between animals treated with mFc and those treated with mll-1 Trap (F_{(1,33)}=.18, p=.673). There was no interaction between genotype and treatment (F_{(1,33)}=.03, p=.873). These results indicate that transgenic mice did not differ in overall level of locomotor activity compared to wild type mice and that level of activity was not affected by treatment with mIL-1 trap.

### Plaque Burden Analysis

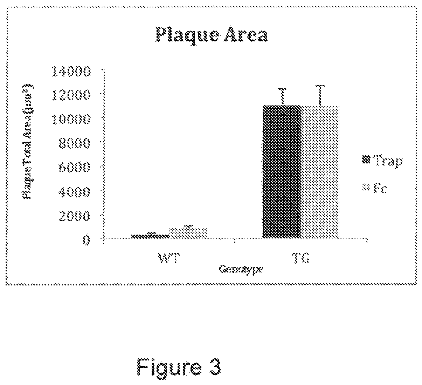

As expected, the plaque burden contrast analysis revealed that swAPP/PS-1 transgenic mice had significantly more plaques (*F*(1,13)=34.21, *p<.001),* more total area covered by plaques (*F*(1,13)=31.04, *p<.001),* and a higher average plaque size (*F*(1,13)=7.43, *p*=.02) than the wild type animals. Indeed, no Congo red-positive plaques were observed in any wild type animal (data not shown). There was no significant difference between animals given mIL-1 Trap and mFc on any of these measures, and no interactions between genotype and treatment (Figure 3).

We also examined the relationship between plaque burden and spatial learning acquisition on block 5 (halfway through learning) and block 10 (final performance block) of water maze acquisition. For animals given mFc, there was a significant positive correlation between total plaque area and latency to escape on block 5 (*r*(6)=.90, *p*=.002), such that animals with more plaque coverage took longer to find the platform during the learning trial. The correlation remained significant when wild type animals, which had no plaques, were excluded from analysis (*r*(2)=.96, *p*=.04). A regression analysis could not be conducted to regress plaques against learning in the wild type animals, because no plaques were detected in any of these animals. The mIL-1 Trap animals had a smaller, non-significant correlation between plaque burden and learning performance (*r*(7)=.46, *p*=.21), which disappeared completely when wild type animals were excluded from analysis (*r*(3)=.004, *p*=.99), suggesting that mIL-1 Trap eliminated the association between plaque burden and cognitive performance in the swAPP/PS-1 transgenic mice. By block 10, when animals had reached peak performance, the amount of plaque coverage was no longer significantly correlated with water maze performance for either treatment group. Sample sizes were too small to determine whether the differences in correlations between the two genotypes were statistically significant.

### Inflammation

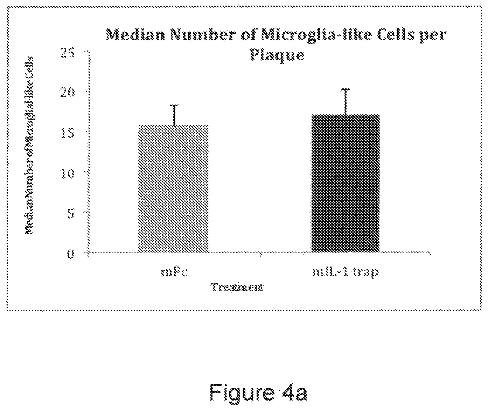

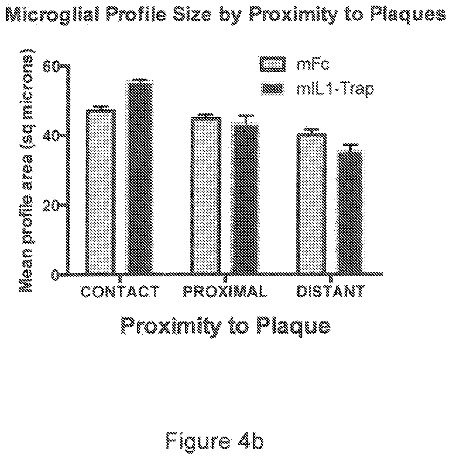

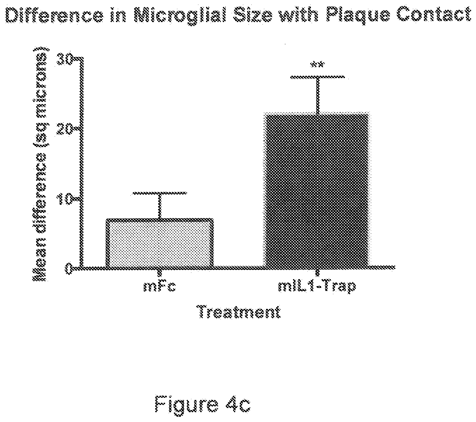

A log-linear analysis of overall subjective inflammation ratings did not reveal a significant difference between mFc and mIL-1 Trap-treated animals (*G*²(4)=5.22, *p*=.26) (Table 1). An independent groups t-test was conducted on the measure of the overall number of microglia-like cells surrounding the plaque-like deposits in the hippocampi of transgenic animals, and there was no significant difference in microglial profile counts between the Fc and mIL-1 Trap-treated animals. (*t*₍₇₎= -.30, *p*=.78) (Figure 4a). However, an analysis of microglial profile size showed a statistically significant change in microglial size with IL-1 Trap treatment interacting with distance from plaques (Figure 4b, interaction F(2,12)= 16.127, p=0.0004). Specifically, while microglial sizes were larger for microglia contacting plaques in all animals, the differences in average size for plaques contacting microglia were significantly greater in IL-1 Trap-treated animals. To better illustrate this effect, the difference between the mean size of microglia in unaffected tissue versus plaque-containing tissue for each animal was calculated. This difference in microglial size per animal showed a statistically significant effect of IL-1 Trap on enhancement in microglial size upon plaque contact (Figure 4c, t(6)=4.864, p=0.0028).

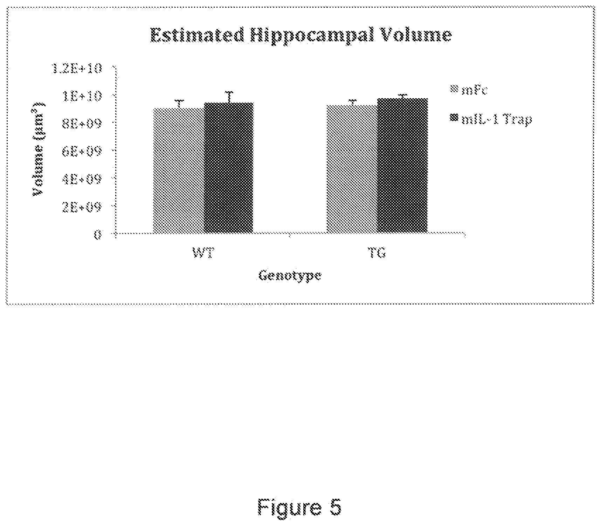

In addition, as shown in Figure 5, this model of disease produces no significant effect on overall hippocampal volume (F(1,13)=0.236, p=0.635), nor does IL-1 Trap impact hippocampal volume in either the wild type or transgenic mice (treatment F(1,13)=0.800, p=0.387; interaction F(1,13)=0.015, p-0.903).

**Table 1: Distribution of Overall Inflammation Ratings**

| | **WT** | | | |
|---|---|---|---|---|
| | **None** | **Mild** | **Moderate** | **Marked** |
| **mFc** | 40% | 60% | 0% | 0% |
| **mIL-1 Trap** | 20% | 20% | 60% | 0% |
| | | | | |

| | **TG** | | | |
|---|---|---|---|---|
| | **None** | **Mild** | **Moderate** | **Marked** |
| **mFc** | 0% | 25% | 25% | 50% |
| **mIL-1 Trap** | 20% | 50% | 20% | 20% |

### Discussion

Chronic neuroinflammation, which includes elevated IL-1 expression, is a prominent feature of Alzheimer's disease, which may contribute to the neurodegeneration and associated cognitive dysfunction observed in patients with Alzheimer's Disease. In this study, systemic administration of the mIL-1 Trap was used to inhibit interleukin-1 signaling for 5 months after the onset of disease in order to observe its effects on both behavior and Alzheimer's-like brain pathology. The study was done to determine if IL-1 inhibition would improve performance on measures of learning and memory while reducing amyloid plaque burden. Although the mIL-1 Trap did improve water maze performance in transgenic mice, it did not significantly alter amyloid plaque burden. It did, however, increase the size of microglia contacting amyloid plaques while decreasing the size of microglia overall in the brain, suggesting that although it didn't alter the amount of beta-amyloid deposited in the transgenic brains, it may have altered the nature of the immune response triggered by the plaques. In addition, mIL-1 Trap completely eliminated the significant positive relationship between plaque burden and cognitive impairments, showing that although the plaques were present, they were no longer associated with cognitive impairments in the animals.

### Water maze

Our finding that the mIL-1 Trap selectively improved water maze performance for transgenic animals shows that IL-1 inhibition can slow the cognitive decline resulting from overexpression of mutant Presenilin-1 and the Swedish APP mutation. In the present study, IL-1 inhibition improved acquisition (e.g. learning) while having no effect on retention (e.g. memory), showing that the treated mice may have used compensatory strategies to assist with their performance of the task.

### Open Field

The open field test is used to evaluate locomotor and exploratory behaviors in rodents. Results in swAPP-PS1 transgenic mice showed no significant difference in locomotor activity compared to wild type mice in a six-minute open field test. The lack of significant differences between groups on both open field and on water maze swim speed suggest that differences in learning and memory performance cannot be attributed to alterations in motor or exploratory behavior.

### Amyloid Plaques

In addition to the behavioral testing as a measure of Alzheimer's like pathology, a Congo Red stain was performed for the detection of amyloid plaques. We hypothesized that chronic IL-1 inhibition would result in reduced amyloid plaque pathology. However, the mIL-1 Trap did not significantly decrease hippocampal plaque burden in the transgenic animals.

The water maze data taken together with the plaque analysis provides an interesting look at the nature of AD-like pathology in the swAPP-PS1 transgenic mice. If, as our data indicate, the Trap did improve spatial memory in these mice without reducing the amount of amyloid plaques in the brain, it would imply that factors other than β-amyloid deposition are contributing to their cognitive deficits. One explanation is that the mIL-1 Trap may be improving memory by inhibiting the immune response to the plaques without reducing the plaques themselves.

To further evaluate the role of amyloid plaques, we looked at the relationship between plaque deposits and water maze performance for transgenic animals treated with mFc and for animals treated with mIL-1 Trap. In this study, transgenic AD animals given mFc showed a very strong significant correlation (r=.96) between plaque burden and water maze acquisition performance during spatial memory learning (block 5), such that animals with more amyloid deposition took longer to find the platform. However, animals given mIL-1 Trap showed no correlation (r=.004) between plaque burden and the same water maze measure. The fact that these results were obtained with such small sample sizes is especially promising, although further studies with larger sample size are warranted. If the mIL-1 Trap, which suppresses part of the neuroinflammatory response, actually eliminates the relationship between amyloid plaques and memory, it would support the hypothesis that it is the immune response to the plaques, rather than the plaques themselves, that are causing the memory impairments.

### Inflammation

In order to probe the possibility that mIL-1 Trap changed the immune response to the plaques, a subjective analysis of the overall level of inflammation around the plaques in the hippocampi of the transgenic animals was conducted. The initial analysis involved the assignment of an overall subjective rating of peri-plaque inflammation by an experienced histologist blind to animal treatment. This analysis did not reveal any significant overall differences in inflammatory cell investment around the plaques. In support of this finding, a quantitative count of one specific inflammatory cell type, the resident brain macrophages (microglia), revealed no difference in overall microglial count per plaque. However, a statistical trend was observed toward a significant difference in microglial size between transgenic animals treated with IL-1 Trap versus mFc control. The differences that were observed included a shift toward smaller microglia at rest, but larger microglia contacting plaques. Although more research will be necessary to fully interpret this trend, one possibility is that microglia are less activated basally in IL-1 Trap treated animals, but are more phagocytic when confronted with pathological deposits. While we do not have direct evidence that IL-1 Trap changed the activation subtype of microglia, this pattern of sizes may indicate that more M2 than M1 microglia were present in the brains of IL-1 Trap-treated animals. M2 microglia tend to be less activated overall, but have more potential for phagocytosis.

It is possible that the inhibition of interleukin 1 in the present study is affecting some of the detrimental microglial processes, without improving their plaque-clearing ability. Further morphometric and neurochemical analysis will help determine the full extent of changes in microglial activation and morphology.

### Summary

The current study showed a potentially protective role of systemic mIL-1 Trap treatment in the swAPP/PS-1 double transgenic model of Alzheimer's Disease. In particular, the current study provides support for the growing hypothesis that amyloid plaques do not, in themselves, underlie the hallmark cognitive impairments of Alzheimer's Disease. Indeed, it supports the idea that components of the inflammatory cascade, perhaps triggered in part by the presence of the plaques, are major pathogenic contributors. In addition, our data provide initial proof of concept for the potential use of IL-1 inhibition to treat the cognitive impairments of Alzheimer's Disease. Finally, our data show that even large biological inhibitors of IL-1, such as the IL-1 traps described herein, given after disease onset, could provide significant benefit to patients suffering from the profound cognitive impairments characterizing this devastating disease.

## Claims

1. An IL-1 antagonist for use in a method for treating, or delaying the onset, or the progression of a disease **characterized in** part by beta amyloid expression, activity, or deposition in a subject in need thereof, the method comprising administering to the subject a therapeutically effective amount of an IL-1 antagonist as a first therapeutic agent, wherein the IL-1 antagonist is an IL-1 trap, wherein the IL-1 trap is a fusion protein comprising the amino acid sequence of SEQ ID NO: 10.

2. The IL-1 antagonist for use in the method according to claim 1, wherein the subject is a human.

3. The IL-1 antagonist for use in the method of claim 1 or 2, wherein the disease is selected from the group consisting of Alzheimer's disease (AD), Down's syndrome, multi-infarct dementia, cognitive impairment and cerebral amyloid angiopathy (CAA).

4. The IL-1 antagonist for use in the method of claim 3, wherein the Alzheimer's disease is clinical, pre-clinical or prodromal Alzheimer's disease or the cerebral amyloid angiopathy is clinical or pre-clinical cerebral amyloid angiopathy.

5. The IL-1 antagonist for use in the method of any one of the preceding claims, wherein administration is subcutaneous, intramuscular, intranasal, intraarterial, intravenous, intrathecal, intraventricular, intracerebral, topical, transdermal administration or oral.

6. The IL-1 antagonist for use in the method of any of any one of the preceding claims, wherein a therapeutically effective amount is between:
(a) 1 mg/kg to 750 mg/kg;
(b) 10 mg/kg to 500 mg/kg; or
(c) 50 mg/kg to 150 mg/kg.

7. The IL-1 antagonist for use in the method of any one of the preceding claims, further comprising administering to a subject in need thereof a therapeutically effective amount of one or more other therapeutic agents, wherein the disease is lessened in severity or duration, or wherein the onset or progression of the disease is delayed.

8. The IL-1 antagonist for use in the method of claim 7, wherein administration of the other therapeutic agent is subcutaneous, intramuscular, intranasal, intraarterial, intravenous, intrathecal, intraventricular, intracerebral, topical, transdermal administration or oral.

9. The IL-1 antagonist for use in the method of claim 7, wherein the other therapeutic agent is:
(a) an acetylcholinesterase inhibitor or a glutamate pathway modifier; or
(b) from the group consisting of a different IL-1 antagonist, an anti-inflammatory agent, an antibody specific for tau, an antibody specific for beta amyloid and a microtubule stabilizer.

10. The IL-1 antagonist for use in the method of claim 9, wherein:
(a) the acetylcholinesterase inhibitor is selected from the group consisting of ARICEPT® (donepezil HCI), EXELON® (rivastigmine tartrate), and RAZADYNE® (galantamine HBr);
(b) the glutamate pathway modifier is Namenda (memantine); or
(c) the different IL-1 antagonist is selected from the group consisting of an IL-1 alpha or IL-1 beta antibody, a soluble IL-1 receptor, a different IL-1 trap, anakinra and canakinumab;
(d) the anti-inflammatory agent is aspirin or a different NSAID;
(e) the antibody specific for beta amyloid is selected from the group consisting of solanezumab, gantenerumab, and bapineuzumab; or
(f) the microtubule stabilizer is epothilone.

## Patentansprüche

1. IL-1-Antagonist zur Verwendung in einem Verfahren zum Behandeln oder Verzögern des Ausbruchs oder der Fortschreitung einer Krankheit, gekennzeichnet teilweise durch Beta-Amyloid-Expression, -Aktivität oder -Ablagerung bei einem Subjekt, das dessen bedarf, wobei das Verfahren ein Verabreichen einer therapeutisch wirksamen Menge eines IL-1-Antagonisten als ein erstes Therapeutikum an das Subjekt umfasst, wobei der IL-1-Antagonist eine IL-1-Falle (IL-1 trap) ist, wobei die IL-1-Falle ein Fusionsprotein ist, das die Aminosäuresequenz von SEQ ID NO: 10 umfasst.

2. IL-1-Antagonist zur Verwendung in dem Verfahren nach Anspruch 1, wobei das Subjekt ein Mensch ist.

3. IL-1-Antagonist zur Verwendung in dem Verfahren nach Anspruch 1 oder 2, wobei die Krankheit ausgewählt ist aus der Gruppe bestehend aus Alzheimer-Krankheit (AD), Down-Syndrom, Multiinfarktdemenz, kognitiver Beeinträchtigung und zerebraler Amyloidangiopathie (cerebral amyloid angiopathy, CAA).

4. IL-1-Antagonist zur Verwendung in dem Verfahren nach Anspruch 3, wobei die Alzheimer-Krankheit klinische, präklinische oder prodromale Alzheimer-Krankheit ist oder die zerebrale Amyloidangiopathie klinische oder präklinische zerebrale Amyloidangiopathie ist.

5. IL-1-Antagonist zur Verwendung in dem Verfahren nach einem der vorhergehenden Ansprüche, wobei eine Verabreichung subkutane, intramuskuläre, intranasale, intraarterielle, intravenöse, intrathekale, intraventrikuläre, intrazerebrale, topische, transdermale Verabreichung oder oral ist.

6. IL-1-Antagonist zur Verwendung in dem Verfahren nach einem nach einem der vorhergehenden Ansprüche, wobei eine therapeutisch wirksame Menge zwischen Folgendem liegt:
(a) 1 mg/kg bis 750 mg/kg;
(b) 10 mg/kg bis 500 mg/kg; oder
(c) 50 mg/kg bis 150 mg/kg.

7. IL-1-Antagonist zur Verwendung in dem Verfahren nach einem der vorhergehenden Ansprüche, ferner umfassend ein Verabreichen einer therapeutisch wirksamen Menge von einem oder mehreren anderen Therapeutika an ein Subjekt, das deren bedarf, wobei die Krankheit in Schwere oder Dauer verringert wird oder wobei der Ausbruch oder die Fortschreitung der Krankheit verzögert wird.

8. IL-1-Antagonist zur Verwendung in dem Verfahren nach Anspruch 7, wobei eine Verabreichung des anderen Therapeutikums subkutane, intramuskuläre, intranasale, intraarterielle, intravenöse, intrathekale, intraventrikuläre, intrazerebrale, topische, transdermale Verabreichung oder oral ist.

9. IL-1-Antagonist zur Verwendung in dem Verfahren nach Anspruch 7, das andere Therapeutikum Folgendes ist:
(a) ein Acetylcholinesterase-Hemmer oder ein Glutamat-Weg-Modifikator; oder
(b) aus der Gruppe bestehend aus einem anderen IL-1-Antagonisten, einem entzündungshemmenden Mittel, einem für Tau spezifischen Antikörper, einem für Beta-Amyloid spezifischen Antikörper oder einem Mikrotubuli-Stabilisator.

10. IL-1-Antagonist zur Verwendung in dem Verfahren nach Anspruch 9, wobei:
(a) der Acetylcholinesterase-Hemmer ausgewählt ist aus der Gruppe bestehend aus ARICEPT® (Donepezil HCl), EXELON® (Rivastigmin-tartrat) und RAZADYNE® (Galantamin HBr);
(b) der Glutamat-Weg-Modifikator Namenda (Memantin) ist; oder
(c) der andere IL-1-Antagonist ausgewählt ist aus der Gruppe bestehend aus einem IL-1-Alpha- oder IL-1-Beta-Antikörper, einem löslichen IL-1-Rezeptor, einer anderen IL-1-Falle, Anakinra und Canakinumab;
(d) das entzündungshemmende Mittel Aspirin oder ein anderes NSAID ist;
(e) der für Beta-Amyloid spezifische Antikörper ausgewählt ist aus der Gruppe bestehend aus Solanezumab, Gantenerumab und Bapineuzumab ist; oder
(f) der Mikrotubuli-Stabilisator Epothilon ist.

## Revendications

1. Antagoniste IL-1 destiné à une utilisation dans une méthode de traitement, ou de retardement de l'apparition ou de l'évolution d'une maladie caractérisé en partie par l'expression, l'activité ou le dépôt de bêta-amyloïde chez un sujet en ayant besoin, la méthode comprenant l'administration au sujet d'une quantité thérapeutiquement efficace d'un antagoniste IL-1 comme premier agent thérapeutique, l'antagoniste IL-1 étant un piège à IL-1, le piège à IL-1 étant une protéine de fusion comprenant la séquence d'acides aminés de la SEQ ID n° : 10.

2. Antagoniste IL-1 destiné à une utilisation dans la méthode selon la revendication 1, dans lequel le sujet est un être humain.

3. Antagoniste IL-1 destiné à une utilisation dans la méthode selon la revendication 1 ou 2, dans lequel la maladie est choisie dans le groupe constitué par la maladie de Alzheimer (AD), la trisomie 21, la démence par infarctus multiples, la déficience cognitive et l'amylose cérébrovasculaire (CAA).

4. Antagoniste IL-1 destiné à une utilisation dans la méthode selon la revendication 3, dans lequel la maladie de Alzheimer est la maladie de Alzheimer clinique, préclinique ou prodromique ou l'amylose cérébrovasculaire est l'amylose cérébrovasculaire clinique ou préclinique.

5. Antagoniste IL-1 destiné à une utilisation dans la méthode selon l'une quelconque des revendications précédentes, dans lequel l'administration est une administration sous-cutanée, intramusculaire, intranasale, intraartérielle, intraveineuse, intrathécale, intraventriculaire, intracérébrale, topique, transdermique ou orale.

6. Antagoniste IL-1 destiné à une utilisation dans la méthode selon l'une quelconque des revendications précédentes, dans lequel une quantité thérapeutiquement efficace se situe entre :
(a) 1 mg/kg et 750 mg/kg ;
(b) 10 mg/kg et 500 mg/kg ; ou
(c) 50 mg/kg et 150 mg/kg.

7. Antagoniste IL-1 destiné à une utilisation dans la méthode selon l'une quelconque des revendications précédentes, comprenant en outre l'administration à un sujet en ayant besoin d'une quantité thérapeutiquement efficace d'au moins un agent thérapeutique, la gravité ou la durée de la maladie étant atténuée, ou l'apparition ou l'évolution de la maladie étant retardée.

8. Antagoniste IL-1 destiné à une utilisation dans la méthode selon la revendication 7, dans lequel l'administration de l'autre agent thérapeutique est une administration sous-cutanée, intramusculaire, intranasale, intraartérielle, intraveineuse, intrathécale, intraventriculaire, intracérébrale, topique, transdermique ou orale.

9. Antagoniste IL-1 destiné à une utilisation dans la méthode selon la revendication 7, dans lequel l'autre agent thérapeutique est :
(a) un inhibiteur d'acétylcholinestérase ou un modificateur de trajet de glutamate ; ou
(b) parmi le groupe constitué par un antagoniste IL-1 différent, un agent anti-inflammatoire, un anticorps spécifique de Tau, un anticorps spécifique de la bêta-amyloïde et un stabilisateur de microtubule.

10. Antagoniste IL-1 destiné à une utilisation dans la méthode selon la revendication 9, dans lequel :
(a) l'inhibiteur d'acétylcholinestérase est choisi dans le groupe constitué par ARICEPT® (donépézil HCI), EXELON® (tartrate de rivastigmine) et RAZADYNE® (galantamine HBr) ;
(b) le modificateur de trajet de glutamate est Namenda (mémantine) ; ou
(c) l'antagoniste IL-1 différent est choisi dans le groupe constitué par un anticorps IL-1 alpha ou IL-1 bêta, un récepteur de l'IL-1 soluble, un piège à IL-1 différent, anakinra et canakinumab ;
(d) l'agent anti-inflammatoire est l'aspirine ou un anti-inflammatoire non stéroïdien (AINS) différent ;
(e) l'anticorps spécifique de la bêta-amyloïde est choisi dans le groupe constitué par solanezumab, ganténérumab et bapineuzumab ;
(f) le stabilisateur de microtubule est l'épothilone.


### SEQUENCE LISTING

<110> Regeneron Pharmaceuticals, Inc
<120> METHODS OF USING IL-1 ANTAGONISTS TO TREAT ALZHEIMER'S DISEASE
<130> 5070WO
<140> TBA
   <141> 2014-05-30
<150> 61/829,713
   <151> 2013-05-31
<160> 28
<170> FastSEQ for Windows Version 4.0
<210> 1
   <211> 2734
   <212> DNA
   <213> Artificial Sequence
<220>
   <223> Synthetic
<400> 1
   datggtgctt ctgtggtgtg tagtgagtct ctacttttat ggaatcctgc aaagtgatgc 60
   ctcagaacgc tgcgatgact ggggactaga caccatgagg caaatccaag tgtttgaaga 120
   tgagccagct cgcatcaagt gcccactctt tgaacacttc ttgaaattca actacagcac 180
   agcccattca gctggcctta ctctgatctg gtattggact aggcaggacc gggaccttga 240
   ggagccaatt aacttccgcc tccccgagaa ccgcattagt aaggagaaag atgtgctgtg 300
   gttccggccc actctcctca atgacactgg caactatacc tgcatgttaa ggaacactac 360
   atattgcagc aaagttgcat ttcccttgga agttgttcaa aaagacagct gtttcaattc 420
   ccccatgaaa ctcccagtgc ataaactgta tatagaatat ggcattcaga ggatcacttg 480
   tccaaatgta gatggatatt ttccttccag tgtcaaaccg actatcactt ggtatatggg 540
   ctgttataaa atacagaatt ttaataatgt aatacccgaa ggtatgaact tgagtttcct 600
   cattgcctta atttcaaata atggaaatta cacatgtgtt gttacatatc cagaaaatgg 660
   acgtacgttt catctcacca ggactctgac tgtaaaggta gtaggctctc caaaaaatgc 720
   agtgccccct gtgatccatt cacctaatga tcatgtggtc tatgagaaag aaccaggaga 780
   ggagctactc attccctgta cggtctattt tagttttctg atggattctc gcaatgaggt 840
   ttggtggacc attgatggaa aaaaacctga tgacatcact attgatgtca ccattaacga 900
   aagtataagt catagtagaa cagaagatga aacaagaact cagattttga gcatcaagaa 960
   agttacctct gaggatctca agcgcagcta tgtctgtcat gctagaagtg ccaaaggcga 1020
   agttgccaaa gcagccaagg tgaagcagaa agtgccagct ccaagataca cagtgtccgg 1080
   tggcgcgcct atgctgagcg aggctgataa atgcaaggaa cgtgaagaaa aaataatttt 1140
   agtgtcatct gcaaatgaaa ttgatgttcg tccctgtcct cttaacccaa atgaacacaa 1200
   aggcactata acttggtata aggatgacag caagacacct gtatctacag aacaagcctc 1260
   caggattcat caacacaaag agaaactttg gtttgttcct gctaaggtgg aggattcagg 1320
   acattactat tgcgtggtaa gaaattcatc ttactgcctc agaattaaaa taagtgcaaa 1380
   atttgtggag aatgagccta acttatgtta taatgcacaa gccatattta agcagaaact 1440
   acccgttgca ggagacggag gacttgtgtg cccttatatg gagtttttta aaaatgaaaa 1500
   taatgagtta cctaaattac agtggtataa ggattgcaaa cctctacttc ttgacaatat 1560
   acactttagt ggagtcaaag ataggctcat cgtgatgaat gtggctgaaa agcatagagg 1620
   gaactatact tgtcatgcat cctacacata cttgggcaag caatatccta ttacccgggt 1680
   aatagaattt attactctag aggaaaacaa acccacaagg cctgtgattg tgagcccagc 1740
   taatgagaca atggaagtag acttgggatc ccagatacaa ttgatctgta atgtcaccgg 1800
   ccagttgagt gacattgctt actggaagtg gaatgggtca gtaattgatg aagatgaccc 1860
   agtgctaggg gaagactatt acagtgtgga aaatcctgca aacaaaagaa ggagtaccct 1920
   catcacagtg cttaatatat cggaaattga gagtagattt tataaacatc catttacctg 1980
   ttttgccaag aatacacatg gtatagatgc agcatatatc cagttaatat atccagtcac 2040
   taattccgga gacaaaactc acacatgccc accgtgccca gcacctgaac tcctgggggg 2100
   accgtcagtc ttcctcttcc ccccaaaacc caaggacacc ctcatgatct cccggacccc 2160
   tgaggtcaca tgcgtggtgg tggacgtgag ccacgaagac cctgaggtca agttcaactg 2220
   gtacgtggac ggcgtggagg tgcataatgc caagacaaag ccgcgggagg agcagtacaa 2280
   cagcacgtac cgtgtggtca gcgtcctcac cgtcctgcac caggactggc tgaatggcaa 2340
   ggagtacaag tgcaaggtct ccaacaaagc cctcccagcc cccatcgaga aaaccatctc 2400
   caaagccaaa gggcagcccc gagaaccaca ggtgtacacc ctgcccccat cccgggagga 2460
   gatgaccaag aaccaggtca gcctgacctg cctggtcaaa ggcttctatc ccagcgacat 2520
   cgccgtggag tgggagagca atgggcagcc ggagaacaac tacaagacca cgcctcccgt 2580
   gctggactcc gacggctcct tcttcctcta tagcaagctc accgtggaca agagcaggtg 2640
   gcagcagggg aacgtcttct catgctccgt gatgcatgag gctctgcaca accactacac 2700
   gcagaagagc ctctccctgt ctccgggtaa atga                            2734
<210> 2
   <211> 911
   <212> PRT
   <213> Artificial Sequence
<220>
   <223> Synthetic
<400> 2
   Pro Met Val Leu Leu Trp Cys Val Val Ser Leu Tyr Phe Tyr Gly Ile
    1               5                  10                  15
   Leu Gln Ser Asp Ala Ser Glu Arg Cys Asp Asp Trp Gly Leu Asp Thr
               20                  25                  30
   Met Arg Gln Ile Gln Val Phe Glu Asp Glu Pro Ala Arg Ile Lys Cys
           35                  40                  45
   Pro Leu Phe Glu His Phe Leu Lys Phe Asn Tyr Ser Thr Ala His Ser
       50                  55                  60
   Ala Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
               100                 105                 110
   Tyr Thr Cys Met Leu Arg Asn Thr Thr Tyr Cys Ser Lys Val Ala Phe
           115                 120                 125
   Pro Leu Glu Val Val Gln Lys Asp Ser Cys Phe Asn Ser Pro Met Lys
       130                 135                 140
   Leu Pro Val His Lys Leu Tyr Ile Glu Tyr Gly Ile Gln Arg Ile Thr
   145                 150                 155                 160
   Cys Pro Asn Val Asp Gly Tyr Phe Pro Ser Ser Val Lys Pro Thr Ile
                   165                 170                 175
   Thr Trp Tyr Met Gly Cys Tyr Lys Ile Gln Asn Phe Asn Asn Val Ile
               180                 185                 190
   Pro Glu Gly Met Asn Leu Ser Phe Leu Ile Ala Leu Ile Ser Asn Asn
           195                 200                 205
   Gly Asn Tyr Thr Cys Val Val Thr Tyr Pro Glu Asn Gly Arg Thr Phe
       210                 215                 220
   His Leu Thr Arg Thr Leu Thr Val Lys Val Val Gly Ser Pro Lys Asn
   225                 230                 235                 240
   Ala Val Pro Pro Val Ile His Ser Pro Asn Asp His Val Val Tyr Glu
                   245                 250                 255
   Lys Glu Pro Gly Glu Glu Leu Leu Ile Pro Cys Thr Val Tyr Phe Ser
               260                 265                 270
   Phe Leu Met Asp Ser Arg Asn Glu Val Trp Trp Thr Ile Asp Gly Lys
           275                 280                 285
   Lys Pro Asp Asp Ile Thr Ile Asp Val Thr Ile Asn Glu Ser Ile Ser
       290                 295                 300
   His Ser Arg Thr Glu Asp Glu Thr Arg Thr Gln Ile Leu Ser Ile Lys
   305                 310                 315                 320
   Lys Val Thr Ser Glu Asp Leu Lys Arg Ser Tyr Val Cys His Ala Arg
                   325                 330                 335
   Ser Ala Lys Gly Glu Val Ala Lys Ala Ala Lys Val Lys Gln Lys Val
               340                 345                 350
   Pro Ala Pro Arg Tyr Thr Val Ser Gly Gly Ala Pro Met Leu Ser Glu
           355                 360                 365
   Ala Asp Lys Cys Lys Glu Arg Glu Glu Lys Ile Ile Leu Val Ser Ser
       370                 375                 380
   Ala Asn Glu Ile Asp Val Arg Pro Cys Pro Leu Asn Pro Asn Glu His
   385                 390                 395                 400
   Lys Gly Thr Ile Thr Trp Tyr Lys Asp Asp Ser Lys Thr Pro Val Ser
                   405                 410                 415
   Thr Glu Gln Ala Ser Arg Ile His Gln His Lys Glu Lys Leu Trp Phe
               420                 425                 430
   Val Pro Ala Lys Val Glu Asp Ser Gly His Tyr Tyr Cys Val Val Arg
           435                 440                 445
   Asn Ser Ser Tyr Cys Leu Arg Ile Lys Ile Ser Ala Lys Phe Val Glu
       450                 455                 460
   Asn Glu Pro Asn Leu Cys Tyr Asn Ala Gln Ala Ile Phe Lys Gln Lys
   465                 470                 475                 480
   Leu Pro Val Ala Gly Asp Gly Gly Leu Val Cys Pro Tyr Met Glu Phe
                   485                 490                 495
   Phe Lys Asn Glu Asn Asn Glu Leu Pro Lys Leu Gln Trp Tyr Lys Asp
               500                 505                 510
   Cys Lys Pro Leu Leu Leu Asp Asn Ile His Phe Ser Gly Val Lys Asp
           515                 520                 525
   Arg Leu Ile Val Met Asn Val Ala Glu Lys His Arg Gly Asn Tyr Thr
       530                 535                 540
   Cys His Ala Ser Tyr Thr Tyr Leu Gly Lys Gln Tyr Pro Ile Thr Arg
   545                 550                 555                 560
   Val Ile Glu Phe Ile Thr Leu Glu Glu Asn Lys Pro Thr Arg Pro Val
                   565                 570                 575
   Ile Val Ser Pro Ala Asn Glu Thr Met Glu Val Asp Leu Gly Ser Gln
               580                 585                 590
   Ile Gln Leu Ile Cys Asn Val Thr Gly Gln Leu Ser Asp Ile Ala Tyr
           595                 600                 605
   Trp Lys Trp Asn Gly Ser Val Ile Asp Glu Asp Asp Pro Val Leu Gly
       610                 615                 620
   Glu Asp Tyr Tyr Ser Val Glu Asn Pro Ala Asn Lys Arg Arg Ser Thr
   625                 630                 635                 640
   Leu Ile Thr Val Leu Asn Ile Ser Glu Ile Glu Ser Arg Phe Tyr Lys
                   645                 650                 655
   His Pro Phe Thr Cys Phe Ala Lys Asn Thr His Gly Ile Asp Ala Ala
               660                 665                 670
   Tyr Ile Gln Leu Ile Tyr Pro Val Thr Asn Ser Gly Asp Lys Thr His
           675                 680                 685
   Thr Cys Pro Pro Cys Pro Ala Pro Glu Leu Leu Gly Gly Pro Ser Val
       690                 695                 700
   Phe Leu Phe Pro Pro Lys Pro Lys Asp Thr Leu Met Ile Ser Arg Thr
   705                 710                 715                 720
   Pro Glu Val Thr Cys Val Val Val Asp Val Ser His Glu Asp Pro Glu
                   725                 730                 735
   Val Lys Phe Asn Trp Tyr Val Asp Gly Val Glu Val His Asn Ala Lys
               740                 745                 750
   Thr Lys Pro Arg Glu Glu Gln Tyr Asn Ser Thr Tyr Arg Val Val Ser
           755                 760                 765
   Val Leu Thr Val Leu His Gln Asp Trp Leu Asn Gly Lys Glu Tyr Lys
       770                 775                 780
   Cys Lys Val Ser Asn Lys Ala Leu Pro Ala Pro Ile Glu Lys Thr Ile
   785                 790                 795                 800
   Ser Lys Ala Lys Gly Gln Pro Arg Glu Pro Gln Val Tyr Thr Leu Pro
                   805                 810                 815
   Pro Ser Arg Glu Glu Met Thr Lys Asn Gln Val Ser Leu Thr Cys Leu
               820                 825                 830
   Val Lys Gly Phe Tyr Pro Ser Asp Ile Ala Val Glu Trp Glu Ser Asn
           835                 840                 845
   Gly Gln Pro Glu Asn Asn Tyr Lys Thr Thr Pro Pro Val Leu Asp Ser
       850                 855                 860
   Asp Gly Ser Phe Phe Leu Tyr Ser Lys Leu Thr Val Asp Lys Ser Arg
   865                 870                 875                 880
   Trp Gln Gln Gly Asn Val Phe Ser Cys Ser Val Met His Glu Ala Leu
                   885                 890                 895
   His Asn His Tyr Thr Gln Lys Ser Leu Ser Leu Ser Pro Gly Lys
               900                 905                 910
<210> 3
   <211> 2704
   <212> DNA
   <213> Artificial Sequence
<220>
   <223> Synthetic
<400> 3
   datggtgtta ctcagactta tttgtttcat agctctactg atttcttctc tggaggctga 60
   taaatgcaag gaacgtgaag aaaaaataat tttagtgtca tctgcaaatg aaattgatgt 120
   tcgtccctgt cctcttaacc caaatgaaca caaaggcact ataacttggt ataaggatga 180
   cagcaagaca cctgtatcta cagaacaagc ctccaggatt catcaacaca aagagaaact 240
   ttggtttgtt cctgctaagg tggaggattc aggacattac tattgcgtgg taagaaattc 300
   atcttactgc ctcagaatta aaataagtgc aaaatttgtg gagaatgagc ctaacttatg 360
   ttataatgca caagccatat ttaagcagaa actacccgtt gcaggagacg gaggacttgt 420
   gtgcccttat atggagtttt ttaaaaatga aaataatgag ttacctaaat tacagtggta 480
   taaggattgc aaacctctac ttcttgacaa tatacacttt agtggagtca aagataggct 540
   catcgtgatg aatgtggctg aaaagcatag agggaactat acttgtcatg catcctacac 600
   atacttgggc aagcaatatc ctattacccg ggtaatagaa tttattactc tagaggaaaa 660
   caaacccaca aggcctgtga ttgtgagccc agctaatgag acaatggaag tagacttggg 720
   atcccagata caattgatct gtaatgtcac cggccagttg agtgacattg cttactggaa 780
   gtggaatggg tcagtaattg atgaagatga cccagtgcta ggggaagact attacagtgt 840
   ggaaaatcct gcaaacaaaa gaaggagtac cctcatcaca gtgcttaata tatcggaaat 900
   tgagagtaga ttttataaac atccatttac ctgttttgcc aagaatacac atggtataga 960
   tgcagcatat atccagttaa tatatccagt cactaattca gaacgctgcg atgactgggg 1020
   actagacacc atgaggcaaa tccaagtgtt tgaagatgag ccagctcgca tcaagtgccc 1080
   actctttgaa cacttcttga aattcaacta cagcacagcc cattcagctg gccttactct 1140
   gatctggtat tggactaggc aggaccggga ccttgaggag ccaattaact tccgcctccc 1200
   cgagaaccgc attagtaagg agaaagatgt gctgtggttc cggcccactc tcctcaatga 1260
   cactggcaac tatacctgca tgttaaggaa cactacatat tgcagcaaag ttgcatttcc 1320
   cttggaagtt gttcaaaaag acagctgttt caattccccc atgaaactcc cagtgcataa 1380
   actgtatata gaatatggca ttcagaggat cacttgtcca aatgtagatg gatattttcc 1440
   ttccagtgtc aaaccgacta tcacttggta tatgggctgt tataaaatac agaattttaa 1500
   taatgtaata cccgaaggta tgaacttgag tttcctcatt gccttaattt caaataatgg 1560
   aaattacaca tgtgttgtta catatccaga aaatggacgt acgtttcatc tcaccaggac 1620
   tctgactgta aaggtagtag gctctccaaa aaatgcagtg ccccctgtga tccattcacc 1680
   taatgatcat gtggtctatg agaaagaacc aggagaggag ctactcattc cctgtacggt 1740
   ctattttagt tttctgatgg attctcgcaa tgaggtttgg tggaccattg atggaaaaaa 1800
   acctgatgac atcactattg atgtcaccat taacgaaagt ataagtcata gtagaacaga 1860
   agatgaaaca agaactcaga ttttgagcat caagaaagtt acctctgagg atctcaagcg 1920
   cagctatgtc tgtcatgcta gaagtgccaa aggcgaagtt gccaaagcag ccaaggtgaa 1980
   gcagaaagtg ccagctccaa gatacacagt ggaatccgga gacaaaactc acacatgccc 2040
   accgtgccca gcacctgaac tcctgggggg accgtcagtc ttcctcttcc ccccaaaacc 2100
   caaggacacc ctcatgatct cccggacccc tgaggtcaca tgcgtggtgg tggacgtgag 2160
   ccacgaagac cctgaggtca agttcaactg gtacgtggac ggcgtggagg tgcataatgc 2220
   caagacaaag ccgcgggagg agcagtacaa cagcacgtac cgtgtggtca gcgtcctcac 2280
   cgtcctgcac caggactggc tgaatggcaa ggagtacaag tgcaaggtct ccaacaaagc 2340
   cctcccagcc cccatcgaga aaaccatctc caaagccaaa gggcagcccc gagaaccaca 2400
   ggtgtacacc ctgcccccat cccgggagga gatgaccaag aaccaggtca gcctgacctg 2460
   cctggtcaaa ggcttctatc ccagcgacat cgccgtggag tgggagagca atgggcagcc 2520
   ggagaacaac tacaagacca cgcctcccgt gctggactcc gacggctcct tcttcctcta 2580
   tagcaagctc accgtggaca agagcaggtg gcagcagggg aacgtcttct catgctccgt 2640
   gatgcatgag gctctgcaca accactacac gcagaagagc ctctccctgt ctccgggtaa 2700
   atga 2704
<210> 4
   <211> 901
   <212> PRT
   <213> Artificial Sequence
<220>
   <223> Synthetic
<400> 4
   Pro Met Val Leu Leu Arg Leu Ile Cys Phe Ile Ala Leu Leu Ile Ser
    1               5                  10                  15
   Ser Leu Glu Ala Asp Lys Cys Lys Glu Arg Glu Glu Lys Ile Ile Leu
               20                  25                  30
   Val Ser Ser Ala Asn Glu Ile Asp Val Arg Pro Cys Pro Leu Asn Pro
           35                  40                  45
   Asn Glu His Lys Gly Thr Ile Thr Trp Tyr Lys Asp Asp Ser Lys Thr
       50                  55                  60
   Pro Val Ser Thr Glu Gln Ala Ser Arg Ile His Gln His Lys Glu Lys
   65                  70                  75                  80
   Leu Trp Phe Val Pro Ala Lys Val Glu Asp Ser Gly His Tyr Tyr Cys
                   85                  90                  95
   Val Val Arg Asn Ser Ser Tyr Cys Leu Arg Ile Lys Ile Ser Ala Lys
               100                 105                 110
   Phe Val Glu Asn Glu Pro Asn Leu Cys Tyr Asn Ala Gln Ala Ile Phe
           115                 120                 125
   Lys Gln Lys Leu Pro Val Ala Gly Asp Gly Gly Leu Val Cys Pro Tyr
       130                 135                 140
   Met Glu Phe Phe Lys Asn Glu Asn Asn Glu Leu Pro Lys Leu Gln Trp
   145                 150                 155                 160
   Tyr Lys Asp Cys Lys Pro Leu Leu Leu Asp Asn Ile His Phe Ser Gly
                   165                 170                 175
   Val Lys Asp Arg Leu Ile Val Met Asn Val Ala Glu Lys His Arg Gly
               180                 185                 190
   Asn Tyr Thr Cys His Ala Ser Tyr Thr Tyr Leu Gly Lys Gln Tyr Pro
           195                 200                 205
   Ile Thr Arg Val Ile Glu Phe Ile Thr Leu Glu Glu Asn Lys Pro Thr
       210                 215                 220
   Arg Pro Val Ile Val Ser Pro Ala Asn Glu Thr Met Glu Val Asp Leu
   225                 230                 235                 240
   Gly Ser Gln Ile Gln Leu Ile Cys Asn Val Thr Gly Gln Leu Ser Asp
                   245                 250                 255
   Ile Ala Tyr Trp Lys Trp Asn Gly Ser Val Ile Asp Glu Asp Asp Pro
               260                 265                 270
   Val Leu Gly Glu Asp Tyr Tyr Ser Val Glu Asn Pro Ala Asn Lys Arg
           275                 280                 285
   Arg Ser Thr Leu Ile Thr Val Leu Asn Ile Ser Glu Ile Glu Ser Arg
       290                 295                 300
   Phe Tyr Lys His Pro Phe Thr Cys Phe Ala Lys Asn Thr His Gly Ile
   305                 310                 315                 320
   Asp Ala Ala Tyr Ile Gln Leu Ile Tyr Pro Val Thr Asn Ser Glu Arg
                   325                 330                 335
   Cys Asp Asp Trp Gly Leu Asp Thr Met Arg Gln Ile Gln Val Phe Glu
               340                 345                 350
   Asp Glu Pro Ala Arg Ile Lys Cys Pro Leu Phe Glu His Phe Leu Lys
           355                 360                 365
   Phe Asn Tyr Ser Thr Ala His Ser Ala Gly Leu Thr Leu Ile Trp Tyr
       370                 375                 380
   Trp Thr Arg Gln Asp Arg Asp Leu Glu Glu Pro Ile Asn Phe Arg Leu
   385                 390                 395                 400
   Pro Glu Asn Arg Ile Ser Lys Glu Lys Asp Val Leu Trp Phe Arg Pro
                   405                 410                 415
   Thr Leu Leu Asn Asp Thr Gly Asn Tyr Thr Cys Met Leu Arg Asn Thr
               420                 425                 430
   Thr Tyr Cys Ser Lys Val Ala Phe Pro Leu Glu Val Val Gln Lys Asp
           435                 440                 445
   Ser Cys Phe Asn Ser Pro Met Lys Leu Pro Val His Lys Leu Tyr Ile
       450                 455                 460
   Glu Tyr Gly Ile Gln Arg Ile Thr Cys Pro Asn Val Asp Gly Tyr Phe
   465                 470                 475                 480
   Pro Ser Ser Val Lys Pro Thr Ile Thr Trp Tyr Met Gly Cys Tyr Lys
                   485                 490                 495
   Ile Gln Asn Phe Asn Asn Val Ile Pro Glu Gly Met Asn Leu Ser Phe
               500                 505                 510
   Leu Ile Ala Leu Ile Ser Asn Asn Gly Asn Tyr Thr Cys Val Val Thr
           515                 520                 525
   Tyr Pro Glu Asn Gly Arg Thr Phe His Leu Thr Arg Thr Leu Thr Val
       530                 535                 540
   Lys Val Val Gly Ser Pro Lys Asn Ala Val Pro Pro Val Ile His Ser
   545                 550                 555                 560
   Pro Asn Asp His Val Val Tyr Glu Lys Glu Pro Gly Glu Glu Leu Leu
                   565                 570                 575
   Ile Pro Cys Thr Val Tyr Phe Ser Phe Leu Met Asp Ser Arg Asn Glu
               580                 585                 590
   Val Trp Trp Thr Ile Asp Gly Lys Lys Pro Asp Asp Ile Thr Ile Asp
           595                 600                 605
   Val Thr Ile Asn Glu Ser Ile Ser His Ser Arg Thr Glu Asp Glu Thr
       610                 615                 620
   Arg Thr Gln Ile Leu Ser Ile Lys Lys Val Thr Ser Glu Asp Leu Lys
   625                 630                 635                 640
   Arg Ser Tyr Val Cys His Ala Arg Ser Ala Lys Gly Glu Val Ala Lys
                   645                 650                 655
   Ala Ala Lys Val Lys Gln Lys Val Pro Ala Pro Arg Tyr Thr Val Glu
               660                 665                 670
   Ser Gly Asp Lys Thr His Thr Cys Pro Pro Cys Pro Ala Pro Glu Leu
           675                 680                 685
   Leu Gly Gly Pro Ser Val Phe Leu Phe Pro Pro Lys Pro Lys Asp Thr
       690                 695                 700
   Leu Met Ile Ser Arg Thr Pro Glu Val Thr Cys Val Val Val Asp Val
   705                 710                 715                 720
   Ser His Glu Asp Pro Glu Val Lys Phe Asn Trp Tyr Val Asp Gly Val
                   725                 730                 735
   Glu Val His Asn Ala Lys Thr Lys Pro Arg Glu Glu Gln Tyr Asn Ser
               740                 745                 750
   Thr Tyr Arg Val Val Ser Val Leu Thr Val Leu His Gln Asp Trp Leu
           755                 760                 765
   Asn Gly Lys Glu Tyr Lys Cys Lys Val Ser Asn Lys Ala Leu Pro Ala
       770                 775                 780
   Pro Ile Glu Lys Thr Ile Ser Lys Ala Lys Gly Gln Pro Arg Glu Pro
   785                 790                 795                 800
   Gln Val Tyr Thr Leu Pro Pro Ser Arg Glu Glu Met Thr Lys Asn Gln
                   805                 810                 815
   Val Ser Leu Thr Cys Leu Val Lys Gly Phe Tyr Pro Ser Asp Ile Ala
               820                 825                 830
   Val Glu Trp Glu Ser Asn Gly Gln Pro Glu Asn Asn Tyr Lys Thr Thr
           835                 840                 845
   Pro Pro Val Leu Asp Ser Asp Gly Ser Phe Phe Leu Tyr Ser Lys Leu
       850                 855                 860
   Thr Val Asp Lys Ser Arg Trp Gln Gln Gly Asn Val Phe Ser Cys Ser
   865                 870                 875                 880
   Val Met His Glu Ala Leu His Asn His Tyr Thr Gln Lys Ser Leu Ser
                   885                 890                 895
   Leu Ser Pro Gly Lys
               900
<210> 5
   <211> 2710
   <212> DNA
   <213> Artificial Sequence
<220>
   <223> Synthetic
<400> 5
   datggtgtta ctcagactta tttgtttcat agctctactg atttcttctc tggaggctga 60
   taaatgcaag gaacgtgaag aaaaaataat tttagtgtca tctgcaaatg aaattgatgt 120
   tcgtccctgt cctcttaacc caaatgaaca caaaggcact ataacttggt ataaggatga 180
   cagcaagaca cctgtatcta cagaacaagc ctccaggatt catcaacaca aagagaaact 240
   ttggtttgtt cctgctaagg tggaggattc aggacattac tattgcgtgg taagaaattc 300
   atcttactgc ctcagaatta aaataagtgc aaaatttgtg gagaatgagc ctaacttatg 360
   ttataatgca caagccatat ttaagcagaa actacccgtt gcaggagacg gaggacttgt 420
   gtgcccttat atggagtttt ttaaaaatga aaataatgag ttacctaaat tacagtggta 480
   taaggattgc aaacctctac ttcttgacaa tatacacttt agtggagtca aagataggct 540
   catcgtgatg aatgtggctg aaaagcatag agggaactat acttgtcatg catcctacac 600
   atacttgggc aagcaatatc ctattacccg ggtaatagaa tttattactc tagaggaaaa 660
   caaacccaca aggcctgtga ttgtgagccc agctaatgag acaatggaag tagacttggg 720
   atcccagata caattgatct gtaatgtcac cggccagttg agtgacattg cttactggaa 780
   gtggaatggg tcagtaattg atgaagatga cccagtgcta ggggaagact attacagtgt 840
   ggaaaatcct gcaaacaaaa gaaggagtac cctcatcaca gtgcttaata tatcggaaat 900
   tgagagtaga ttttataaac atccatttac ctgttttgcc aagaatacac atggtataga 960
   tgcagcatat atccagttaa tatatccagt cactaattca gaacgctgcg atgactgggg 1020
   actagacacc atgaggcaaa tccaagtgtt tgaagatgag ccagctcgca tcaagtgccc 1080
   actctttgaa cacttcttga aattcaacta cagcacagcc cattcagctg gccttactct 1140
   gatctggtat tggactaggc aggaccggga ccttgaggag ccaattaact tccgcctccc 1200
   cgagaaccgc attagtaagg agaaagatgt gctgtggttc cggcccactc tcctcaatga 1260
   cactggcaac tatacctgca tgttaaggaa cactacatat tgcagcaaag ttgcatttcc 1320
   cttggaagtt gttcaaaaag acagctgttt caattccccc atgaaactcc cagtgcataa 1380
   actgtatata gaatatggca ttcagaggat cacttgtcca aatgtagatg gatattttcc 1440
   ttccagtgtc aaaccgacta tcacttggta tatgggctgt tataaaatac agaattttaa 1500
   taatgtaata cccgaaggta tgaacttgag tttcctcatt gccttaattt caaataatgg 1560
   aaattacaca tgtgttgtta catatccaga aaatggacgt acgtttcatc tcaccaggac 1620
   tctgactgta aaggtagtag gctctccaaa aaatgcagtg ccccctgtga tccattcacc 1680
   taatgatcat gtggtctatg agaaagaacc aggagaggag ctactcattc cctgtacggt 1740
   ctattttagt tttctgatgg attctcgcaa tgaggtttgg tggaccattg atggaaaaaa 1800
   acctgatgac atcactattg atgtcaccat taacgaaagt ataagtcata gtagaacaga 1860
   agatgaaaca agaactcaga ttttgagcat caagaaagtt acctctgagg atctcaagcg 1920
   cagctatgtc tgtcatgcta gaagtgccaa aggcgaagtt gccaaagcag ccaaggtgaa 1980
   gcagaaagtg ccagctccaa gatacacagt ggaatccgga gagtccaaat acggtccgcc 2040
   atgcccatca tgcccagcac ctgagttcct ggggggacca tcagtcttcc tgttcccccc 2100
   aaaacccaag gacactctca tgatctcccg gacccctgag gtcacgtgcg tggtggtgga 2160
   cgtgagccag gaagaccccg aggtccagtt caactggtac gtggatggcg tggaggtgca 2220
   taatgccaag acaaagccgc gggaggagca gttcaacagc acgtaccgtg tggtcagcgt 2280
   cctcaccgtc ctgcaccagg actggctgaa cggcaaggag tacaagtgca aggtctccaa 2340
   caaaggcctc ccgtcctcca tcgagaaaac catctccaaa gccaaagggc agccccgaga 2400
   gccacaggtg tacaccctgc ccccatccca ggaggagatg accaagaacc aggtcagcct 2460
   gacctgcctg gtcaaaggct tctaccccag cgacatcgcc gtggagtggg agagcaatgg 2520
   gcagccggag aacaactaca agaccacgcc tcccgtgctg gactccgacg gctccttctt 2580
   cctctacagc aggctaaccg tggacaagag caggtggcag gaggggaatg tcttctcatg 2640
   ctccgtgatg catgaggctc tgcacaacca ctacacacag aagagcctct ccctgtctct 2700
   gggtaaatga 2710
<210> 6
   <211> 903
   <212> PRT
   <213> Artificial Sequence
<220>
   <223> Synthetic
<400> 6
   Pro Met Val Leu Leu Arg Leu Ile Cys Phe Ile Ala Leu Leu Ile Ser
    1               5                  10                  15
   Ser Leu Glu Ala Asp Lys Cys Lys Glu Arg Glu Glu Lys Ile Ile Leu
               20                  25                  30
   Val Ser Ser Ala Asn Glu Ile Asp Val Arg Pro Cys Pro Leu Asn Pro
           35                  40                  45
   Asn Glu His Lys Gly Thr Ile Thr Trp Tyr Lys Asp Asp Ser Lys Thr
       50                  55                  60
   Pro Val Ser Thr Glu Gln Ala Ser Arg Ile His Gln His Lys Glu Lys
   65                  70                  75                  80
   Leu Trp Phe Val Pro Ala Lys Val Glu Asp Ser Gly His Tyr Tyr Cys
                   85                  90                  95
   Val Val Arg Asn Ser Ser Tyr Cys Leu Arg Ile Lys Ile Ser Ala Lys
               100                 105                 110
   Phe Val Glu Asn Glu Pro Asn Leu Cys Tyr Asn Ala Gln Ala Ile Phe
           115                 120                 125
   Lys Gln Lys Leu Pro Val Ala Gly Asp Gly Gly Leu Val Cys Pro Tyr
       130                 135                 140
   Met Glu Phe Phe Lys Asn Glu Asn Asn Glu Leu Pro Lys Leu Gln Trp
   145                 150                 155                 160
   Tyr Lys Asp Cys Lys Pro Leu Leu Leu Asp Asn Ile His Phe Ser Gly
                   165                 170                 175
   Val Lys Asp Arg Leu Ile Val Met Asn Val Ala Glu Lys His Arg Gly
               180                 185                 190
   Asn Tyr Thr Cys His Ala Ser Tyr Thr Tyr Leu Gly Lys Gln Tyr Pro
           195                 200                 205
   Ile Thr Arg Val Ile Glu Phe Ile Thr Leu Glu Glu Asn Lys Pro Thr
       210                 215                 220
   Arg Pro Val Ile Val Ser Pro Ala Asn Glu Thr Met Glu Val Asp Leu
   225                 230                 235                 240
   Gly Ser Gln Ile Gln Leu Ile Cys Asn Val Thr Gly Gln Leu Ser Asp
                   245                 250                 255
   Ile Ala Tyr Trp Lys Trp Asn Gly Ser Val Ile Asp Glu Asp Asp Pro
               260                 265                 270
   Val Leu Gly Glu Asp Tyr Tyr Ser Val Glu Asn Pro Ala Asn Lys Arg
           275                 280                 285
   Arg Ser Thr Leu Ile Thr Val Leu Asn Ile Ser Glu Ile Glu Ser Arg
       290                 295                 300
   Phe Tyr Lys His Pro Phe Thr Cys Phe Ala Lys Asn Thr His Gly Ile
   305                 310                 315                 320
   Asp Ala Ala Tyr Ile Gln Leu Ile Tyr Pro Val Thr Asn Ser Glu Arg
                   325                 330                 335
   Cys Asp Asp Trp Gly Leu Asp Thr Met Arg Gln Ile Gln Val Phe Glu
               340                 345                 350
   Asp Glu Pro Ala Arg Ile Lys Cys Pro Leu Phe Glu His Phe Leu Lys
           355                 360                 365
   Phe Asn Tyr Ser Thr Ala His Ser Ala Gly Leu Thr Leu Ile Trp Tyr
       370                 375                 380
   Trp Thr Arg Gln Asp Arg Asp Leu Glu Glu Pro Ile Asn Phe Arg Leu
   385                 390                 395                 400
   Pro Glu Asn Arg Ile Ser Lys Glu Lys Asp Val Leu Trp Phe Arg Pro
                   405                 410                 415
   Thr Leu Leu Asn Asp Thr Gly Asn Tyr Thr Cys Met Leu Arg Asn Thr
               420                 425                 430
   Thr Tyr Cys Ser Lys Val Ala Phe Pro Leu Glu Val Val Gln Lys Asp
           435                 440                 445
   Ser Cys Phe Asn Ser Pro Met Lys Leu Pro Val His Lys Leu Tyr Ile
       450                 455                 460
   Glu Tyr Gly Ile Gln Arg Ile Thr Cys Pro Asn Val Asp Gly Tyr Phe
   465                 470                 475                 480
   Pro Ser Ser Val Lys Pro Thr Ile Thr Trp Tyr Met Gly Cys Tyr Lys
                   485                 490                 495
   Ile Gln Asn Phe Asn Asn Val Ile Pro Glu Gly Met Asn Leu Ser Phe
               500                 505                 510
   Leu Ile Ala Leu Ile Ser Asn Asn Gly Asn Tyr Thr Cys Val Val Thr
           515                 520                 525
   Tyr Pro Glu Asn Gly Arg Thr Phe His Leu Thr Arg Thr Leu Thr Val
       530                 535                 540
   Lys Val Val Gly Ser Pro Lys Asn Ala Val Pro Pro Val Ile His Ser
   545                 550                 555                 560
   Pro Asn Asp His Val Val Tyr Glu Lys Glu Pro Gly Glu Glu Leu Leu
                   565                 570                 575
   Ile Pro Cys Thr Val Tyr Phe Ser Phe Leu Met Asp Ser Arg Asn Glu
               580                 585                 590
   Val Trp Trp Thr Ile Asp Gly Lys Lys Pro Asp Asp Ile Thr Ile Asp
           595                 600                 605
   Val Thr Ile Asn Glu Ser Ile Ser His Ser Arg Thr Glu Asp Glu Thr
       610                 615                 620
   Arg Thr Gln Ile Leu Ser Ile Lys Lys Val Thr Ser Glu Asp Leu Lys
   625                 630                 635                 640
   Arg Ser Tyr Val Cys His Ala Arg Ser Ala Lys Gly Glu Val Ala Lys
                   645                 650                 655
   Ala Ala Lys Val Lys Gln Lys Val Pro Ala Pro Arg Tyr Thr Val Glu
               660                 665                 670
   Ser Gly Glu Ser Lys Tyr Gly Pro Pro Cys Pro Ser Cys Pro Ala Pro
           675                 680                 685
   Glu Phe Leu Gly Gly Pro Ser Val Phe Leu Phe Pro Pro Lys Pro Lys
       690                 695                 700
   Asp Thr Leu Met Ile Ser Arg Thr Pro Glu Val Thr Cys Val Val Val
   705                 710                 715                 720
   Asp Val Ser Gln Glu Asp Pro Glu Val Gln Phe Asn Trp Tyr Val Asp
                   725                 730                 735
   Gly Val Glu Val His Asn Ala Lys Thr Lys Pro Arg Glu Glu Gln Phe
               740                 745                 750
   Asn Ser Thr Tyr Arg Val Val Ser Val Leu Thr Val Leu His Gln Asp
           755                 760                 765
   Trp Leu Asn Gly Lys Glu Tyr Lys Cys Lys Val Ser Asn Lys Gly Leu
       770                 775                 780
   Pro Ser Ser Ile Glu Lys Thr Ile Ser Lys Ala Lys Gly Gln Pro Arg
   785                 790                 795                 800
   Glu Pro Gln Val Tyr Thr Leu Pro Pro Ser Gln Glu Glu Met Thr Lys
                   805                 810                 815
   Asn Gln Val Ser Leu Thr Cys Leu Val Lys Gly Phe Tyr Pro Ser Asp
               820                 825                 830
   Ile Ala Val Glu Trp Glu Ser Asn Gly Gln Pro Glu Asn Asn Tyr Lys
           835                 840                 845
   Thr Thr Pro Pro Val Leu Asp Ser Asp Gly Ser Phe Phe Leu Tyr Ser
       850                 855                 860
   Arg Leu Thr Val Asp Lys Ser Arg Trp Gln Glu Gly Asn Val Phe Ser
   865                 870                 875                 880
   Cys Ser Val Met His Glu Ala Leu His Asn His Tyr Thr Gln Lys Ser
                   885                 890                 895
   Leu Ser Leu Ser Leu Gly Lys
               900
<210> 7
   <211> 2710
   <212> DNA
   <213> Artificial Sequence
<220>
   <223> Synthetic
<400> 7
   datggtgtta ctcagactta tttgtttcat agctctactg atttcttctc tggaggctga 60
   taaatgcaag gaacgtgaag aaaaaataat tttagtgtca tctgcaaatg aaattgatgt 120
   tcgtccctgt cctcttaacc caaatgaaca caaaggcact ataacttggt ataaggatga 180
   cagcaagaca cctgtatcta cagaacaagc ctccaggatt catcaacaca aagagaaact 240
   ttggtttgtt cctgctaagg tggaggattc aggacattac tattgcgtgg taagaaattc 300
   atcttactgc ctcagaatta aaataagtgc aaaatttgtg gagaatgagc ctaacttatg 360
   ttataatgca caagccatat ttaagcagaa actacccgtt gcaggagacg gaggacttgt 420
   gtgcccttat atggagtttt ttaaaaatga aaataatgag ttacctaaat tacagtggta 480
   taaggattgc aaacctctac ttcttgacaa tatacacttt agtggagtca aagataggct 540
   catcgtgatg aatgtggctg aaaagcatag agggaactat acttgtcatg catcctacac 600
   atacttgggc aagcaatatc ctattacccg ggtaatagaa tttattactc tagaggaaaa 660
   caaacccaca aggcctgtga ttgtgagccc agctaatgag acaatggaag tagacttggg 720
   atcccagata caattgatct gtaatgtcac cggccagttg agtgacattg cttactggaa 780
   gtggaatggg tcagtaattg atgaagatga cccagtgcta ggggaagact attacagtgt 840
   ggaaaatcct gcaaacaaaa gaaggagtac cctcatcaca gtgcttaata tatcggaaat 900
   tgagagtaga ttttataaac atccatttac ctgttttgcc aagaatacac atggtataga 960
   tgcagcatat atccagttaa tatatccagt cactaattca gaacgctgcg atgactgggg 1020
   actagacacc atgaggcaaa tccaagtgtt tgaagatgag ccagctcgca tcaagtgccc 1080
   actctttgaa cacttcttga aattcaacta cagcacagcc cattcagctg gccttactct 1140
   gatctggtat tggactaggc aggaccggga ccttgaggag ccaattaact tccgcctccc 1200
   cgagaaccgc attagtaagg agaaagatgt gctgtggttc cggcccactc tcctcaatga 1260
   cactggcaac tatacctgca tgttaaggaa cactacatat tgcagcaaag ttgcatttcc 1320
   cttggaagtt gttcaaaaag acagctgttt caattccccc atgaaactcc cagtgcataa 1380
   actgtatata gaatatggca ttcagaggat cacttgtcca aatgtagatg gatattttcc 1440
   ttccagtgtc aaaccgacta tcacttggta tatgggctgt tataaaatac agaattttaa 1500
   taatgtaata cccgaaggta tgaacttgag tttcctcatt gccttaattt caaataatgg 1560
   aaattacaca tgtgttgtta catatccaga aaatggacgt acgtttcatc tcaccaggac 1620
   tctgactgta aaggtagtag gctctccaaa aaatgcagtg ccccctgtga tccattcacc 1680
   taatgatcat gtggtctatg agaaagaacc aggagaggag ctactcattc cctgtacggt 1740
   ctattttagt tttctgatgg attctcgcaa tgaggtttgg tggaccattg atggaaaaaa 1800
   acctgatgac atcactattg atgtcaccat taacgaaagt ataagtcata gtagaacaga 1860
   agatgaaaca agaactcaga ttttgagcat caagaaagtt acctctgagg atctcaagcg 1920
   cagctatgtc tgtcatgcta gaagtgccaa aggcgaagtt gccaaagcag ccaaggtgaa 1980
   gcagaaagtg ccagctccaa gatacacagt ggaatccgga gagtccaaat acggtccgcc 2040
   atgcccacca tgcccagcac ctgagttcct ggggggacca tcagtcttcc tgttcccccc 2100
   aaaacccaag gacactctca tgatctcccg gacccctgag gtcacgtgcg tggtggtgga 2160
   cgtgagccag gaagaccccg aggtccagtt caactggtac gtggatggcg tggaggtgca 2220
   taatgccaag acaaagccgc gggaggagca gttcaacagc acgtaccgtg tggtcagcgt 2280
   cctcaccgtc ctgcaccagg actggctgaa cggcaaggag tacaagtgca aggtctccaa 2340
   caaaggcctc ccgtcctcca tcgagaaaac catctccaaa gccaaagggc agccccgaga 2400
   gccacaggtg tacaccctgc ccccatccca ggaggagatg accaagaacc aggtcagcct 2460
   gacctgcctg gtcaaaggct tctaccccag cgacatcgcc gtggagtggg agagcaatgg 2520
   gcagccggag aacaactaca agaccacgcc tcccgtgctg gactccgacg gctccttctt 2580
   cctctacagc aggctaaccg tggacaagag caggtggcag gaggggaatg tcttctcatg 2640
   ctccgtgatg catgaggctc tgcacaacca ctacacacag aagagcctct ccctgtctct 2700
   gggtaaatga                                                        2710
<210> 8
   <211> 903
   <212> PRT
   <213> Artificial Sequence
<220>
   <223> Synthetic
<400> 8
   Pro Met Val Leu Leu Arg Leu Ile Cys Phe Ile Ala Leu Leu Ile Ser
    1               5                  10                  15
   Ser Leu Glu Ala Asp Lys Cys Lys Glu Arg Glu Glu Lys Ile Ile Leu
               20                  25                  30
   Val Ser Ser Ala Asn Glu Ile Asp Val Arg Pro Cys Pro Leu Asn Pro
           35                  40                  45
   Asn Glu His Lys Gly Thr Ile Thr Trp Tyr Lys Asp Asp Ser Lys Thr
       50                  55                  60
   Pro Val Ser Thr Glu Gln Ala Ser Arg Ile His Gln His Lys Glu Lys
   65                  70                  75                  80
   Leu Trp Phe Val Pro Ala Lys Val Glu Asp Ser Gly His Tyr Tyr Cys
                   85                  90                  95
   Val Val Arg Asn Ser Ser Tyr Cys Leu Arg Ile Lys Ile Ser Ala Lys
               100                 105                 110
   Phe Val Glu Asn Glu Pro Asn Leu Cys Tyr Asn Ala Gln Ala Ile Phe
           115                 120                 125
   Lys Gln Lys Leu Pro Val Ala Gly Asp Gly Gly Leu Val Cys Pro Tyr
       130                 135                 140
   Met Glu Phe Phe Lys Asn Glu Asn Asn Glu Leu Pro Lys Leu Gln Trp
   145                 150                 155                 160
   Tyr Lys Asp Cys Lys Pro Leu Leu Leu Asp Asn Ile His Phe Ser Gly
                   165                 170                 175
   Val Lys Asp Arg Leu Ile Val Met Asn Val Ala Glu Lys His Arg Gly
               180                 185                 190
   Asn Tyr Thr Cys His Ala Ser Tyr Thr Tyr Leu Gly Lys Gln Tyr Pro
           195                 200                 205
   Ile Thr Arg Val Ile Glu Phe Ile Thr Leu Glu Glu Asn Lys Pro Thr
       210                 215                 220
   Arg Pro Val Ile Val Ser Pro Ala Asn Glu Thr Met Glu Val Asp Leu
   225                 230                 235                 240
   Gly Ser Gln Ile Gln Leu Ile Cys Asn Val Thr Gly Gln Leu Ser Asp
                   245                 250                 255
   Ile Ala Tyr Trp Lys Trp Asn Gly Ser Val Ile Asp Glu Asp Asp Pro
               260                 265                 270
   Val Leu Gly Glu Asp Tyr Tyr Ser Val Glu Asn Pro Ala Asn Lys Arg
           275                 280                 285
   Arg Ser Thr Leu Ile Thr Val Leu Asn Ile Ser Glu Ile Glu Ser Arg
       290                 295                 300
   Phe Tyr Lys His Pro Phe Thr Cys Phe Ala Lys Asn Thr His Gly Ile
   305                 310                 315                 320
   Asp Ala Ala Tyr Ile Gln Leu Ile Tyr Pro Val Thr Asn Ser Glu Arg
                   325                 330                 335
   Cys Asp Asp Trp Gly Leu Asp Thr Met Arg Gln Ile Gln Val Phe Glu
               340                 345                 350
   Asp Glu Pro Ala Arg Ile Lys Cys Pro Leu Phe Glu His Phe Leu Lys
           355                 360                 365
   Phe Asn Tyr Ser Thr Ala His Ser Ala Gly Leu Thr Leu Ile Trp Tyr
       370                 375                 380
   Trp Thr Arg Gln Asp Arg Asp Leu Glu Glu Pro Ile Asn Phe Arg Leu
   385                 390                 395                 400
   Pro Glu Asn Arg Ile Ser Lys Glu Lys Asp Val Leu Trp Phe Arg Pro
                   405                 410                 415
   Thr Leu Leu Asn Asp Thr Gly Asn Tyr Thr Cys Met Leu Arg Asn Thr
               420                 425                 430
   Thr Tyr Cys Ser Lys Val Ala Phe Pro Leu Glu Val Val Gln Lys Asp
           435                 440                 445
   Ser Cys Phe Asn Ser Pro Met Lys Leu Pro Val His Lys Leu Tyr Ile
       450                 455                 460
   Glu Tyr Gly Ile Gln Arg Ile Thr Cys Pro Asn Val Asp Gly Tyr Phe
   465                 470                 475                 480
   Pro Ser Ser Val Lys Pro Thr Ile Thr Trp Tyr Met Gly Cys Tyr Lys
                   485                 490                 495
   Ile Gln Asn Phe Asn Asn Val Ile Pro Glu Gly Met Asn Leu Ser Phe
               500                 505                 510
   Leu Ile Ala Leu Ile Ser Asn Asn Gly Asn Tyr Thr Cys Val Val Thr
           515                 520                 525
   Tyr Pro Glu Asn Gly Arg Thr Phe His Leu Thr Arg Thr Leu Thr Val
       530                 535                 540
   Lys Val Val Gly Ser Pro Lys Asn Ala Val Pro Pro Val Ile His Ser
   545                 550                 555                 560
   Pro Asn Asp His Val Val Tyr Glu Lys Glu Pro Gly Glu Glu Leu Leu
                   565                 570                 575
   Ile Pro Cys Thr Val Tyr Phe Ser Phe Leu Met Asp Ser Arg Asn Glu
               580                 585                 590
   Val Trp Trp Thr Ile Asp Gly Lys Lys Pro Asp Asp Ile Thr Ile Asp
           595                 600                 605
   Val Thr Ile Asn Glu Ser Ile Ser His Ser Arg Thr Glu Asp Glu Thr
       610                 615                 620
   Arg Thr Gln Ile Leu Ser Ile Lys Lys Val Thr Ser Glu Asp Leu Lys
   625                 630                 635                 640
   Arg Ser Tyr Val Cys His Ala Arg Ser Ala Lys Gly Glu Val Ala Lys
                   645                 650                 655
   Ala Ala Lys Val Lys Gln Lys Val Pro Ala Pro Arg Tyr Thr Val Glu
               660                 665                 670
   Ser Gly Glu Ser Lys Tyr Gly Pro Pro Cys Pro Pro Cys Pro Ala Pro
           675                 680                 685
   Glu Phe Leu Gly Gly Pro Ser Val Phe Leu Phe Pro Pro Lys Pro Lys
       690                 695                 700
   Asp Thr Leu Met Ile Ser Arg Thr Pro Glu Val Thr Cys Val Val Val
   705                 710                 715                 720
   Asp Val Ser Gln Glu Asp Pro Glu Val Gln Phe Asn Trp Tyr Val Asp
                   725                 730                 735
   Gly Val Glu Val His Asn Ala Lys Thr Lys Pro Arg Glu Glu Gln Phe
               740                 745                 750
   Asn Ser Thr Tyr Arg Val Val Ser Val Leu Thr Val Leu His Gln Asp
           755                 760                 765
   Trp Leu Asn Gly Lys Glu Tyr Lys Cys Lys Val Ser Asn Lys Gly Leu
       770                 775                 780
   Pro Ser Ser Ile Glu Lys Thr Ile Ser Lys Ala Lys Gly Gln Pro Arg
   785                 790                 795                 800
   Glu Pro Gln Val Tyr Thr Leu Pro Pro Ser Gln Glu Glu Met Thr Lys
                   805                 810                 815
   Asn Gln Val Ser Leu Thr Cys Leu Val Lys Gly Phe Tyr Pro Ser Asp
               820                 825                 830
   Ile Ala Val Glu Trp Glu Ser Asn Gly Gln Pro Glu Asn Asn Tyr Lys
           835                 840                 845
   Thr Thr Pro Pro Val Leu Asp Ser Asp Gly Ser Phe Phe Leu Tyr Ser
       850                 855                 860
   Arg Leu Thr Val Asp Lys Ser Arg Trp Gln Glu Gly Asn Val Phe Ser
   865                 870                 875                 880
   Cys Ser Val Met His Glu Ala Leu His Asn His Tyr Thr Gln Lys Ser
                   885                 890                 895
   Leu Ser Leu Ser Leu Gly Lys
               900
<210> 9
   <211> 2704
   <212> DNA
   <213> Artificial Sequence
<220>
   <223> Synthetic
<400> 9
   datggtgctt ctgtggtgtg tagtgagtct ctacttttat ggaatcctgc aaagtgatgc 60
   ctcagaacgc tgcgatgact ggggactaga caccatgagg caaatccaag tgtttgaaga 120
   tgagccagct cgcatcaagt gcccactctt tgaacacttc ttgaaattca actacagcac 180
   agcccattca gctggcctta ctctgatctg gtattggact aggcaggacc gggaccttga 240
   ggagccaatt aacttccgcc tccccgagaa ccgcattagt aaggagaaag atgtgctgtg 300
   gttccggccc actctcctca atgacactgg caactatacc tgcatgttaa ggaacactac 360
   atattgcagc aaagttgcat ttcccttgga agttgttcaa aaagacagct gtttcaattc 420
   ccccatgaaa ctcccagtgc ataaactgta tatagaatat ggcattcaga ggatcacttg 480
   tccaaatgta gatggatatt ttccttccag tgtcaaaccg actatcactt ggtatatggg 540
   ctgttataaa atacagaatt ttaataatgt aatacccgaa ggtatgaact tgagtttcct 600
   cattgcctta atttcaaata atggaaatta cacatgtgtt gttacatatc cagaaaatgg 660
   acgtacgttt catctcacca ggactctgac tgtaaaggta gtaggctctc caaaaaatgc 720
   agtgccccct gtgatccatt cacctaatga tcatgtggtc tatgagaaag aaccaggaga 780
   ggagctactc attccctgta cggtctattt tagttttctg atggattctc gcaatgaggt 840
   ttggtggacc attgatggaa aaaaacctga tgacatcact attgatgtca ccattaacga 900
   aagtataagt catagtagaa cagaagatga aacaagaact cagattttga gcatcaagaa 960
   agttacctct gaggatctca agcgcagcta tgtctgtcat gctagaagtg ccaaaggcga 1020
   agttgccaaa gcagccaagg tgaagcagaa agtgccagct ccaagataca cagtggaaaa 1080
   atgcaaggaa cgtgaagaaa aaataatttt agtgagctca gcaaatgaaa tcgatgttcg 1140
   tccctgtcct cttaacccaa atgaacacaa aggcactata acttggtata aggatgacag 1200
   caagacacct gtatctacag aacaagcctc caggattcat caacacaaag agaaactttg 1260
   gtttgttcct gctaaggtgg aggattcagg acattactat tgcgtggtaa gaaattcatc 1320
   ttactgcctc agaattaaaa taagtgcaaa atttgtggag aatgagccta acttatgtta 1380
   taatgcacaa gccatattta agcagaaact acccgttgca ggagacggag gacttgtgtg 1440
   cccttatatg gagtttttta aaaatgaaaa taatgagtta cctaaattac agtggtataa 1500
   ggattgcaaa cctctacttc ttgacaatat acactttagt ggagtcaaag ataggctcat 1560
   cgtgatgaat gtggctgaaa agcatagagg gaactatact tgtcatgcat cctacacata 1620
   cttgggcaag caatatccta ttacccgggt aatagaattt attactctag aggaaaacaa 1680
   acccacaagg cctgtgattg tgagcccagc taatgagaca atggaagtag acttgggatc 1740
   ccagatacaa ttgatctgta atgtcaccgg ccagttgagt gacattgctt actggaagtg 1800
   gaatgggtca gtaattgatg aagatgaccc agtgctaggg gaagactatt acagtgtgga 1860
   aaatcctgca aacaaaagaa ggagtaccct catcacagtg cttaatatat cggaaattga 1920
   gagtagattt tataaacatc catttacctg ttttgccaag aatacacatg gtatagatgc 1980
   agcatatatc cagttaatat atccagtcac taattccgga gacaaaactc acacatgccc 2040
   accgtgccca gcacctgaac tcctgggggg accgtcagtc ttcctcttcc ccccaaaacc 2100
   caaggacacc ctcatgatct cccggacccc tgaggtcaca tgcgtggtgg tggacgtgag 2160
   ccacgaagac cctgaggtca agttcaactg gtacgtggac ggcgtggagg tgcataatgc 2220
   caagacaaag ccgcgggagg agcagtacaa cagcacgtac cgtgtggtca gcgtcctcac 2280
   cgtcctgcac caggactggc tgaatggcaa ggagtacaag tgcaaggtct ccaacaaagc 2340
   cctcccagcc cccatcgaga aaaccatctc caaagccaaa gggcagcccc gagaaccaca 2400
   ggtgtacacc ctgcccccat cccgggatga gctgaccaag aaccaggtca gcctgacctg 2460
   cctggtcaaa ggcttctatc ccagcgacat cgccgtggag tgggagagca atgggcagcc 2520
   ggagaacaac tacaagacca cgcctcccgt gctggactcc gacggctcct tcttcctcta 2580
   cagcaagctc accgtggaca agagcaggtg gcagcagggg aacgtcttct catgctccgt 2640
   gatgcatgag gctctgcaca accactacac gcagaagagc ctctccctgt ctccgggtaa 2700
   atga 2704
<210> 10
   <211> 901
   <212> PRT
   <213> Artificial Sequence
<220>
   <223> Synthetic
<400> 10
   Pro Met Val Leu Leu Trp Cys Val Val Ser Leu Tyr Phe Tyr Gly Ile
    1               5                  10                  15
   Leu Gln Ser Asp Ala Ser Glu Arg Cys Asp Asp Trp Gly Leu Asp Thr
                   20                  25                  30
   Met Arg Gln Ile Gln Val Phe Glu Asp Glu Pro Ala Arg Ile Lys Cys
               35                  40                  45
   Pro Leu Phe Glu His Phe Leu Lys Phe Asn Tyr Ser Thr Ala His Ser
       50                  55                  60
   Ala Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
               100                 105                 110
   Tyr Thr Cys Met Leu Arg Asn Thr Thr Tyr Cys Ser Lys Val Ala Phe
           115                 120                 125
   Pro Leu Glu Val Val Gln Lys Asp Ser Cys Phe Asn Ser Pro Met Lys
       130                 135                 140
   Leu Pro Val His Lys Leu Tyr Ile Glu Tyr Gly Ile Gln Arg Ile Thr
   145                 150                 155                 160
   Cys Pro Asn Val Asp Gly Tyr Phe Pro Ser Ser Val Lys Pro Thr Ile
                   165                 170                 175
   Thr Trp Tyr Met Gly Cys Tyr Lys Ile Gln Asn Phe Asn Asn Val Ile
               180                 185                 190
   Pro Glu Gly Met Asn Leu Ser Phe Leu Ile Ala Leu Ile Ser Asn Asn
           195                 200                 205
   Gly Asn Tyr Thr Cys Val Val Thr Tyr Pro Glu Asn Gly Arg Thr Phe
       210                 215                 220
   His Leu Thr Arg Thr Leu Thr Val Lys Val Val Gly Ser Pro Lys Asn
   225                 230                 235                 240
   Ala Val Pro Pro Val Ile His Ser Pro Asn Asp His Val Val Tyr Glu
                   245                 250                 255
   Lys Glu Pro Gly Glu Glu Leu Leu Ile Pro Cys Thr Val Tyr Phe Ser
               260                 265                 270
   Phe Leu Met Asp Ser Arg Asn Glu Val Trp Trp Thr Ile Asp Gly Lys
           275                 280                 285
   Lys Pro Asp Asp Ile Thr Ile Asp Val Thr Ile Asn Glu Ser Ile Ser
       290                 295                 300
   His Ser Arg Thr Glu Asp Glu Thr Arg Thr Gln Ile Leu Ser Ile Lys
   305                 310                 315                 320
   Lys Val Thr Ser Glu Asp Leu Lys Arg Ser Tyr Val Cys His Ala Arg
                   325                 330                 335
   Ser Ala Lys Gly Glu Val Ala Lys Ala Ala Lys Val Lys Gln Lys Val
               340                 345                 350
   Pro Ala Pro Arg Tyr Thr Val Glu Lys Cys Lys Glu Arg Glu Glu Lys
           355                 360                 365
   Ile Ile Leu Val Ser Ser Ala Asn Glu Ile Asp Val Arg Pro Cys Pro
       370                 375                 380
   Leu Asn Pro Asn Glu His Lys Gly Thr Ile Thr Trp Tyr Lys Asp Asp
   385                 390                 395                 400
   Ser Lys Thr Pro Val Ser Thr Glu Gln Ala Ser Arg Ile His Gln His
                   405                 410                 415
   Lys Glu Lys Leu Trp Phe Val Pro Ala Lys Val Glu Asp Ser Gly His
               420                 425                 430
   Tyr Tyr Cys Val Val Arg Asn Ser Ser Tyr Cys Leu Arg Ile Lys Ile
           435                 440                 445
   Ser Ala Lys Phe Val Glu Asn Glu Pro Asn Leu Cys Tyr Asn Ala Gln
       450                 455                 460
   Ala Ile Phe Lys Gln Lys Leu Pro Val Ala Gly Asp Gly Gly Leu Val
   465                 470                 475                 480
   Cys Pro Tyr Met Glu Phe Phe Lys Asn Glu Asn Asn Glu Leu Pro Lys
                       485                     490                     495
   Leu Gln Trp Tyr Lys Asp Cys Lys Pro Leu Leu Leu Asp Asn Ile His
                   500                     505                     510
   Phe Ser Gly Val Lys Asp Arg Leu Ile Val Met Asn Val Ala Glu Lys
               515                     520                     525
   His Arg Gly Asn Tyr Thr Cys His Ala Ser Tyr Thr Tyr Leu Gly Lys
           530                     535                     540
   Gln Tyr Pro Ile Thr Arg Val Ile Glu Phe Ile Thr Leu Glu Glu Asn
   545                     550                     555                     560
   Lys Pro Thr Arg Pro Val Ile Val Ser Pro Ala Asn Glu Thr Met Glu
                       565                     570                     575
   Val Asp Leu Gly Ser Gln Ile Gln Leu Ile Cys Asn Val Thr Gly Gln
                   580                     585                     590
   Leu Ser Asp Ile Ala Tyr Trp Lys Trp Asn Gly Ser Val Ile Asp Glu
               595                     600                     605
   Asp Asp Pro Val Leu Gly Glu Asp Tyr Tyr Ser Val Glu Asn Pro Ala
           610                     615                     620
   Asn Lys Arg Arg Ser Thr Leu Ile Thr Val Leu Asn Ile Ser Glu Ile
   625                     630                     635                     640
   Glu Ser Arg Phe Tyr Lys His Pro Phe Thr Cys Phe Ala Lys Asn Thr
                       645                     650                     655
   His Gly Ile Asp Ala Ala Tyr Ile Gln Leu Ile Tyr Pro Val Thr Asn
                   660                     665                     670
   Ser Gly Asp Lys Thr His Thr Cys Pro Pro Cys Pro Ala Pro Glu Leu
               675                     680                     685
   Leu Gly Gly Pro Ser Val Phe Leu Phe Pro Pro Lys Pro Lys Asp Thr
           690                     695                     700
   Leu Met Ile Ser Arg Thr Pro Glu Val Thr Cys Val Val Val Asp Val
   705                     710                     715                     720
   Ser His Glu Asp Pro Glu Val Lys Phe Asn Trp Tyr Val Asp Gly Val
                       725                     730                     735
   Glu Val His Asn Ala Lys Thr Lys Pro Arg Glu Glu Gln Tyr Asn Ser
                   740                     745                     750
   Thr Tyr Arg Val Val Ser Val Leu Thr Val Leu His Gln Asp Trp Leu
               755                     760                     765
   Asn Gly Lys Glu Tyr Lys Cys Lys Val Ser Asn Lys Ala Leu Pro Ala
           770                     775                     780
   Pro Ile Glu Lys Thr Ile Ser Lys Ala Lys Gly Gln Pro Arg Glu Pro
   785                     790                     795                     800
   Gln Val Tyr Thr Leu Pro Pro Ser Arg Asp Glu Leu Thr Lys Asn Gln
                       805                     810                     815
   Val Ser Leu Thr Cys Leu Val Lys Gly Phe Tyr Pro Ser Asp Ile Ala
                   820                     825                     830
   Val Glu Trp Glu Ser Asn Gly Gln Pro Glu Asn Asn Tyr Lys Thr Thr
               835                     840                     845
   Pro Pro Val Leu Asp Ser Asp Gly Ser Phe Phe Leu Tyr Ser Lys Leu
           850                     855                     860
   Thr Val Asp Lys Ser Arg Trp Gln Gln Gly Asn Val Phe Ser Cys Ser
   865                     870                     875                     880
   Val Met His Glu Ala Leu His Asn His Tyr Thr Gln Lys Ser Leu Ser
                       885                     890                     895
   Leu Ser Pro Gly Lys
                   900
<210> 11
   <211> 2710
   <212> DNA
<210> 12
   <211> 903
   <212> PRT
   <213> Artificial Sequence
<220>
   <223> Synthetic
<400> 12
   Pro Met Val Leu Leu Trp Cys Val Val Ser Leu Tyr Phe Tyr Gly Ile
    1               5                  10                  15
   Leu Gln Ser Asp Ala Ser Glu Arg Cys Asp Asp Trp Gly Leu Asp Thr
               20                  25                  30
   Met Arg Gln Ile Gln Val Phe Glu Asp Glu Pro Ala Arg Ile Lys Cys
           35                  40                  45
   Pro Leu Phe Glu His Phe Leu Lys Phe Asn Tyr Ser Thr Ala His Ser
       50                  55                  60
   Ala Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
               100                 105                 110
   Tyr Thr Cys Met Leu Arg Asn Thr Thr Tyr Cys Ser Lys Val Ala Phe
           115                 120                 125
   Pro Leu Glu Val Val Gln Lys Asp Ser Cys Phe Asn Ser Pro Met Lys
       130                 135                 140
   Leu Pro Val His Lys Leu Tyr Ile Glu Tyr Gly Ile Gln Arg Ile Thr
   145                 150                 155                 160
   Cys Pro Asn Val Asp Gly Tyr Phe Pro Ser Ser Val Lys Pro Thr Ile
                   165                 170                 175
   Thr Trp Tyr Met Gly Cys Tyr Lys Ile Gln Asn Phe Asn Asn Val Ile
               180                 185                 190
   Pro Glu Gly Met Asn Leu Ser Phe Leu Ile Ala Leu Ile Ser Asn Asn
           195                 200                 205
   Gly Asn Tyr Thr Cys Val Val Thr Tyr Pro Glu Asn Gly Arg Thr Phe
       210                 215                 220
   His Leu Thr Arg Thr Leu Thr Val Lys Val Val Gly Ser Pro Lys Asn
   225                 230                 235                 240
   Ala Val Pro Pro Val Ile His Ser Pro Asn Asp His Val Val Tyr Glu
                   245                 250                 255
   Lys Glu Pro Gly Glu Glu Leu Leu Ile Pro Cys Thr Val Tyr Phe Ser
               260                 265                 270
   Phe Leu Met Asp Ser Arg Asn Glu Val Trp Trp Thr Ile Asp Gly Lys
           275                 280                 285
   Lys Pro Asp Asp Ile Thr Ile Asp Val Thr Ile Asn Glu Ser Ile Ser
       290                 295                 300
   His Ser Arg Thr Glu Asp Glu Thr Arg Thr Gln Ile Leu Ser Ile Lys
   305                 310                 315                 320
   Lys Val Thr Ser Glu Asp Leu Lys Arg Ser Tyr Val Cys His Ala Arg
                   325                 330                 335
   Ser Ala Lys Gly Glu Val Ala Lys Ala Ala Lys Val Lys Gln Lys Val
               340                 345                 350
   Pro Ala Pro Arg Tyr Thr Val Glu Lys Cys Lys Glu Arg Glu Glu Lys
           355                 360                 365
   Ile Ile Leu Val Ser Ser Ala Asn Glu Ile Asp Val Arg Pro Cys Pro
       370                 375                 380
   Leu Asn Pro Asn Glu His Lys Gly Thr Ile Thr Trp Tyr Lys Asp Asp
   385                 390                 395                 400
   Ser Lys Thr Pro Val Ser Thr Glu Gln Ala Ser Arg Ile His Gln His
                   405                 410                 415
   Lys Glu Lys Leu Trp Phe Val Pro Ala Lys Val Glu Asp Ser Gly His
               420                 425                 430
   Tyr Tyr Cys Val Val Arg Asn Ser Ser Tyr Cys Leu Arg Ile Lys Ile
           435                 440                 445
   Ser Ala Lys Phe Val Glu Asn Glu Pro Asn Leu Cys Tyr Asn Ala Gln
       450                 455                 460
   Ala Ile Phe Lys Gln Lys Leu Pro Val Ala Gly Asp Gly Gly Leu Val
   465                 470                 475                 480
   Cys Pro Tyr Met Glu Phe Phe Lys Asn Glu Asn Asn Glu Leu Pro Lys
                   485                 490                 495
   Leu Gln Trp Tyr Lys Asp Cys Lys Pro Leu Leu Leu Asp Asn Ile His
               500                 505                 510
   Phe Ser Gly Val Lys Asp Arg Leu Ile Val Met Asn Val Ala Glu Lys
           515                 520                 525
   His Arg Gly Asn Tyr Thr Cys His Ala Ser Tyr Thr Tyr Leu Gly Lys
       530                 535                 540
   Gln Tyr Pro Ile Thr Arg Val Ile Glu Phe Ile Thr Leu Glu Glu Asn
   545                 550                 555                 560
   Lys Pro Thr Arg Pro Val Ile Val Ser Pro Ala Asn Glu Thr Met Glu
                   565                 570                 575
   Val Asp Leu Gly Ser Gln Ile Gln Leu Ile Cys Asn Val Thr Gly Gln
               580                 585                 590
   Leu Ser Asp Ile Ala Tyr Trp Lys Trp Asn Gly Ser Val Ile Asp Glu
           595                 600                 605
   Asp Asp Pro Val Leu Gly Glu Asp Tyr Tyr Ser Val Glu Asn Pro Ala
       610                 615                 620
   Asn Lys Arg Arg Ser Thr Leu Ile Thr Val Leu Asn Ile Ser Glu Ile
   625                 630                 635                 640
   Glu Ser Arg Phe Tyr Lys His Pro Phe Thr Cys Phe Ala Lys Asn Thr
                   645                 650                 655
   His Gly Ile Asp Ala Ala Tyr Ile Gln Leu Ile Tyr Pro Val Thr Asn
               660                 665                 670
   Ser Gly Glu Ser Lys Tyr Gly Pro Pro Cys Pro Ser Cys Pro Ala Pro
           675                 680                 685
   Glu Phe Leu Gly Gly Pro Ser Val Phe Leu Phe Pro Pro Lys Pro Lys
       690                 695                 700
   Asp Thr Leu Met Ile Ser Arg Thr Pro Glu Val Thr Cys Val Val Val
   705                 710                 715                 720
   Asp Val Ser Gln Glu Asp Pro Glu Val Gln Phe Asn Trp Tyr Val Asp
                   725                 730                 735
   Gly Val Glu Val His Asn Ala Lys Thr Lys Pro Arg Glu Glu Gln Phe
               740                 745                 750
   Asn Ser Thr Tyr Arg Val Val Ser Val Leu Thr Val Leu His Gln Asp
           755                 760                 765
   Trp Leu Asn Gly Lys Glu Tyr Lys Cys Lys Val Ser Asn Lys Gly Leu
       770                 775                 780
   Pro Ser Ser Ile Glu Lys Thr Ile Ser Lys Ala Lys Gly Gln Pro Arg
   785                 790                 795                 800
   Glu Pro Gln Val Tyr Thr Leu Pro Pro Ser Gln Glu Glu Met Thr Lys
                   805                 810                 815
   Asn Gln Val Ser Leu Thr Cys Leu Val Lys Gly Phe Tyr Pro Ser Asp
               820                 825                 830
   Ile Ala Val Glu Trp Glu Ser Asn Gly Gln Pro Glu Asn Asn Tyr Lys
           835                 840                 845
   Thr Thr Pro Pro Val Leu Asp Ser Asp Gly Ser Phe Phe Leu Tyr Ser
       850                 855                 860
   Arg Leu Thr Val Asp Lys Ser Arg Trp Gln Glu Gly Asn Val Phe Ser
   865                 870                 875                 880
   Cys Ser Val Met His Glu Ala Leu His Asn His Tyr Thr Gln Lys Ser
                   885                 890                 895
   Leu Ser Leu Ser Leu Gly Lys
   900
<210> 13
   <211> 2710
   <212> DNA
   <213> artificial
<220>
   <223> synthetic
<400> 13
   datggtgctt ctgtggtgtg tagtgagtct ctacttttat ggaatcctgc aaagtgatgc 60
   ctcagaacgc tgcgatgact ggggactaga caccatgagg caaatccaag tgtttgaaga 120
   tgagccagct cgcatcaagt gcccactctt tgaacacttc ttgaaattca actacagcac 180
   agcccattca gctggcctta ctctgatctg gtattggact aggcaggacc gggaccttga 240
   ggagccaatt aacttccgcc tccccgagaa ccgcattagt aaggagaaag atgtgctgtg 300
   gttccggccc actctcctca atgacactgg caactatacc tgcatgttaa ggaacactac 360
   atattgcagc aaagttgcat ttcccttgga agttgttcaa aaagacagct gtttcaattc 420
   ccccatgaaa ctcccagtgc ataaactgta tatagaatat ggcattcaga ggatcacttg 480
   tccaaatgta gatggatatt ttccttccag tgtcaaaccg actatcactt ggtatatggg 540
   ctgttataaa atacagaatt ttaataatgt aatacccgaa ggtatgaact tgagtttcct 600
   cattgcctta atttcaaata atggaaatta cacatgtgtt gttacatatc cagaaaatgg 660
   acgtacgttt catctcacca ggactctgac tgtaaaggta gtaggctctc caaaaaatgc 720
   agtgccccct gtgatccatt cacctaatga tcatgtggtc tatgagaaag aaccaggaga 780
   ggagctactc attccctgta cggtctattt tagttttctg atggattctc gcaatgaggt 840
   ttggtggacc attgatggaa aaaaacctga tgacatcact attgatgtca ccattaacga 900
   aagtataagt catagtagaa cagaagatga aacaagaact cagattttga gcatcaagaa 960
   agttacctct gaggatctca agcgcagcta tgtctgtcat gctagaagtg ccaaaggcga 1020
   agttgccaaa gcagccaagg tgaagcagaa agtgccagct ccaagataca cagtggaaaa 1080
   atgcaaggaa cgtgaagaaa aaataatttt agtgagctca gcaaatgaaa tcgatgttcg 1140
   tccctgtcct cttaacccaa atgaacacaa aggcactata acttggtata aggatgacag 1200
   caagacacct gtatctacag aacaagcctc caggattcat caacacaaag agaaactttg 1260
   gtttgttcct gctaaggtgg aggattcagg acattactat tgcgtggtaa gaaattcatc 1320
   ttactgcctc agaattaaaa taagtgcaaa atttgtggag aatgagccta acttatgtta 1380
   taatgcacaa gccatattta agcagaaact acccgttgca ggagacggag gacttgtgtg 1440
   cccttatatg gagtttttta aaaatgaaaa taatgagtta cctaaattac agtggtataa 1500
   ggattgcaaa cctctacttc ttgacaatat acactttagt ggagtcaaag ataggctcat 1560
   cgtgatgaat gtggctgaaa agcatagagg gaactatact tgtcatgcat cctacacata 1620
   cttgggcaag caatatccta ttacccgggt aatagaattt attactctag aggaaaacaa 1680
   acccacaagg cctgtgattg tgagcccagc taatgagaca atggaagtag acttgggatc 1740
   ccagatacaa ttgatctgta atgtcaccgg ccagttgagt gacattgctt actggaagtg 1800
   gaatgggtca gtaattgatg aagatgaccc agtgctaggg gaagactatt acagtgtgga 1860
   aaatcctgca aacaaaagaa ggagtaccct catcacagtg cttaatatat cggaaattga 1920
   gagtagattt tataaacatc catttacctg ttttgccaag aatacacatg gtatagatgc 1980
   agcatatatc cagttaatat atccagtcac taattccgga gagtccaaat acggtccgcc 2040
   atgcccacca tgcccagcac ctgagttcct ggggggacca tcagtcttcc tgttcccccc 2100
   aaaacccaag gacactctca tgatctcccg gacccctgag gtcacgtgcg tggtggtgga 2160
   cgtgagccag gaagaccccg aggtccagtt caactggtac gtggatggcg tggaggtgca 2220
   taatgccaag acaaagccgc gggaggagca gttcaacagc acgtaccgtg tggtcagcgt 2280
   cctcaccgtc ctgcaccagg actggctgaa cggcaaggag tacaagtgca aggtctccaa 2340
   caaaggcctc ccgtcctcca tcgagaaaac catctccaaa gccaaagggc agccccgaga 2400
   gccacaggtg tacaccctgc ccccatccca ggaggagatg accaagaacc aggtcagcct 2460
   gacctgcctg gtcaaaggct tctaccccag cgacatcgcc gtggagtggg agagcaatgg 2520
   gcagccggag aacaactaca agaccacgcc tcccgtgctg gactccgacg gctccttctt 2580
   cctctacagc aggctaaccg tggacaagag caggtggcag gaggggaatg tcttctcatg 2640
   ctccgtgatg catgaggctc tgcacaacca ctacacacag aagagcctct ccctgtctct 2700
   gggtaaatga                                                        2710
<210> 14
   <211> 903
   <212> PRT
   <213> Artificial Sequence
<220>
   <223> Synthetic
<400> 14
   Pro Met Val Leu Leu Trp Cys Val Val Ser Leu Tyr Phe Tyr Gly Ile
    1               5                  10                  15
   Leu Gln Ser Asp Ala Ser Glu Arg Cys Asp Asp Trp Gly Leu Asp Thr
               20                  25                  30
   Met Arg Gln Ile Gln Val Phe Glu Asp Glu Pro Ala Arg Ile Lys Cys
           35                  40                  45
   Pro Leu Phe Glu His Phe Leu Lys Phe Asn Tyr Ser Thr Ala His Ser
       50                  55                  60
   Ala Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
               100                 105                 110
   Tyr Thr Cys Met Leu Arg Asn Thr Thr Tyr Cys Ser Lys Val Ala Phe
           115                 120                 125
   Pro Leu Glu Val Val Gln Lys Asp Ser Cys Phe Asn Ser Pro Met Lys
       130                 135                 140
   Leu Pro Val His Lys Leu Tyr Ile Glu Tyr Gly Ile Gln Arg Ile Thr
   145                 150                 155                 160
   Cys Pro Asn Val Asp Gly Tyr Phe Pro Ser Ser Val Lys Pro Thr Ile
                   165                 170                 175
   Thr Trp Tyr Met Gly Cys Tyr Lys Ile Gln Asn Phe Asn Asn Val Ile
               180                 185                 190
   Pro Glu Gly Met Asn Leu Ser Phe Leu Ile Ala Leu Ile Ser Asn Asn
           195                 200                 205
   Gly Asn Tyr Thr Cys Val Val Thr Tyr Pro Glu Asn Gly Arg Thr Phe
       210                 215                 220
   His Leu Thr Arg Thr Leu Thr Val Lys Val Val Gly Ser Pro Lys Asn
   225                 230                 235                 240
   Ala Val Pro Pro Val Ile His Ser Pro Asn Asp His Val Val Tyr Glu
                   245                 250                 255
   Lys Glu Pro Gly Glu Glu Leu Leu Ile Pro Cys Thr Val Tyr Phe Ser
               260                 265                 270
   Phe Leu Met Asp Ser Arg Asn Glu Val Trp Trp Thr Ile Asp Gly Lys
           275                 280                 285
   Lys Pro Asp Asp Ile Thr Ile Asp Val Thr Ile Asn Glu Ser Ile Ser
       290                 295                 300
   His Ser Arg Thr Glu Asp Glu Thr Arg Thr Gln Ile Leu Ser Ile Lys
   305                 310                 315                 320
   Lys Val Thr Ser Glu Asp Leu Lys Arg Ser Tyr Val Cys His Ala Arg
                   325                 330                 335
   Ser Ala Lys Gly Glu Val Ala Lys Ala Ala Lys Val Lys Gln Lys Val
               340                 345                 350
   Pro Ala Pro Arg Tyr Thr Val Glu Lys Cys Lys Glu Arg Glu Glu Lys
           355                 360                 365
   Ile Ile Leu Val Ser Ser Ala Asn Glu Ile Asp Val Arg Pro Cys Pro
   370 375 380
   Leu Asn Pro Asn Glu His Lys Gly Thr Ile Thr Trp Tyr Lys Asp Asp
   385 390 395 400
   Ser Lys Thr Pro Val Ser Thr Glu Gln Ala Ser Arg Ile His Gln His
   405 410 415
   Lys Glu Lys Leu Trp Phe Val Pro Ala Lys Val Glu Asp Ser Gly His
   420 425 430
   Tyr Tyr Cys Val Val Arg Asn Ser Ser Tyr Cys Leu Arg Ile Lys Ile
   435 440 445
   Ser Ala Lys Phe Val Glu Asn Glu Pro Asn Leu Cys Tyr Asn Ala Gln
   450 455 460
   Ala Ile Phe Lys Gln Lys Leu Pro Val Ala Gly Asp Gly Gly Leu Val
   465 470 475 480
   Cys Pro Tyr Met Glu Phe Phe Lys Asn Glu Asn Asn Glu Leu Pro Lys
   485 490 495
   Leu Gln Trp Tyr Lys Asp Cys Lys Pro Leu Leu Leu Asp Asn Ile His
   500 505 510
   Phe Ser Gly Val Lys Asp Arg Leu Ile Val Met Asn Val Ala Glu Lys
   515 520 525
   His Arg Gly Asn Tyr Thr Cys His Ala Ser Tyr Thr Tyr Leu Gly Lys
   530 535 540
   Gln Tyr Pro Ile Thr Arg Val Ile Glu Phe Ile Thr Leu Glu Glu Asn
   545 550 555 560
   Lys Pro Thr Arg Pro Val Ile Val Ser Pro Ala Asn Glu Thr Met Glu
   565 570 575
   Val Asp Leu Gly Ser Gln Ile Gln Leu Ile Cys Asn Val Thr Gly Gln
   580 585 590
   Leu Ser Asp Ile Ala Tyr Trp Lys Trp Asn Gly Ser Val Ile Asp Glu
   595 600 605
   Asp Asp Pro Val Leu Gly Glu Asp Tyr Tyr Ser Val Glu Asn Pro Ala
   610 615 620
   Asn Lys Arg Arg Ser Thr Leu Ile Thr Val Leu Asn Ile Ser Glu Ile
   625 630 635 640
   Glu Ser Arg Phe Tyr Lys His Pro Phe Thr Cys Phe Ala Lys Asn Thr
   645 650 655
   His Gly Ile Asp Ala Ala Tyr Ile Gln Leu Ile Tyr Pro Val Thr Asn
   660 665 670
   Ser Gly Glu Ser Lys Tyr Gly Pro Pro Cys Pro Pro Cys Pro Ala Pro
   675 680 685
   Glu Phe Leu Gly Gly Pro Ser Val Phe Leu Phe Pro Pro Lys Pro Lys
   690 695 700
   Asp Thr Leu Met Ile Ser Arg Thr Pro Glu Val Thr Cys Val Val Val
   705 710 715 720
   Asp Val Ser Gln Glu Asp Pro Glu Val Gln Phe Asn Trp Tyr Val Asp
   725 730 735
   Gly Val Glu Val His Asn Ala Lys Thr Lys Pro Arg Glu Glu Gln Phe
   740 745 750
   Asn Ser Thr Tyr Arg Val Val Ser Val Leu Thr Val Leu His Gln Asp
   755 760 765
   Trp Leu Asn Gly Lys Glu Tyr Lys Cys Lys Val Ser Asn Lys Gly Leu
   770 775 780
   Pro Ser Ser Ile Glu Lys Thr Ile Ser Lys Ala Lys Gly Gln Pro Arg
   785 790 795 800
   Glu Pro Gln Val Tyr Thr Leu Pro Pro Ser Gln Glu Glu Met Thr Lys
   805 810 815
   Asn Gln Val Ser Leu Thr Cys Leu Val Lys Gly Phe Tyr Pro Ser Asp
   820 825 830
   Ile Ala Val Glu Trp Glu Ser Asn Gly Gln Pro Glu Asn Asn Tyr Lys
               835                 840                 845
   Thr Thr Pro Pro Val Leu Asp Ser Asp Gly Ser Phe Phe Leu Tyr Ser
           850                 855                 860
   Arg Leu Thr Val Asp Lys Ser Arg Trp Gln Glu Gly Asn Val Phe Ser
   865                 870                 875                 880
   Cys Ser Val Met His Glu Ala Leu His Asn His Tyr Thr Gln Lys Ser
                   885                 890                 895
   Leu Ser Leu Ser Leu Gly Lys
               900
<210> 15
   <211> 2749
   <212> DNA
   <213> Artificial Sequence
<220>
   <223> Synthetic
<400> 15
   datggtgcgc ttgtacgtgt tggtaatggg agtttctgcc ttcacccttc agcctgcggc 60
   acacacaggg gctgccagaa gctgccggtt tcgtgggagg cattacaagc gggagttcag 120
   gctggaaggg gagcctgtag ccctgaggtg cccccaggtg ccctactggt tgtgggcctc 180
   tgtcagcccc cgcatcaacc tgacatggca taaaaatgac tctgctagga cggtcccagg 240
   agaagaagag acacggatgt gggcccagga cggtgctctg tggcttctgc cagccttgca 300
   ggaggactct ggcacctacg tctgcactac tagaaatgct tcttactgtg acaaaatgtc 360
   cattgagctc agagtttttg agaatacaga tgctttcctg ccgttcatct catacccgca 420
   aattttaacc ttgtcaacct ctggggtatt agtatgccct gacctgagtg aattcacccg 480
   tgacaaaact gacgtgaaga ttcaatggta caaggattct cttcttttgg ataaagacaa 540
   tgagaaattt ctaagtgtga gggggaccac tcacttactc gtacacgatg tggccctgga 600
   agatgctggc tattaccgct gtgtcctgac atttgcccat gaaggccagc aatacaacat 660
   cactaggagt attgagctac gcatcaagaa aaaaaaagaa gagaccattc ctgtgatcat 720
   ttcccccctc aagaccatat cagcttctct ggggtcaaga ctgacaatcc catgtaaggt 780
   gtttctggga accggcacac ccttaaccac catgctgtgg tggacggcca atgacaccca 840
   catagagagc gcctacccgg gaggccgcgt gaccgagggg ccacgccagg aatattcaga 900
   aaataatgag aactacattg aagtgccatt gatttttgat cctgtcacaa gagaggattt 960
   gcacatggat tttaaatgtg ttgtccataa taccctgagt tttcagacac tacgcaccac 1020
   agtcaaggaa gcctcctcca cgttctcaga acgctgcgat gactggggac tagacaccat 1080
   gaggcaaatc caagtgtttg aagatgagcc agctcgcatc aagtgcccac tctttgaaca 1140
   cttcttgaaa ttcaactaca gcacagccca ttcagctggc cttactctga tctggtattg 1200
   gactaggcag gaccgggacc ttgaggagcc aattaacttc cgcctccccg agaaccgcat 1260
   tagtaaggag aaagatgtgc tgtggttccg gcccactctc ctcaatgaca ctggcaacta 1320
   tacctgcatg ttaaggaaca ctacatattg cagcaaagtt gcatttccct tggaagttgt 1380
   tcaaaaagac agctgtttca attcccccat gaaactccca gtgcataaac tgtatataga 1440
   atatggcatt cagaggatca cttgtccaaa tgtagatgga tattttcctt ccagtgtcaa 1500
   accgactatc acttggtata tgggctgtta taaaatacag aattttaata atgtaatacc 1560
   cgaaggtatg aacttgagtt tcctcattgc cttaatttca aataatggaa attacacatg 1620
   tgttgttaca tatccagaaa atggacgtac gtttcatctc accaggactc tgactgtaaa 1680
   ggtagtaggc tctccaaaaa atgcagtgcc ccctgtgatc cattcaccta atgatcatgt 1740
   ggtctatgag aaagaaccag gagaggagct actcattccc tgtacggtct attttagttt 1800
   tctgatggat tctcgcaatg aggtttggtg gaccattgat ggaaaaaaac ctgatgacat 1860
   cactattgat gtcaccatta acgaaagtat aagtcatagt agaacagaag atgaaacaag 1920
   aactcagatt ttgagcatca agaaagttac ctctgaggat ctcaagcgca gctatgtctg 1980
   tcatgctaga agtgccaaag gcgaagttgc caaagcagcc aaggtgaagc agaaagtgcc 2040
   agctccaaga tacacagtgt ccggagacaa aactcacaca tgcccaccgt gcccagcacc 2100
   tgaactcctg gggggaccgt cagtcttcct cttcccccca aaacccaagg acaccctcat 2160
   gatctcccgg acccctgagg tcacatgcgt ggtggtggac gtgagccacg aagaccctga 2220
   ggtcaagttc aactggtacg tggacggcgt ggaggtgcat aatgccaaga caaagccgcg 2280
   ggaggagcag tacaacagca cgtaccgtgt ggtcagcgtc ctcaccgtcc tgcaccagga 2340
   ctggctgaat ggcaaggagt acaagtgcaa ggtctccaac aaagccctcc cagcccccat 2400
   cgagaaaacc atctccaaag ccaaagggca gccccgagaa ccacaggtgt acaccctgcc 2460
   cccatcccgg gatgagctga ccaagaacca ggtcagcctg acctgcctgg tcaaaggctt 2520
   ctatcccagc gacatcgccg tggagtggga gagcaatggg cagccggaga acaactacaa 2580
   gaccacgcct cccgtgctgg actccgacgg ctccttcttc ctctatagca agctcaccgt 2640
   ggacaagagc aggtggcagc aggggaacgt cttctcatgc tccgtgatgc atgaggctct 2700
   gcacaaccac tacacgcaga agagcctctc cctgtctccg ggtaaatga 2749
<210> 16
   <211> 916
   <212> PRT
   <213> Artificial Sequence
<220>
   <223> Synthetic
<400> 16
   Pro Met Val Arg Leu Tyr Val Leu Val Met Gly Val Ser Ala Phe Thr
    1               5                  10                  15
   Leu Gln Pro Ala Ala His Thr Gly Ala Ala Arg Ser Cys Arg Phe Arg
               20                  25                  30
   Gly Arg His Tyr Lys Arg Glu Phe Arg Leu Glu Gly Glu Pro Val Ala
           35                  40                  45
   Leu Arg Cys Pro Gln Val Pro Tyr Trp Leu Trp Ala Ser Val Ser Pro
       50                  55                  60
   Arg Ile Asn Leu Thr Trp His Lys Asn Asp Ser Ala Arg Thr Val Pro
   65                  70                  75                  80
   Gly Glu Glu Glu Thr Arg Met Trp Ala Gln Asp Gly Ala Leu Trp Leu
                   85                  90                  95
   Leu Pro Ala Leu Gln Glu Asp Ser Gly Thr Tyr Val Cys Thr Thr Arg
               100                 105                 110
   Asn Ala Ser Tyr Cys Asp Lys Met Ser Ile Glu Leu Arg Val Phe Glu
           115                 120                 125
   Asn Thr Asp Ala Phe Leu Pro Phe Ile Ser Tyr Pro Gln Ile Leu Thr
       130                 135                 140
   Leu Ser Thr Ser Gly Val Leu Val Cys Pro Asp Leu Ser Glu Phe Thr
   145                 150                 155                 160
   Arg Asp Lys Thr Asp Val Lys Ile Gln Trp Tyr Lys Asp Ser Leu Leu
                   165                 170                 175
   Leu Asp Lys Asp Asn Glu Lys Phe Leu Ser Val Arg Gly Thr Thr His
               180                 185                 190
   Leu Leu Val His Asp Val Ala Leu Glu Asp Ala Gly Tyr Tyr Arg Cys
           195                 200                 205
   Val Leu Thr Phe Ala His Glu Gly Gln Gln Tyr Asn Ile Thr Arg Ser
       210                 215                 220
   Ile Glu Leu Arg Ile Lys Lys Lys Lys Glu Glu Thr Ile Pro Val Ile
   225                 230                 235                 240
   Ile Ser Pro Leu Lys Thr Ile Ser Ala Ser Leu Gly Ser Arg Leu Thr
                   245                 250                 255
   Ile Pro Cys Lys Val Phe Leu Gly Thr Gly Thr Pro Leu Thr Thr Met
               260                 265                 270
   Leu Trp Trp Thr Ala Asn Asp Thr His Ile Glu Ser Ala Tyr Pro Gly
           275                 280                 285
   Gly Arg Val Thr Glu Gly Pro Arg Gln Glu Tyr Ser Glu Asn Asn Glu
       290                 295                 300
   Asn Tyr Ile Glu Val Pro Leu Ile Phe Asp Pro Val Thr Arg Glu Asp
   305                 310                 315                 320
   Leu His Met Asp Phe Lys Cys Val Val His Asn Thr Leu Ser Phe Gln
                   325                 330                 335
   Thr Leu Arg Thr Thr Val Lys Glu Ala Ser Ser Thr Phe Ser Glu Arg
               340                 345                 350
   Cys Asp Asp Trp Gly Leu Asp Thr Met Arg Gln Ile Gln Val Phe Glu
           355                 360                 365
   Asp Glu Pro Ala Arg Ile Lys Cys Pro Leu Phe Glu His Phe Leu Lys
       370                 375                 380
   Phe Asn Tyr Ser Thr Ala His Ser Ala Gly Leu Thr Leu Ile Trp Tyr
   385                 390                 395                 400
   Trp Thr Arg Gln Asp Arg Asp Leu Glu Glu Pro Ile Asn Phe Arg Leu
                   405                 410                 415
   Pro Glu Asn Arg Ile Ser Lys Glu Lys Asp Val Leu Trp Phe Arg Pro
               420                 425                 430
   Thr Leu Leu Asn Asp Thr Gly Asn Tyr Thr Cys Met Leu Arg Asn Thr
           435                 440                 445
   Thr Tyr Cys Ser Lys Val Ala Phe Pro Leu Glu Val Val Gln Lys Asp
       450                 455                 460
   Ser Cys Phe Asn Ser Pro Met Lys Leu Pro Val His Lys Leu Tyr Ile
   465                 470                 475                 480
   Glu Tyr Gly Ile Gln Arg Ile Thr Cys Pro Asn Val Asp Gly Tyr Phe
                   485                 490                 495
   Pro Ser Ser Val Lys Pro Thr Ile Thr Trp Tyr Met Gly Cys Tyr Lys
               500                 505                 510
   Ile Gln Asn Phe Asn Asn Val Ile Pro Glu Gly Met Asn Leu Ser Phe
           515                 520                 525
   Leu Ile Ala Leu Ile Ser Asn Asn Gly Asn Tyr Thr Cys Val Val Thr
       530                 535                 540
   Tyr Pro Glu Asn Gly Arg Thr Phe His Leu Thr Arg Thr Leu Thr Val
   545                 550                 555                 560
   Lys Val Val Gly Ser Pro Lys Asn Ala Val Pro Pro Val Ile His Ser
                   565                 570                 575
   Pro Asn Asp His Val Val Tyr Glu Lys Glu Pro Gly Glu Glu Leu Leu
               580                 585                 590
   Ile Pro Cys Thr Val Tyr Phe Ser Phe Leu Met Asp Ser Arg Asn Glu
           595                 600                 605
   Val Trp Trp Thr Ile Asp Gly Lys Lys Pro Asp Asp Ile Thr Ile Asp
       610                 615                 620
   Val Thr Ile Asn Glu Ser Ile Ser His Ser Arg Thr Glu Asp Glu Thr
   625                 630                 635                 640
   Arg Thr Gln Ile Leu Ser Ile Lys Lys Val Thr Ser Glu Asp Leu Lys
                   645                 650                 655
   Arg Ser Tyr Val Cys His Ala Arg Ser Ala Lys Gly Glu Val Ala Lys
               660                 665                 670
   Ala Ala Lys Val Lys Gln Lys Val Pro Ala Pro Arg Tyr Thr Val Ser
           675                 680                 685
   Gly Asp Lys Thr His Thr Cys Pro Pro Cys Pro Ala Pro Glu Leu Leu
       690                 695                 700
   Gly Gly Pro Ser Val Phe Leu Phe Pro Pro Lys Pro Lys Asp Thr Leu
   705                 710                 715                 720
   Met Ile Ser Arg Thr Pro Glu Val Thr Cys Val Val Val Asp Val Ser
                   725                 730                 735
   His Glu Asp Pro Glu Val Lys Phe Asn Trp Tyr Val Asp Gly Val Glu
               740                 745                 750
   Val His Asn Ala Lys Thr Lys Pro Arg Glu Glu Gln Tyr Asn Ser Thr
           755                 760                 765
   Tyr Arg Val Val Ser Val Leu Thr Val Leu His Gln Asp Trp Leu Asn
       770                 775                 780
   Gly Lys Glu Tyr Lys Cys Lys Val Ser Asn Lys Ala Leu Pro Ala Pro
   785                 790                 795                 800
   Ile Glu Lys Thr Ile Ser Lys Ala Lys Gly Gln Pro Arg Glu Pro Gln
                   805                 810                 815
   Val Tyr Thr Leu Pro Pro Ser Arg Asp Glu Leu Thr Lys Asn Gln Val
               820                 825                 830
   Ser Leu Thr Cys Leu Val Lys Gly Phe Tyr Pro Ser Asp Ile Ala Val
           835                 840                 845
   Glu Trp Glu Ser Asn Gly Gln Pro Glu Asn Asn Tyr Lys Thr Thr Pro
       850                 855                 860
   Pro Val Leu Asp Ser Asp Gly Ser Phe Phe Leu Tyr Ser Lys Leu Thr
   865                 870                 875                 880
   Val Asp Lys Ser Arg Trp Gln Gln Gly Asn Val Phe Ser Cys Ser Val
                   885                 890                 895
   Met His Glu Ala Leu His Asn His Tyr Thr Gln Lys Ser Leu Ser Leu
               900                 905                 910
   Ser Pro Gly Lys
           915
<210> 17
   <211> 2755
   <212> DNA
   <213> Artificial Sequence
<220>
   <223> Synthetic
<400> 17
   datggtgcgc ttgtacgtgt tggtaatggg agtttctgcc ttcacccttc agcctgcggc 60
   acacacaggg gctgccagaa gctgccggtt tcgtgggagg cattacaagc gggagttcag 120
   gctggaaggg gagcctgtag ccctgaggtg cccccaggtg ccctactggt tgtgggcctc 180
   tgtcagcccc cgcatcaacc tgacatggca taaaaatgac tctgctagga cggtcccagg 240
   agaagaagag acacggatgt gggcccagga cggtgctctg tggcttctgc cagccttgca 300
   ggaggactct ggcacctacg tctgcactac tagaaatgct tcttactgtg acaaaatgtc 360
   cattgagctc agagtttttg agaatacaga tgctttcctg ccgttcatct catacccgca 420
   aattttaacc ttgtcaacct ctggggtatt agtatgccct gacctgagtg aattcacccg 480
   tgacaaaact gacgtgaaga ttcaatggta caaggattct cttcttttgg ataaagacaa 540
   tgagaaattt ctaagtgtga gggggaccac tcacttactc gtacacgatg tggccctgga 600
   agatgctggc tattaccgct gtgtcctgac atttgcccat gaaggccagc aatacaacat 660
   cactaggagt attgagctac gcatcaagaa aaaaaaagaa gagaccattc ctgtgatcat 720
   ttcccccctc aagaccatat cagcttctct ggggtcaaga ctgacaatcc catgtaaggt 780
   gtttctggga accggcacac ccttaaccac catgctgtgg tggacggcca atgacaccca 840
   catagagagc gcctacccgg gaggccgcgt gaccgagggg ccacgccagg aatattcaga 900
   aaataatgag aactacattg aagtgccatt gatttttgat cctgtcacaa gagaggattt 960
   gcacatggat tttaaatgtg ttgtccataa taccctgagt tttcagacac tacgcaccac 1020
   agtcaaggaa gcctcctcca cgttctcaga acgctgcgat gactggggac tagacaccat 1080
   gaggcaaatc caagtgtttg aagatgagcc agctcgcatc aagtgcccac tctttgaaca 1140
   cttcttgaaa ttcaactaca gcacagccca ttcagctggc cttactctga tctggtattg 1200
   gactaggcag gaccgggacc ttgaggagcc aattaacttc cgcctccccg agaaccgcat 1260
   tagtaaggag aaagatgtgc tgtggttccg gcccactctc ctcaatgaca ctggcaacta 1320
   tacctgcatg ttaaggaaca ctacatattg cagcaaagtt gcatttccct tggaagttgt 1380
   tcaaaaagac agctgtttca attcccccat gaaactccca gtgcataaac tgtatataga 1440
   atatggcatt cagaggatca cttgtccaaa tgtagatgga tattttcctt ccagtgtcaa 1500
   accgactatc acttggtata tgggctgtta taaaatacag aattttaata atgtaatacc 1560
   cgaaggtatg aacttgagtt tcctcattgc cttaatttca aataatggaa attacacatg 1620
   tgttgttaca tatccagaaa atggacgtac gtttcatctc accaggactc tgactgtaaa 1680
   ggtagtaggc tctccaaaaa atgcagtgcc ccctgtgatc cattcaccta atgatcatgt 1740
   ggtctatgag aaagaaccag gagaggagct actcattccc tgtacggtct attttagttt 1800
   tctgatggat tctcgcaatg aggtttggtg gaccattgat ggaaaaaaac ctgatgacat 1860
   cactattgat gtcaccatta acgaaagtat aagtcatagt agaacagaag atgaaacaag 1920
   aactcagatt ttgagcatca agaaagttac ctctgaggat ctcaagcgca gctatgtctg 1980
   tcatgctaga agtgccaaag gcgaagttgc caaagcagcc aaggtgaagc agaaagtgcc 2040
   agctccaaga tacacagtgt ccggagagtc caaatacggt ccgccatgcc catcatgccc 2100
   agcacctgag ttcctggggg gaccatcagt cttcctgttc cccccaaaac ccaaggacac 2160
   tctcatgatc tcccggaccc ctgaggtcac gtgcgtggtg gtggacgtga gccaggaaga 2220
   ccccgaggtc cagttcaact ggtacgtgga tggcgtggag gtgcataatg ccaagacaaa 2280
   gccgcgggag gagcagttca acagcacgta ccgtgtggtc agcgtcctca ccgtcctgca 2340
   ccaggactgg ctgaacggca aggagtacaa gtgcaaggtc tccaacaaag gcctcccgtc 2400
   ctccatcgag aaaaccatct ccaaagccaa agggcagccc cgagagccac aggtgtacac 2460
   cctgccccca tcccaggagg agatgaccaa gaaccaggtc agcctgacct gcctggtcaa 2520
   aggcttctac cccagcgaca tcgccgtgga gtgggagagc aatgggcagc cggagaacaa 2580
   ctacaagacc acgcctcccg tgctggactc cgacggctcc ttcttcctct acagcaggct 2640
   aaccgtggac aagagcaggt ggcaggaggg gaatgtcttc tcatgctccg tgatgcatga 2700
   ggctctgcac aaccactaca cacagaagag cctctccctg tctctgggta aatga      2755
<210> 18
   <211> 918
   <212> PRT
   <213> Artificial Sequence
<220>
   <223> Synthetic
<400> 18
   Pro Met Val Arg Leu Tyr Val Leu Val Met Gly Val Ser Ala Phe Thr
    1               5                  10                  15
   Leu Gln Pro Ala Ala His Thr Gly Ala Ala Arg Ser Cys Arg Phe Arg
               20                  25                  30
   Gly Arg His Tyr Lys Arg Glu Phe Arg Leu Glu Gly Glu Pro Val Ala
           35                  40                  45
   Leu Arg Cys Pro Gln Val Pro Tyr Trp Leu Trp Ala Ser Val Ser Pro
       50                  55                  60
   Arg Ile Asn Leu Thr Trp His Lys Asn Asp Ser Ala Arg Thr Val Pro
   65                  70                  75                  80
   Gly Glu Glu Glu Thr Arg Met Trp Ala Gln Asp Gly Ala Leu Trp Leu
                   85                  90                  95
   Leu Pro Ala Leu Gln Glu Asp Ser Gly Thr Tyr Val Cys Thr Thr Arg
               100                 105                 110
   Asn Ala Ser Tyr Cys Asp Lys Met Ser Ile Glu Leu Arg Val Phe Glu
           115                 120                 125
   Asn Thr Asp Ala Phe Leu Pro Phe Ile Ser Tyr Pro Gln Ile Leu Thr
       130                 135                 140
   Leu Ser Thr Ser Gly Val Leu Val Cys Pro Asp Leu Ser Glu Phe Thr
   145                 150                 155                 160
   Arg Asp Lys Thr Asp Val Lys Ile Gln Trp Tyr Lys Asp Ser Leu Leu
                   165                 170                 175
   Leu Asp Lys Asp Asn Glu Lys Phe Leu Ser Val Arg Gly Thr Thr His
               180                 185                 190
   Leu Leu Val His Asp Val Ala Leu Glu Asp Ala Gly Tyr Tyr Arg Cys
           195                 200                 205
   Val Leu Thr Phe Ala His Glu Gly Gln Gln Tyr Asn Ile Thr Arg Ser
       210                 215                 220
   Ile Glu Leu Arg Ile Lys Lys Lys Lys Glu Glu Thr Ile Pro Val Ile
   225                 230                 235                 240
   Ile Ser Pro Leu Lys Thr Ile Ser Ala Ser Leu Gly Ser Arg Leu Thr
                   245                 250                 255
   Ile Pro Cys Lys Val Phe Leu Gly Thr Gly Thr Pro Leu Thr Thr Met
               260                 265                 270
   Leu Trp Trp Thr Ala Asn Asp Thr His Ile Glu Ser Ala Tyr Pro Gly
           275                 280                 285
   Gly Arg Val Thr Glu Gly Pro Arg Gln Glu Tyr Ser Glu Asn Asn Glu
       290                 295                 300
   Asn Tyr Ile Glu Val Pro Leu Ile Phe Asp Pro Val Thr Arg Glu Asp
   305                 310                 315                 320
   Leu His Met Asp Phe Lys Cys Val Val His Asn Thr Leu Ser Phe Gln
                   325                 330                 335
   Thr Leu Arg Thr Thr Val Lys Glu Ala Ser Ser Thr Phe Ser Glu Arg
               340                 345                 350
   Cys Asp Asp Trp Gly Leu Asp Thr Met Arg Gln Ile Gln Val Phe Glu
           355                 360                 365
   Asp Glu Pro Ala Arg Ile Lys Cys Pro Leu Phe Glu His Phe Leu Lys
       370                 375                 380
   Phe Asn Tyr Ser Thr Ala His Ser Ala Gly Leu Thr Leu Ile Trp Tyr
   385                 390                 395                 400
   Trp Thr Arg Gln Asp Arg Asp Leu Glu Glu Pro Ile Asn Phe Arg Leu
                   405                 410                 415
   Pro Glu Asn Arg Ile Ser Lys Glu Lys Asp Val Leu Trp Phe Arg Pro
               420                 425                 430
   Thr Leu Leu Asn Asp Thr Gly Asn Tyr Thr Cys Met Leu Arg Asn Thr
           435                 440                 445
   Thr Tyr Cys Ser Lys Val Ala Phe Pro Leu Glu Val Val Gln Lys Asp
       450                 455                 460
   Ser Cys Phe Asn Ser Pro Met Lys Leu Pro Val His Lys Leu Tyr Ile
   465                 470                 475                 480
   Glu Tyr Gly Ile Gln Arg Ile Thr Cys Pro Asn Val Asp Gly Tyr Phe
                   485                 490                 495
   Pro Ser Ser Val Lys Pro Thr Ile Thr Trp Tyr Met Gly Cys Tyr Lys
               500                 505                 510
   Ile Gln Asn Phe Asn Asn Val Ile Pro Glu Gly Met Asn Leu Ser Phe
           515                 520                 525
   Leu Ile Ala Leu Ile Ser Asn Asn Gly Asn Tyr Thr Cys Val Val Thr
       530                 535                 540
   Tyr Pro Glu Asn Gly Arg Thr Phe His Leu Thr Arg Thr Leu Thr Val
   545                 550                 555                 560
   Lys Val Val Gly Ser Pro Lys Asn Ala Val Pro Pro Val Ile His Ser
                   565                 570                 575
   Pro Asn Asp His Val Val Tyr Glu Lys Glu Pro Gly Glu Glu Leu Leu
               580                 585                 590
   Ile Pro Cys Thr Val Tyr Phe Ser Phe Leu Met Asp Ser Arg Asn Glu
           595                 600                 605
   Val Trp Trp Thr Ile Asp Gly Lys Lys Pro Asp Asp Ile Thr Ile Asp
       610                 615                 620
   Val Thr Ile Asn Glu Ser Ile Ser His Ser Arg Thr Glu Asp Glu Thr
   625                 630                 635                 640
   Arg Thr Gln Ile Leu Ser Ile Lys Lys Val Thr Ser Glu Asp Leu Lys
                   645                 650                 655
   Arg Ser Tyr Val Cys His Ala Arg Ser Ala Lys Gly Glu Val Ala Lys
               660                 665                 670
   Ala Ala Lys Val Lys Gln Lys Val Pro Ala Pro Arg Tyr Thr Val Ser
           675                 680                 685
   Gly Glu Ser Lys Tyr Gly Pro Pro Cys Pro Ser Cys Pro Ala Pro Glu
       690                 695                 700
   Phe Leu Gly Gly Pro Ser Val Phe Leu Phe Pro Pro Lys Pro Lys Asp
   705                 710                 715                 720
   Thr Leu Met Ile Ser Arg Thr Pro Glu Val Thr Cys Val Val Val Asp
                   725                 730                 735
   Val Ser Gln Glu Asp Pro Glu Val Gln Phe Asn Trp Tyr Val Asp Gly
               740                 745                 750
   Val Glu Val His Asn Ala Lys Thr Lys Pro Arg Glu Glu Gln Phe Asn
           755                 760                 765
   Ser Thr Tyr Arg Val Val Ser Val Leu Thr Val Leu His Gln Asp Trp
       770                 775                 780
   Leu Asn Gly Lys Glu Tyr Lys Cys Lys Val Ser Asn Lys Gly Leu Pro
   785                 790                 795                 800
   Ser Ser Ile Glu Lys Thr Ile Ser Lys Ala Lys Gly Gln Pro Arg Glu
                   805                 810                 815
   Pro Gln Val Tyr Thr Leu Pro Pro Ser Gln Glu Glu Met Thr Lys Asn
               820                 825                 830
   Gln Val Ser Leu Thr Cys Leu Val Lys Gly Phe Tyr Pro Ser Asp Ile
           835                 840                 845
   Ala Val Glu Trp Glu Ser Asn Gly Gln Pro Glu Asn Asn Tyr Lys Thr
       850                 855                 860
   Thr Pro Pro Val Leu Asp Ser Asp Gly Ser Phe Phe Leu Tyr Ser Arg
   865                 870                 875                 880
   Leu Thr Val Asp Lys Ser Arg Trp Gln Glu Gly Asn Val Phe Ser Cys
                   885                 890                 895
   Ser Val Met His Glu Ala Leu His Asn His Tyr Thr Gln Lys Ser Leu
               900                 905                 910
   Ser Leu Ser Leu Gly Lys
           915
<210> 19
   <211> 2755
   <212> DNA
   <213> Artificial Sequence
<220>
   <223> Synthetic
<400> 19
   datggtgcgc ttgtacgtgt tggtaatggg agtttctgcc ttcacccttc agcctgcggc 60
   acacacaggg gctgccagaa gctgccggtt tcgtgggagg cattacaagc gggagttcag 120
   gctggaaggg gagcctgtag ccctgaggtg cccccaggtg ccctactggt tgtgggcctc 180
   tgtcagcccc cgcatcaacc tgacatggca taaaaatgac tctgctagga cggtcccagg 240
   agaagaagag acacggatgt gggcccagga cggtgctctg tggcttctgc cagccttgca 300
   ggaggactct ggcacctacg tctgcactac tagaaatgct tcttactgtg acaaaatgtc 360
   cattgagctc agagtttttg agaatacaga tgctttcctg ccgttcatct catacccgca 420
   aattttaacc ttgtcaacct ctggggtatt agtatgccct gacctgagtg aattcacccg 480
   tgacaaaact gacgtgaaga ttcaatggta caaggattct cttcttttgg ataaagacaa 540
   tgagaaattt ctaagtgtga gggggaccac tcacttactc gtacacgatg tggccctgga 600
   agatgctggc tattaccgct gtgtcctgac atttgcccat gaaggccagc aatacaacat 660
   cactaggagt attgagctac gcatcaagaa aaaaaaagaa gagaccattc ctgtgatcat 720
   ttcccccctc aagaccatat cagcttctct ggggtcaaga ctgacaatcc catgtaaggt 780
   gtttctggga accggcacac ccttaaccac catgctgtgg tggacggcca atgacaccca 840
   catagagagc gcctacccgg gaggccgcgt gaccgagggg ccacgccagg aatattcaga 900
   aaataatgag aactacattg aagtgccatt gatttttgat cctgtcacaa gagaggattt 960
   gcacatggat tttaaatgtg ttgtccataa taccctgagt tttcagacac tacgcaccac 1020
   agtcaaggaa gcctcctcca cgttctcaga acgctgcgat gactggggac tagacaccat 1080
   gaggcaaatc caagtgtttg aagatgagcc agctcgcatc aagtgcccac tctttgaaca 1140
   cttcttgaaa ttcaactaca gcacagccca ttcagctggc cttactctga tctggtattg 1200
   gactaggcag gaccgggacc ttgaggagcc aattaacttc cgcctccccg agaaccgcat 1260
   tagtaaggag aaagatgtgc tgtggttccg gcccactctc ctcaatgaca ctggcaacta 1320
   tacctgcatg ttaaggaaca ctacatattg cagcaaagtt gcatttccct tggaagttgt 1380
   tcaaaaagac agctgtttca attcccccat gaaactccca gtgcataaac tgtatataga 1440
   atatggcatt cagaggatca cttgtccaaa tgtagatgga tattttcctt ccagtgtcaa 1500
   accgactatc acttggtata tgggctgtta taaaatacag aattttaata atgtaatacc 1560
   cgaaggtatg aacttgagtt tcctcattgc cttaatttca aataatggaa attacacatg 1620
   tgttgttaca tatccagaaa atggacgtac gtttcatctc accaggactc tgactgtaaa 1680
   ggtagtaggc tctccaaaaa atgcagtgcc ccctgtgatc cattcaccta atgatcatgt 1740
   ggtctatgag aaagaaccag gagaggagct actcattccc tgtacggtct attttagttt 1800
   tctgatggat tctcgcaatg aggtttggtg gaccattgat ggaaaaaaac ctgatgacat 1860
   cactattgat gtcaccatta acgaaagtat aagtcatagt agaacagaag atgaaacaag 1920
   aactcagatt ttgagcatca agaaagttac ctctgaggat ctcaagcgca gctatgtctg 1980
   tcatgctaga agtgccaaag gcgaagttgc caaagcagcc aaggtgaagc agaaagtgcc 2040
   agctccaaga tacacagtgt ccggagagtc caaatacggt ccgccatgcc caccatgccc 2100
   agcacctgag ttcctggggg gaccatcagt cttcctgttc cccccaaaac ccaaggacac 2160
   tctcatgatc tcccggaccc ctgaggtcac gtgcgtggtg gtggacgtga gccaggaaga 2220
   ccccgaggtc cagttcaact ggtacgtgga tggcgtggag gtgcataatg ccaagacaaa 2280
   gccgcgggag gagcagttca acagcacgta ccgtgtggtc agcgtcctca ccgtcctgca 2340
   ccaggactgg ctgaacggca aggagtacaa gtgcaaggtc tccaacaaag gcctcccgtc 2400
   ctccatcgag aaaaccatct ccaaagccaa agggcagccc cgagagccac aggtgtacac 2460
   cctgccccca tcccaggagg agatgaccaa gaaccaggtc agcctgacct gcctggtcaa 2520
   aggcttctac cccagcgaca tcgccgtgga gtgggagagc aatgggcagc cggagaacaa 2580
   ctacaagacc acgcctcccg tgctggactc cgacggctcc ttcttcctct acagcaggct 2640
   aaccgtggac aagagcaggt ggcaggaggg gaatgtcttc tcatgctccg tgatgcatga 2700
   ggctctgcac aaccactaca cacagaagag cctctccctg tctctgggta aatga      2755
<210> 20
   <211> 918
   <212> PRT
   <213> Artificial Sequence
<220>
   <223> Synthetic
<400> 20
   Pro Met Val Arg Leu Tyr Val Leu Val Met Gly Val Ser Ala Phe Thr
    1               5                  10                  15
   Leu Gln Pro Ala Ala His Thr Gly Ala Ala Arg Ser Cys Arg Phe Arg
               20                  25                  30
   Gly Arg His Tyr Lys Arg Glu Phe Arg Leu Glu Gly Glu Pro Val Ala
           35                  40                  45
   Leu Arg Cys Pro Gln Val Pro Tyr Trp Leu Trp Ala Ser Val Ser Pro
       50                  55                  60
   Arg Ile Asn Leu Thr Trp His Lys Asn Asp Ser Ala Arg Thr Val Pro
   65                  70                  75                  80
   Gly Glu Glu Glu Thr Arg Met Trp Ala Gln Asp Gly Ala Leu Trp Leu
                   85                  90                  95
   Leu Pro Ala Leu Gln Glu Asp Ser Gly Thr Tyr Val Cys Thr Thr Arg
               100                 105                 110
   Asn Ala Ser Tyr Cys Asp Lys Met Ser Ile Glu Leu Arg Val Phe Glu
           115                 120                 125
   Asn Thr Asp Ala Phe Leu Pro Phe Ile Ser Tyr Pro Gln Ile Leu Thr
       130                 135                 140
   Leu Ser Thr Ser Gly Val Leu Val Cys Pro Asp Leu Ser Glu Phe Thr
   145                 150                 155                 160
   Arg Asp Lys Thr Asp Val Lys Ile Gln Trp Tyr Lys Asp Ser Leu Leu
                   165                 170                 175
   Leu Asp Lys Asp Asn Glu Lys Phe Leu Ser Val Arg Gly Thr Thr His
               180                 185                 190
   Leu Leu Val His Asp Val Ala Leu Glu Asp Ala Gly Tyr Tyr Arg Cys
           195                 200                 205
   Val Leu Thr Phe Ala His Glu Gly Gln Gln Tyr Asn Ile Thr Arg Ser
       210                 215                 220
   Ile Glu Leu Arg Ile Lys Lys Lys Lys Glu Glu Thr Ile Pro Val Ile
   225                 230                 235                 240
   Ile Ser Pro Leu Lys Thr Ile Ser Ala Ser Leu Gly Ser Arg Leu Thr
                   245                 250                 255
   Ile Pro Cys Lys Val Phe Leu Gly Thr Gly Thr Pro Leu Thr Thr Met
               260                 265                 270
   Leu Trp Trp Thr Ala Asn Asp Thr His Ile Glu Ser Ala Tyr Pro Gly
           275                 280                 285
   Gly Arg Val Thr Glu Gly Pro Arg Gln Glu Tyr Ser Glu Asn Asn Glu
       290                 295                 300
   Asn Tyr Ile Glu Val Pro Leu Ile Phe Asp Pro Val Thr Arg Glu Asp
   305                 310                 315                 320
   Leu His Met Asp Phe Lys Cys Val Val His Asn Thr Leu Ser Phe Gln
                   325                 330                 335
   Thr Leu Arg Thr Thr Val Lys Glu Ala Ser Ser Thr Phe Ser Glu Arg
               340                 345                 350
   Cys Asp Asp Trp Gly Leu Asp Thr Met Arg Gln Ile Gln Val Phe Glu
           355                 360                 365
   Asp Glu Pro Ala Arg Ile Lys Cys Pro Leu Phe Glu His Phe Leu Lys
       370                 375                 380
   Phe Asn Tyr Ser Thr Ala His Ser Ala Gly Leu Thr Leu Ile Trp Tyr
   385                 390                 395                 400
   Trp Thr Arg Gln Asp Arg Asp Leu Glu Glu Pro Ile Asn Phe Arg Leu
                   405                 410                 415
   Pro Glu Asn Arg Ile Ser Lys Glu Lys Asp Val Leu Trp Phe Arg Pro
               420                 425                 430
   Thr Leu Leu Asn Asp Thr Gly Asn Tyr Thr Cys Met Leu Arg Asn Thr
           435                 440                 445
   Thr Tyr Cys Ser Lys Val Ala Phe Pro Leu Glu Val Val Gln Lys Asp
       450                 455                 460
   Ser Cys Phe Asn Ser Pro Met Lys Leu Pro Val His Lys Leu Tyr Ile
   465                 470                 475                 480
   Glu Tyr Gly Ile Gln Arg Ile Thr Cys Pro Asn Val Asp Gly Tyr Phe
                   485                 490                 495
   Pro Ser Ser Val Lys Pro Thr Ile Thr Trp Tyr Met Gly Cys Tyr Lys
               500                 505                 510
   Ile Gln Asn Phe Asn Asn Val Ile Pro Glu Gly Met Asn Leu Ser Phe
           515                 520                 525
   Leu Ile Ala Leu Ile Ser Asn Asn Gly Asn Tyr Thr Cys Val Val Thr
       530                 535                 540
   Tyr Pro Glu Asn Gly Arg Thr Phe His Leu Thr Arg Thr Leu Thr Val
   545                 550                 555                 560
   Lys Val Val Gly Ser Pro Lys Asn Ala Val Pro Pro Val Ile His Ser
                   565                 570                 575
   Pro Asn Asp His Val Val Tyr Glu Lys Glu Pro Gly Glu Glu Leu Leu
               580                 585                 590
   Ile Pro Cys Thr Val Tyr Phe Ser Phe Leu Met Asp Ser Arg Asn Glu
           595                 600                 605
   Val Trp Trp Thr Ile Asp Gly Lys Lys Pro Asp Asp Ile Thr Ile Asp
       610                 615                 620
   Val Thr Ile Asn Glu Ser Ile Ser His Ser Arg Thr Glu Asp Glu Thr
   625                 630                 635                 640
   Arg Thr Gln Ile Leu Ser Ile Lys Lys Val Thr Ser Glu Asp Leu Lys
                   645                 650                 655
   Arg Ser Tyr Val Cys His Ala Arg Ser Ala Lys Gly Glu Val Ala Lys
               660                 665                 670
   Ala Ala Lys Val Lys Gln Lys Val Pro Ala Pro Arg Tyr Thr Val Ser
           675                 680                 685
   Gly Glu Ser Lys Tyr Gly Pro Pro Cys Pro Pro Cys Pro Ala Pro Glu
       690                 695                 700
   Phe Leu Gly Gly Pro Ser Val Phe Leu Phe Pro Pro Lys Pro Lys Asp
   705                 710                 715                 720
   Thr Leu Met Ile Ser Arg Thr Pro Glu Val Thr Cys Val Val Val Asp
                   725                 730                 735
   Val Ser Gln Glu Asp Pro Glu Val Gln Phe Asn Trp Tyr Val Asp Gly
               740                 745                 750
   Val Glu Val His Asn Ala Lys Thr Lys Pro Arg Glu Glu Gln Phe Asn
           755                 760                 765
   Ser Thr Tyr Arg Val Val Ser Val Leu Thr Val Leu His Gln Asp Trp
       770                 775                 780
   Leu Asn Gly Lys Glu Tyr Lys Cys Lys Val Ser Asn Lys Gly Leu Pro
   785                 790                 795                 800
   Ser Ser Ile Glu Lys Thr Ile Ser Lys Ala Lys Gly Gln Pro Arg Glu
                   805                 810                 815
   Pro Gln Val Tyr Thr Leu Pro Pro Ser Gln Glu Glu Met Thr Lys Asn
               820                 825                 830
   Gln Val Ser Leu Thr Cys Leu Val Lys Gly Phe Tyr Pro Ser Asp Ile
           835                 840                 845
   Ala Val Glu Trp Glu Ser Asn Gly Gln Pro Glu Asn Asn Tyr Lys Thr
       850                 855                 860
   Thr Pro Pro Val Leu Asp Ser Asp Gly Ser Phe Phe Leu Tyr Ser Arg
   865                 870                 875                 880
   Leu Thr Val Asp Lys Ser Arg Trp Gln Glu Gly Asn Val Phe Ser Cys
                   885                 890                 895
   Ser Val Met His Glu Ala Leu His Asn His Tyr Thr Gln Lys Ser Leu
               900                 905                 910
   Ser Leu Ser Leu Gly Lys
           915
<210> 21
   <211> 2749
   <212> DNA
   <213> Artificial Sequence
<220>
   <223> Synthetic
<400> 21
   datggtgctt ctgtggtgtg tagtgagtct ctacttttat ggaatcctgc aaagtgatgc 60
   ctcagaacgc tgcgatgact ggggactaga caccatgagg caaatccaag tgtttgaaga 120
   tgagccagct cgcatcaagt gcccactctt tgaacacttc ttgaaattca actacagcac 180
   agcccattca gctggcctta ctctgatctg gtattggact aggcaggacc gggaccttga 240
   ggagccaatt aacttccgcc tccccgagaa ccgcattagt aaggagaaag atgtgctgtg 300
   gttccggccc actctcctca atgacactgg caactatacc tgcatgttaa ggaacactac 360
   atattgcagc aaagttgcat ttcccttgga agttgttcaa aaagacagct gtttcaattc 420
   ccccatgaaa ctcccagtgc ataaactgta tatagaatat ggcattcaga ggatcacttg 480
   tccaaatgta gatggatatt ttccttccag tgtcaaaccg actatcactt ggtatatggg 540
   ctgttataaa atacagaatt ttaataatgt aatacccgaa ggtatgaact tgagtttcct 600
   cattgcctta atttcaaata atggaaatta cacatgtgtt gttacatatc cagaaaatgg 660
   acgtacgttt catctcacca ggactctgac tgtaaaggta gtaggctctc caaaaaatgc 720
   agtgccccct gtgatccatt cacctaatga tcatgtggtc tatgagaaag aaccaggaga 780
   ggagctactc attccctgta cggtctattt tagttttctg atggattctc gcaatgaggt 840
   ttggtggacc attgatggaa aaaaacctga tgacatcact attgatgtca ccattaacga 900
   aagtataagt catagtagaa cagaagatga aacaagaact cagattttga gcatcaagaa 960
   agttacctct gaggatctca agcgcagcta tgtctgtcat gctagaagtg ccaaaggcga 1020
   agttgccaaa gcagccaagg tgaagcagaa agtgccagct ccaagataca cagtgcacac 1080
   aggggctgcc agaagctgcc ggtttcgtgg gaggcattac aagcgggagt tcaggctgga 1140
   aggggagcct gtagccctga ggtgccccca ggtgccctac tggttgtggg cctctgtcag 1200
   cccccgcatc aacctgacat ggcataaaaa tgactctgct aggacggtcc caggagaaga 1260
   agagacacgg atgtgggccc aggacggtgc tctgtggctt ctgccagcct tgcaggagga 1320
   ctctggcacc tacgtctgca ctactagaaa tgcttcttac tgtgacaaaa tgtccattga 1380
   gctcagagtt tttgagaata cagatgcttt cctgccgttc atctcatacc cgcaaatttt 1440
   aaccttgtca acctctgggg tattagtatg ccctgacctg agtgaattca cccgtgacaa 1500
   aactgacgtg aagattcaat ggtacaagga ttctcttctt ttggataaag acaatgagaa 1560
   atttctaagt gtgaggggga ccactcactt actcgtacac gatgtggccc tggaagatgc 1620
   tggctattac cgctgtgtcc tgacatttgc ccatgaaggc cagcaataca acatcactag 1680
   gagtattgag ctacgcatca agaaaaaaaa agaagagacc attcctgtga tcatttcccc 1740
   cctcaagacc atatcagctt ctctggggtc aagactgaca atcccatgta aggtgtttct 1800
   gggaaccggc acacccttaa ccaccatgct gtggtggacg gccaatgaca cccacataga 1860
   gagcgcctac ccgggaggcc gcgtgaccga ggggccacgc caggaatatt cagaaaataa 1920
   tgagaactac attgaagtgc cattgatttt tgatcctgtc acaagagagg atttgcacat 1980
   ggattttaaa tgtgttgtcc ataataccct gagttttcag acactacgca ccacagtcaa 2040
   ggaagcctcc tccacgttct ccggagacaa aactcacaca tgcccaccgt gcccagcacc 2100
   tgaactcctg gggggaccgt cagtcttcct cttcccccca aaacccaagg acaccctcat 2160
   gatctcccgg acccctgagg tcacatgcgt ggtggtggac gtgagccacg aagaccctga 2220
   ggtcaagttc aactggtacg tggacggcgt ggaggtgcat aatgccaaga caaagccgcg 2280
   ggaggagcag tacaacagca cgtaccgtgt ggtcagcgtc ctcaccgtcc tgcaccagga 2340
   ctggctgaat ggcaaggagt acaagtgcaa ggtctccaac aaagccctcc cagcccccat 2400
   cgagaaaacc atctccaaag ccaaagggca gccccgagaa ccacaggtgt acaccctgcc 2460
   cccatcccgg gatgagctga ccaagaacca ggtcagcctg acctgcctgg tcaaaggctt 2520
   ctatcccagc gacatcgccg tggagtggga gagcaatggg cagccggaga acaactacaa 2580
   gaccacgcct cccgtgctgg actccgacgg ctccttcttc ctctatagca agctcaccgt 2640
   ggacaagagc aggtggcagc aggggaacgt cttctcatgc tccgtgatgc atgaggctct 2700
   gcacaaccac tacacgcaga agagcctctc cctgtctccg ggtaaatga 2749
<210> 22
   <211> 916
   <212> PRT
   <213> Artificial Sequence
<220>
   <223> Synthetic
<400> 22
   Pro Met Val Leu Leu Trp Cys Val Val Ser Leu Tyr Phe Tyr Gly Ile
    1               5                  10                  15
   Leu Gln Ser Asp Ala Ser Glu Arg Cys Asp Asp Trp Gly Leu Asp Thr
               20                  25                  30
   Met Arg Gln Ile Gln Val Phe Glu Asp Glu Pro Ala Arg Ile Lys Cys
           35                  40                  45
   Pro Leu Phe Glu His Phe Leu Lys Phe Asn Tyr Ser Thr Ala His Ser
       50                  55                  60
   Ala Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
           100                 105                 110
   Tyr Thr Cys Met Leu Arg Asn Thr Thr Tyr Cys Ser Lys Val Ala Phe
       115                 120                 125
   Pro Leu Glu Val Val Gln Lys Asp Ser Cys Phe Asn Ser Pro Met Lys
   130                 135                 140
   Leu Pro Val His Lys Leu Tyr Ile Glu Tyr Gly Ile Gln Arg Ile Thr
   145                 150                 155                 160
   Cys Pro Asn Val Asp Gly Tyr Phe Pro Ser Ser Val Lys Pro Thr Ile
                   165                 170                 175
   Thr Trp Tyr Met Gly Cys Tyr Lys Ile Gln Asn Phe Asn Asn Val Ile
               180                 185                 190
   Pro Glu Gly Met Asn Leu Ser Phe Leu Ile Ala Leu Ile Ser Asn Asn
           195                 200                 205
   Gly Asn Tyr Thr Cys Val Val Thr Tyr Pro Glu Asn Gly Arg Thr Phe
       210                 215                 220
   His Leu Thr Arg Thr Leu Thr Val Lys Val Val Gly Ser Pro Lys Asn
   225                 230                 235                 240
   Ala Val Pro Pro Val Ile His Ser Pro Asn Asp His Val Val Tyr Glu
                   245                 250                 255
   Lys Glu Pro Gly Glu Glu Leu Leu Ile Pro Cys Thr Val Tyr Phe Ser
               260                 265                 270
   Phe Leu Met Asp Ser Arg Asn Glu Val Trp Trp Thr Ile Asp Gly Lys
           275                 280                 285
   Lys Pro Asp Asp Ile Thr Ile Asp Val Thr Ile Asn Glu Ser Ile Ser
       290                 295                 300
   His Ser Arg Thr Glu Asp Glu Thr Arg Thr Gln Ile Leu Ser Ile Lys
   305                 310                 315                 320
   Lys Val Thr Ser Glu Asp Leu Lys Arg Ser Tyr Val Cys His Ala Arg
                   325                 330                 335
   Ser Ala Lys Gly Glu Val Ala Lys Ala Ala Lys Val Lys Gln Lys Val
               340                 345                 350
   Pro Ala Pro Arg Tyr Thr Val His Thr Gly Ala Ala Arg Ser Cys Arg
           355                 360                 365
   Phe Arg Gly Arg His Tyr Lys Arg Glu Phe Arg Leu Glu Gly Glu Pro
       370                 375                 380
   Val Ala Leu Arg Cys Pro Gln Val Pro Tyr Trp Leu Trp Ala Ser Val
   385                 390                 395                 400
   Ser Pro Arg Ile Asn Leu Thr Trp His Lys Asn Asp Ser Ala Arg Thr
                   405                 410                 415
   Val Pro Gly Glu Glu Glu Thr Arg Met Trp Ala Gln Asp Gly Ala Leu
               420                 425                 430
   Trp Leu Leu Pro Ala Leu Gln Glu Asp Ser Gly Thr Tyr Val Cys Thr
           435                 440                 445
   Thr Arg Asn Ala Ser Tyr Cys Asp Lys Met Ser Ile Glu Leu Arg Val
       450                 455                 460
   Phe Glu Asn Thr Asp Ala Phe Leu Pro Phe Ile Ser Tyr Pro Gln Ile
   465                 470                 475                 480
   Leu Thr Leu Ser Thr Ser Gly Val Leu Val Cys Pro Asp Leu Ser Glu
                   485                 490                 495
   Phe Thr Arg Asp Lys Thr Asp Val Lys Ile Gln Trp Tyr Lys Asp Ser
               500                 505                 510
   Leu Leu Leu Asp Lys Asp Asn Glu Lys Phe Leu Ser Val Arg Gly Thr
           515                 520                 525
   Thr His Leu Leu Val His Asp Val Ala Leu Glu Asp Ala Gly Tyr Tyr
       530                 535                 540
   Arg Cys Val Leu Thr Phe Ala His Glu Gly Gln Gln Tyr Asn Ile Thr
   545                 550                 555                 560
   Arg Ser Ile Glu Leu Arg Ile Lys Lys Lys Lys Glu Glu Thr Ile Pro
                   565                 570                 575
   Val Ile Ile Ser Pro Leu Lys Thr Ile Ser Ala Ser Leu Gly Ser Arg
               580                 585                 590
   Leu Thr Ile Pro Cys Lys Val Phe Leu Gly Thr Gly Thr Pro Leu Thr
           595                 600                 605
   Thr Met Leu Trp Trp Thr Ala Asn Asp Thr His Ile Glu Ser Ala Tyr
       610                 615                 620
   Pro Gly Gly Arg Val Thr Glu Gly Pro Arg Gln Glu Tyr Ser Glu Asn
   625                 630                 635                 640
   Asn Glu Asn Tyr Ile Glu Val Pro Leu Ile Phe Asp Pro Val Thr Arg
                   645                 650                 655
   Glu Asp Leu His Met Asp Phe Lys Cys Val Val His Asn Thr Leu Ser
               660                 665                 670
   Phe Gln Thr Leu Arg Thr Thr Val Lys Glu Ala Ser Ser Thr Phe Ser
           675                 680                 685
   Gly Asp Lys Thr His Thr Cys Pro Pro Cys Pro Ala Pro Glu Leu Leu
       690                 695                 700
   Gly Gly Pro Ser Val Phe Leu Phe Pro Pro Lys Pro Lys Asp Thr Leu
   705                 710                 715                 720
   Met Ile Ser Arg Thr Pro Glu Val Thr Cys Val Val Val Asp Val Ser
                   725                 730                 735
   His Glu Asp Pro Glu Val Lys Phe Asn Trp Tyr Val Asp Gly Val Glu
               740                 745                 750
   Val His Asn Ala Lys Thr Lys Pro Arg Glu Glu Gln Tyr Asn Ser Thr
           755                 760                 765
   Tyr Arg Val Val Ser Val Leu Thr Val Leu His Gln Asp Trp Leu Asn
       770                 775                 780
   Gly Lys Glu Tyr Lys Cys Lys Val Ser Asn Lys Ala Leu Pro Ala Pro
   785                 790                 795                 800
   Ile Glu Lys Thr Ile Ser Lys Ala Lys Gly Gln Pro Arg Glu Pro Gln
                   805                 810                 815
   Val Tyr Thr Leu Pro Pro Ser Arg Asp Glu Leu Thr Lys Asn Gln Val
               820                 825                 830
   Ser Leu Thr Cys Leu Val Lys Gly Phe Tyr Pro Ser Asp Ile Ala Val
           835                 840                 845
   Glu Trp Glu Ser Asn Gly Gln Pro Glu Asn Asn Tyr Lys Thr Thr Pro
       850                 855                 860
   Pro Val Leu Asp Ser Asp Gly Ser Phe Phe Leu Tyr Ser Lys Leu Thr
   865                 870                 875                 880
   Val Asp Lys Ser Arg Trp Gln Gln Gly Asn Val Phe Ser Cys Ser Val
                   885                 890                 895
   Met His Glu Ala Leu His Asn His Tyr Thr Gln Lys Ser Leu Ser Leu
               900                 905                 910
   Ser Pro Gly Lys
           915
<210> 23
   <211> 2755
   <212> DNA
   <213> Artificial Sequence
<220>
   <223> Synthetic
<400> 23
   datggtgctt ctgtggtgtg tagtgagtct ctacttttat ggaatcctgc aaagtgatgc 60
   ctcagaacgc tgcgatgact ggggactaga caccatgagg caaatccaag tgtttgaaga 120
   tgagccagct cgcatcaagt gcccactctt tgaacacttc ttgaaattca actacagcac 180
   agcccattca gctggcctta ctctgatctg gtattggact aggcaggacc gggaccttga 240
   ggagccaatt aacttccgcc tccccgagaa ccgcattagt aaggagaaag atgtgctgtg 300
   gttccggccc actctcctca atgacactgg caactatacc tgcatgttaa ggaacactac 360
   atattgcagc aaagttgcat ttcccttgga agttgttcaa aaagacagct gtttcaattc 420
   ccccatgaaa ctcccagtgc ataaactgta tatagaatat ggcattcaga ggatcacttg 480
   tccaaatgta gatggatatt ttccttccag tgtcaaaccg actatcactt ggtatatggg 540
   ctgttataaa atacagaatt ttaataatgt aatacccgaa ggtatgaact tgagtttcct 600
   cattgcctta atttcaaata atggaaatta cacatgtgtt gttacatatc cagaaaatgg 660
   acgtacgttt catctcacca ggactctgac tgtaaaggta gtaggctctc caaaaaatgc 720
   agtgccccct gtgatccatt cacctaatga tcatgtggtc tatgagaaag aaccaggaga 780
   ggagctactc attccctgta cggtctattt tagttttctg atggattctc gcaatgaggt 840
   ttggtggacc attgatggaa aaaaacctga tgacatcact attgatgtca ccattaacga 900
   aagtataagt catagtagaa cagaagatga aacaagaact cagattttga gcatcaagaa 960
   agttacctct gaggatctca agcgcagcta tgtctgtcat gctagaagtg ccaaaggcga 1020
   agttgccaaa gcagccaagg tgaagcagaa agtgccagct ccaagataca cagtgcacac 1080
   aggggctgcc agaagctgcc ggtttcgtgg gaggcattac aagcgggagt tcaggctgga 1140
   aggggagcct gtagccctga ggtgccccca ggtgccctac tggttgtggg cctctgtcag 1200
   cccccgcatc aacctgacat ggcataaaaa tgactctgct aggacggtcc caggagaaga 1260
   agagacacgg atgtgggccc aggacggtgc tctgtggctt ctgccagcct tgcaggagga 1320
   ctctggcacc tacgtctgca ctactagaaa tgcttcttac tgtgacaaaa tgtccattga 1380
   gctcagagtt tttgagaata cagatgcttt cctgccgttc atctcatacc cgcaaatttt 1440
   aaccttgtca acctctgggg tattagtatg ccctgacctg agtgaattca cccgtgacaa 1500
   aactgacgtg aagattcaat ggtacaagga ttctcttctt ttggataaag acaatgagaa 1560
   atttctaagt gtgaggggga ccactcactt actcgtacac gatgtggccc tggaagatgc 1620
   tggctattac cgctgtgtcc tgacatttgc ccatgaaggc cagcaataca acatcactag 1680
   gagtattgag ctacgcatca agaaaaaaaa agaagagacc attcctgtga tcatttcccc 1740
   cctcaagacc atatcagctt ctctggggtc aagactgaca atcccatgta aggtgtttct 1800
   gggaaccggc acacccttaa ccaccatgct gtggtggacg gccaatgaca cccacataga 1860
   gagcgcctac ccgggaggcc gcgtgaccga ggggccacgc caggaatatt cagaaaataa 1920
   tgagaactac attgaagtgc cattgatttt tgatcctgtc acaagagagg atttgcacat 1980
   ggattttaaa tgtgttgtcc ataataccct gagttttcag acactacgca ccacagtcaa 2040
   ggaagcctcc tccacgttct ccggagagtc caaatacggt ccgccatgcc catcatgccc 2100
   agcacctgag ttcctggggg gaccatcagt cttcctgttc cccccaaaac ccaaggacac 2160
   tctcatgatc tcccggaccc ctgaggtcac gtgcgtggtg gtggacgtga gccaggaaga 2220
   ccccgaggtc cagttcaact ggtacgtgga tggcgtggag gtgcataatg ccaagacaaa 2280
   gccgcgggag gagcagttca acagcacgta ccgtgtggtc agcgtcctca ccgtcctgca 2340
   ccaggactgg ctgaacggca aggagtacaa gtgcaaggtc tccaacaaag gcctcccgtc 2400
   ctccatcgag aaaaccatct ccaaagccaa agggcagccc cgagagccac aggtgtacac 2460
   cctgccccca tcccaggagg agatgaccaa gaaccaggtc agcctgacct gcctggtcaa 2520
   aggcttctac cccagcgaca tcgccgtgga gtgggagagc aatgggcagc cggagaacaa 2580
   ctacaagacc acgcctcccg tgctggactc cgacggctcc ttcttcctct acagcaggct 2640
   aaccgtggac aagagcaggt ggcaggaggg gaatgtcttc tcatgctccg tgatgcatga 2700
   ggctctgcac aaccactaca cacagaagag cctctccctg tctctgggta aatga      2755
<210> 24
   <211> 918
   <212> PRT
   <213> Artificial Sequence
<220>
   <223> Synthetic
<400> 24
   Pro Met Val Leu Leu Trp Cys Val Val Ser Leu Tyr Phe Tyr Gly Ile
    1              5                   10                  15
   Leu Gln Ser Asp Ala Ser Glu Arg Cys Asp Asp Trp Gly Leu Asp Thr
               20                  25                  30
   Met Arg Gln Ile Gln Val Phe Glu Asp Glu Pro Ala Arg Ile Lys Cys
           35                  40                  45
   Pro Leu Phe Glu His Phe Leu Lys Phe Asn Tyr Ser Thr Ala His Ser
       50                  55                  60
   Ala Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
               100                 105                 110
   Tyr Thr Cys Met Leu Arg Asn Thr Thr Tyr Cys Ser Lys Val Ala Phe
           115                 120                 125
   Pro Leu Glu Val Val Gln Lys Asp Ser Cys Phe Asn Ser Pro Met Lys
       130                 135                 140
   Leu Pro Val His Lys Leu Tyr Ile Glu Tyr Gly Ile Gln Arg Ile Thr
   145                 150                 155                 160
   Cys Pro Asn Val Asp Gly Tyr Phe Pro Ser Ser Val Lys Pro Thr Ile
                   165                 170                 175
   Thr Trp Tyr Met Gly Cys Tyr Lys Ile Gln Asn Phe Asn Asn Val Ile
               180                 185                 190
   Pro Glu Gly Met Asn Leu Ser Phe Leu Ile Ala Leu Ile Ser Asn Asn
           195                 200                 205
   Gly Asn Tyr Thr Cys Val Val Thr Tyr Pro Glu Asn Gly Arg Thr Phe
       210                 215                 220
   His Leu Thr Arg Thr Leu Thr Val Lys Val Val Gly Ser Pro Lys Asn
   225                 230                 235                 240
   Ala Val Pro Pro Val Ile His Ser Pro Asn Asp His Val Val Tyr Glu
                   245                 250                 255
   Lys Glu Pro Gly Glu Glu Leu Leu Ile Pro Cys Thr Val Tyr Phe Ser
               260                 265                 270
   Phe Leu Met Asp Ser Arg Asn Glu Val Trp Trp Thr Ile Asp Gly Lys
           275                 280                 285
   Lys Pro Asp Asp Ile Thr Ile Asp Val Thr Ile Asn Glu Ser Ile Ser
       290                 295                 300
   His Ser Arg Thr Glu Asp Glu Thr Arg Thr Gln Ile Leu Ser Ile Lys
   305                 310                 315                 320
   Lys Val Thr Ser Glu Asp Leu Lys Arg Ser Tyr Val Cys His Ala Arg
                   325                 330                 335
   Ser Ala Lys Gly Glu Val Ala Lys Ala Ala Lys Val Lys Gln Lys Val
               340                 345                 350
   Pro Ala Pro Arg Tyr Thr Val His Thr Gly Ala Ala Arg Ser Cys Arg
           355                 360                 365
   Phe Arg Gly Arg His Tyr Lys Arg Glu Phe Arg Leu Glu Gly Glu Pro
       370                 375                 380
   Val Ala Leu Arg Cys Pro Gln Val Pro Tyr Trp Leu Trp Ala Ser Val
   385                 390                 395                 400
   Ser Pro Arg Ile Asn Leu Thr Trp His Lys Asn Asp Ser Ala Arg Thr
                   405                 410                 415
   Val Pro Gly Glu Glu Glu Thr Arg Met Trp Ala Gln Asp Gly Ala Leu
               420                 425                 430
   Trp Leu Leu Pro Ala Leu Gln Glu Asp Ser Gly Thr Tyr Val Cys Thr
           435                 440                 445
   Thr Arg Asn Ala Ser Tyr Cys Asp Lys Met Ser Ile Glu Leu Arg Val
       450                 455                 460
   Phe Glu Asn Thr Asp Ala Phe Leu Pro Phe Ile Ser Tyr Pro Gln Ile
   465                 470                 475                 480
   Leu Thr Leu Ser Thr Ser Gly Val Leu Val Cys Pro Asp Leu Ser Glu
                   485                 490                 495
   Phe Thr Arg Asp Lys Thr Asp Val Lys Ile Gln Trp Tyr Lys Asp Ser
               500                 505                 510
   Leu Leu Leu Asp Lys Asp Asn Glu Lys Phe Leu Ser Val Arg Gly Thr
           515                 520                 525
   Thr His Leu Leu Val His Asp Val Ala Leu Glu Asp Ala Gly Tyr Tyr
       530                 535                 540
   Arg Cys Val Leu Thr Phe Ala His Glu Gly Gln Gln Tyr Asn Ile Thr
   545                 550                 555                 560
   Arg Ser Ile Glu Leu Arg Ile Lys Lys Lys Lys Glu Glu Thr Ile Pro
                   565                 570                 575
   Val Ile Ile Ser Pro Leu Lys Thr Ile Ser Ala Ser Leu Gly Ser Arg
               580                 585                 590
   Leu Thr Ile Pro Cys Lys Val Phe Leu Gly Thr Gly Thr Pro Leu Thr
           595                 600                 605
   Thr Met Leu Trp Trp Thr Ala Asn Asp Thr His Ile Glu Ser Ala Tyr
       610                 615                 620
   Pro Gly Gly Arg Val Thr Glu Gly Pro Arg Gln Glu Tyr Ser Glu Asn
   625                 630                 635                 640
   Asn Glu Asn Tyr Ile Glu Val Pro Leu Ile Phe Asp Pro Val Thr Arg
                   645                 650                 655
   Glu Asp Leu His Met Asp Phe Lys Cys Val Val His Asn Thr Leu Ser
               660                 665                 670
   Phe Gln Thr Leu Arg Thr Thr Val Lys Glu Ala Ser Ser Thr Phe Ser
           675                 680                 685
   Gly Glu Ser Lys Tyr Gly Pro Pro Cys Pro Ser Cys Pro Ala Pro Glu
       690                 695                 700
   Phe Leu Gly Gly Pro Ser Val Phe Leu Phe Pro Pro Lys Pro Lys Asp
   705                 710                 715                 720
   Thr Leu Met Ile Ser Arg Thr Pro Glu Val Thr Cys Val Val Val Asp
                   725                 730                 735
   Val Ser Gln Glu Asp Pro Glu Val Gln Phe Asn Trp Tyr Val Asp Gly
               740                 745                 750
   Val Glu Val His Asn Ala Lys Thr Lys Pro Arg Glu Glu Gln Phe Asn
           755                 760                 765
   Ser Thr Tyr Arg Val Val Ser Val Leu Thr Val Leu His Gln Asp Trp
       770                 775                 780
   Leu Asn Gly Lys Glu Tyr Lys Cys Lys Val Ser Asn Lys Gly Leu Pro
   785                 790                 795                 800
   Ser Ser Ile Glu Lys Thr Ile Ser Lys Ala Lys Gly Gln Pro Arg Glu
                   805                 810                 815
   Pro Gln Val Tyr Thr Leu Pro Pro Ser Gln Glu Glu Met Thr Lys Asn
               820                 825                 830
   Gln Val Ser Leu Thr Cys Leu Val Lys Gly Phe Tyr Pro Ser Asp Ile
           835                 840                 845
   Ala Val Glu Trp Glu Ser Asn Gly Gln Pro Glu Asn Asn Tyr Lys Thr
       850                 855                 860
   Thr Pro Pro Val Leu Asp Ser Asp Gly Ser Phe Phe Leu Tyr Ser Arg
   865                 870                 875                 880
   Leu Thr Val Asp Lys Ser Arg Trp Gln Glu Gly Asn Val Phe Ser Cys
                   885                 890                 895
   Ser Val Met His Glu Ala Leu His Asn His Tyr Thr Gln Lys Ser Leu
               900                 905                 910
   Ser Leu Ser Leu Gly Lys
           915
<210> 25
   <211> 2755
   <212> DNA
   <213> Artificial Sequence
<220>
   <223> Synthetic
<400> 25
   datggtgctt ctgtggtgtg tagtgagtct ctacttttat ggaatcctgc aaagtgatgc 60
   ctcagaacgc tgcgatgact ggggactaga caccatgagg caaatccaag tgtttgaaga 120
   tgagccagct cgcatcaagt gcccactctt tgaacacttc ttgaaattca actacagcac 180
   agcccattca gctggcctta ctctgatctg gtattggact aggcaggacc gggaccttga 240
   ggagccaatt aacttccgcc tccccgagaa ccgcattagt aaggagaaag atgtgctgtg 300
   gttccggccc actctcctca atgacactgg caactatacc tgcatgttaa ggaacactac 360
   atattgcagc aaagttgcat ttcccttgga agttgttcaa aaagacagct gtttcaattc 420
   ccccatgaaa ctcccagtgc ataaactgta tatagaatat ggcattcaga ggatcacttg 480
   tccaaatgta gatggatatt ttccttccag tgtcaaaccg actatcactt ggtatatggg 540
   ctgttataaa atacagaatt ttaataatgt aatacccgaa ggtatgaact tgagtttcct 600
   cattgcctta atttcaaata atggaaatta cacatgtgtt gttacatatc cagaaaatgg 660
   acgtacgttt catctcacca ggactctgac tgtaaaggta gtaggctctc caaaaaatgc 720
   agtgccccct gtgatccatt cacctaatga tcatgtggtc tatgagaaag aaccaggaga 780
   ggagctactc attccctgta cggtctattt tagttttctg atggattctc gcaatgaggt 840
   ttggtggacc attgatggaa aaaaacctga tgacatcact attgatgtca ccattaacga 900
   aagtataagt catagtagaa cagaagatga aacaagaact cagattttga gcatcaagaa 960
   agttacctct gaggatctca agcgcagcta tgtctgtcat gctagaagtg ccaaaggcga 1020
   agttgccaaa gcagccaagg tgaagcagaa agtgccagct ccaagataca cagtgcacac 1080
   aggggctgcc agaagctgcc ggtttcgtgg gaggcattac aagcgggagt tcaggctgga 1140
   aggggagcct gtagccctga ggtgccccca ggtgccctac tggttgtggg cctctgtcag 1200
   cccccgcatc aacctgacat ggcataaaaa tgactctgct aggacggtcc caggagaaga 1260
   agagacacgg atgtgggccc aggacggtgc tctgtggctt ctgccagcct tgcaggagga 1320
   ctctggcacc tacgtctgca ctactagaaa tgcttcttac tgtgacaaaa tgtccattga 1380
   gctcagagtt tttgagaata cagatgcttt cctgccgttc atctcatacc cgcaaatttt 1440
   aaccttgtca acctctgggg tattagtatg ccctgacctg agtgaattca cccgtgacaa 1500
   aactgacgtg aagattcaat ggtacaagga ttctcttctt ttggataaag acaatgagaa 1560
   atttctaagt gtgaggggga ccactcactt actcgtacac gatgtggccc tggaagatgc 1620
   tggctattac cgctgtgtcc tgacatttgc ccatgaaggc cagcaataca acatcactag 1680
   gagtattgag ctacgcatca agaaaaaaaa agaagagacc attcctgtga tcatttcccc 1740
   cctcaagacc atatcagctt ctctggggtc aagactgaca atcccatgta aggtgtttct 1800
   gggaaccggc acacccttaa ccaccatgct gtggtggacg gccaatgaca cccacataga 1860
   gagcgcctac ccgggaggcc gcgtgaccga ggggccacgc caggaatatt cagaaaataa 1920
   tgagaactac attgaagtgc cattgatttt tgatcctgtc acaagagagg atttgcacat 1980
   ggattttaaa tgtgttgtcc ataataccct gagttttcag acactacgca ccacagtcaa 2040
   ggaagcctcc tccacgttct ccggagagtc caaatacggt ccgccatgcc caccatgccc 2100
   agcacctgag ttcctggggg gaccatcagt cttcctgttc cccccaaaac ccaaggacac 2160
   tctcatgatc tcccggaccc ctgaggtcac gtgcgtggtg gtggacgtga gccaggaaga 2220
   ccccgaggtc cagttcaact ggtacgtgga tggcgtggag gtgcataatg ccaagacaaa 2280
   gccgcgggag gagcagttca acagcacgta ccgtgtggtc agcgtcctca ccgtcctgca 2340
   ccaggactgg ctgaacggca aggagtacaa gtgcaaggtc tccaacaaag gcctcccgtc 2400
   ctccatcgag aaaaccatct ccaaagccaa agggcagccc cgagagccac aggtgtacac 2460
   cctgccccca tcccaggagg agatgaccaa gaaccaggtc agcctgacct gcctggtcaa 2520
   aggcttctac cccagcgaca tcgccgtgga gtgggagagc aatgggcagc cggagaacaa 2580
   ctacaagacc acgcctcccg tgctggactc cgacggctcc ttcttcctct acagcaggct 2640
   aaccgtggac aagagcaggt ggcaggaggg gaatgtcttc tcatgctccg tgatgcatga 2700
   ggctctgcac aaccactaca cacagaagag cctctccctg tctctgggta aatga      2755
<210> 26
   <211> 918
   <212> PRT
   <213> Artificial Sequence
<220>
   <223> Synthetic
<400> 26
   Pro Met Val Leu Leu Trp Cys Val Val Ser Leu Tyr Phe Tyr Gly Ile
   1               5                   10                  15
   Leu Gln Ser Asp Ala Ser Glu Arg Cys Asp Asp Trp Gly Leu Asp Thr
               20                  25                  30
   Met Arg Gln Ile Gln Val Phe Glu Asp Glu Pro Ala Arg Ile Lys Cys
           35                  40                  45
   Pro Leu Phe Glu His Phe Leu Lys Phe Asn Tyr Ser Thr Ala His Ser
       50                  55                  60
   Ala Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
               100                 105                 110
   Tyr Thr Cys Met Leu Arg Asn Thr Thr Tyr Cys Ser Lys Val Ala Phe
           115                 120                 125
   Pro Leu Glu Val Val Gln Lys Asp Ser Cys Phe Asn Ser Pro Met Lys
       130                 135                 140
   Leu Pro Val His Lys Leu Tyr Ile Glu Tyr Gly Ile Gln Arg Ile Thr
   145                 150                 155                 160
   Cys Pro Asn Val Asp Gly Tyr Phe Pro Ser Ser Val Lys Pro Thr Ile
                   165                 170                 175
   Thr Trp Tyr Met Gly Cys Tyr Lys Ile Gln Asn Phe Asn Asn Val Ile
               180                 185                 190
   Pro Glu Gly Met Asn Leu Ser Phe Leu Ile Ala Leu Ile Ser Asn Asn
           195                 200                 205
   Gly Asn Tyr Thr Cys Val Val Thr Tyr Pro Glu Asn Gly Arg Thr Phe
       210                 215                 220
   His Leu Thr Arg Thr Leu Thr Val Lys Val Val Gly Ser Pro Lys Asn
   225                 230                 235                 240
   Ala Val Pro Pro Val Ile His Ser Pro Asn Asp His Val Val Tyr Glu
                   245                 250                 255
   Lys Glu Pro Gly Glu Glu Leu Leu Ile Pro Cys Thr Val Tyr Phe Ser
               260                 265                 270
   Phe Leu Met Asp Ser Arg Asn Glu Val Trp Trp Thr Ile Asp Gly Lys
           275                 280                 285
   Lys Pro Asp Asp Ile Thr Ile Asp Val Thr Ile Asn Glu Ser Ile Ser
       290                 295                 300
   His Ser Arg Thr Glu Asp Glu Thr Arg Thr Gln Ile Leu Ser Ile Lys
   305                 310                 315                 320
   Lys Val Thr Ser Glu Asp Leu Lys Arg Ser Tyr Val Cys His Ala Arg
                   325                 330                 335
   Ser Ala Lys Gly Glu Val Ala Lys Ala Ala Lys Val Lys Gln Lys Val
               340                 345                 350
   Pro Ala Pro Arg Tyr Thr Val His Thr Gly Ala Ala Arg Ser Cys Arg
           355                 360                 365
   Phe Arg Gly Arg His Tyr Lys Arg Glu Phe Arg Leu Glu Gly Glu Pro
       370                 375                 380
   Val Ala Leu Arg Cys Pro Gln Val Pro Tyr Trp Leu Trp Ala Ser Val
   385                 390                 395                 400
   Ser Pro Arg Ile Asn Leu Thr Trp His Lys Asn Asp Ser Ala Arg Thr
                   405                 410                 415
   Val Pro Gly Glu Glu Glu Thr Arg Met Trp Ala Gln Asp Gly Ala Leu
                   420                 425                 430
   Trp Leu Leu Pro Ala Leu Gln Glu Asp Ser Gly Thr Tyr Val Cys Thr
               435                 440                 445
   Thr Arg Asn Ala Ser Tyr Cys Asp Lys Met Ser Ile Glu Leu Arg Val
           450                 455                 460
   Phe Glu Asn Thr Asp Ala Phe Leu Pro Phe Ile Ser Tyr Pro Gln Ile
   465                 470                 475                 480
   Leu Thr Leu Ser Thr Ser Gly Val Leu Val Cys Pro Asp Leu Ser Glu
                   485                 490                 495
   Phe Thr Arg Asp Lys Thr Asp Val Lys Ile Gln Trp Tyr Lys Asp Ser
                   500                 505                 510
   Leu Leu Leu Asp Lys Asp Asn Glu Lys Phe Leu Ser Val Arg Gly Thr
               515                 520                 525
   Thr His Leu Leu Val His Asp Val Ala Leu Glu Asp Ala Gly Tyr Tyr
           530                 535                 540
   Arg Cys Val Leu Thr Phe Ala His Glu Gly Gln Gln Tyr Asn Ile Thr
   545                 550                 555                 560
   Arg Ser Ile Glu Leu Arg Ile Lys Lys Lys Lys Glu Glu Thr Ile Pro
                   565                 570                 575
   Val Ile Ile Ser Pro Leu Lys Thr Ile Ser Ala Ser Leu Gly Ser Arg
                   580                 585                 590
   Leu Thr Ile Pro Cys Lys Val Phe Leu Gly Thr Gly Thr Pro Leu Thr
               595                 600                 605
   Thr Met Leu Trp Trp Thr Ala Asn Asp Thr His Ile Glu Ser Ala Tyr
           610                 615                 620
   Pro Gly Gly Arg Val Thr Glu Gly Pro Arg Gln Glu Tyr Ser Glu Asn
   625                 630                 635                 640
   Asn Glu Asn Tyr Ile Glu Val Pro Leu Ile Phe Asp Pro Val Thr Arg
                   645                 650                 655
   Glu Asp Leu His Met Asp Phe Lys Cys Val Val His Asn Thr Leu Ser
                   660                 665                 670
   Phe Gln Thr Leu Arg Thr Thr Val Lys Glu Ala Ser Ser Thr Phe Ser
               675                 680                 685
   Gly Glu Ser Lys Tyr Gly Pro Pro Cys Pro Pro Cys Pro Ala Pro Glu
           690                 695                 700
   Phe Leu Gly Gly Pro Ser Val Phe Leu Phe Pro Pro Lys Pro Lys Asp
   705                 710                 715                 720
   Thr Leu Met Ile Ser Arg Thr Pro Glu Val Thr Cys Val Val Val Asp
                   725                 730                 735
   Val Ser Gln Glu Asp Pro Glu Val Gln Phe Asn Trp Tyr Val Asp Gly
                   740                 745                 750
   Val Glu Val His Asn Ala Lys Thr Lys Pro Arg Glu Glu Gln Phe Asn
               755                 760                 765
   Ser Thr Tyr Arg Val Val Ser Val Leu Thr Val Leu His Gln Asp Trp
           770                 775                 780
   Leu Asn Gly Lys Glu Tyr Lys Cys Lys Val Ser Asn Lys Gly Leu Pro
   785                 790                 795                 800
   Ser Ser Ile Glu Lys Thr Ile Ser Lys Ala Lys Gly Gln Pro Arg Glu
                   805                 810                 815
   Pro Gln Val Tyr Thr Leu Pro Pro Ser Gln Glu Glu Met Thr Lys Asn
                   820                 825                 830
   Gln Val Ser Leu Thr Cys Leu Val Lys Gly Phe Tyr Pro Ser Asp Ile
               835                 840                 845
   Ala Val Glu Trp Glu Ser Asn Gly Gln Pro Glu Asn Asn Tyr Lys Thr
           850                 855                 860
   Thr Pro Pro Val Leu Asp Ser Asp Gly Ser Phe Phe Leu Tyr Ser Arg
   865                 870                 875                 880
   Leu Thr Val Asp Lys Ser Arg Trp Gln Glu Gly Asn Val Phe Ser Cys
                   885                 890                 895
   Ser Val Met His Glu Ala Leu His Asn His Tyr Thr Gln Lys Ser Leu
               900                 905                 910
   Ser Leu Ser Leu Gly Lys
           915
<210> 27
   <211> 2734
   <212> DNA
   <213> Artificial Sequence
<220>
   <223> Synthetic
<400> 27
   datggtgctt ctgtggtatt tgatgagtct gtccttctat gggatcctgc agagtcatgc 60
   ttcggagcgc tgtgatgact ggggactaga taccatgcga caaatccaag tgtttgaaga 120
   tgagccggct cgaatcaagt gccccctctt tgaacacttc ctgaagtaca actacagcac 180
   tgcccattcc tctggcctta ccctgatctg gtactggacc aggcaagacc gggacctgga 240
   ggagcccatt aacttccgcc tcccagagaa tcgcatcagt aaggagaaag atgtgctctg 300
   gttccggccc accctcctca atgacacggg caattacacc tgcatgttga ggaacacaac 360
   ttactgcagc aaagttgcat ttcccctgga agttgttcag aaggacagct gtttcaattc 420
   tgccatgaga ttcccagtgc acaagatgta tattgaacat ggcattcata agatcacatg 480
   tccaaatgta gacggatact ttccttccag tgtcaaacca tcggtcactt ggtataaggg 540
   ttgtactgaa atagtggact ttcataatgt actacccgag ggcatgaact tgagcttttt 600
   catccccttg gtttcaaata acggaaatta cacatgtgtg gttacatatc ctgaaaacgg 660
   acgtctcttt cacctcacca ggactgtgac tgtaaaggtg gtgggctcac caaaggatgc 720
   attgccaccc cagatctatt ctccaaatga ccgtgttgtc tatgagaaag aaccaggaga 780
   ggaactggtt attccctgca aagtctattt cagtttcatt atggactccc acaatgaggt 840
   ctggtggacc attgatggaa agaagcctga tgacgtcaca gtcgacatca ctattaatga 900
   aagtgtaagt tattcttcaa cggaagatga aacaaggact cagattttga gcatcaagaa 960
   agtcaccccg gaggatctca ggcgcaacta tgtctgtcat gctcgaaata ccaaagggga 1020
   agctgagcag gctgccaagg tgaaacagaa agtcatacca ccaaggtaca cagtactgga 1080
   gattgacgta tgtacagaat atccaaatca gatcgttttg tttttatctg taaatgaaat 1140
   tgatattcgc aagtgtcctc ttactccaaa taaaatgcac ggcgacacca taatttggta 1200
   caagaatgac agcaagaccc ccatatcagc ggaccgggac tccaggattc atcagcagaa 1260
   tgaacatctt tggtttgtac ctgccaaggt ggaggactca ggatattact attgtatagt 1320
   aagaaactca acttactgcc tcaaaactaa agtaaccgta actgtgttag agaatgaccc 1380
   tggcttgtgt tacagcacac aggccacctt cccacagcgg ctccacattg ccggggatgg 1440
   aagtcttgtg tgcccttatg tgagttattt taaagatgaa aataatgagt tacccgaggt 1500
   ccagtggtat aagaactgta aacctctgct tcttgacaac gtgagcttct tcggagtaaa 1560
   agataaactg ttggtgagga atgtggctga agagcacaga ggggactata tatgccgtat 1620
   gtcctatacg ttccggggga agcaatatcc ggtcacacga gtaatacaat ttatcacaat 1680
   agatgaaaac aagagggaca gacctgttat cctgagccct cggaatgaga cgatcgaagc 1740
   tgacccagga tcaatgatac aactgatctg caacgtcacg ggccagttct cagaccttgt 1800
   ctactggaag tggaatggat cagaaattga atggaatgat ccatttctag ctgaagacta 1860
   tcaatttgtg gaacatcctt caaccaaaag aaaatacaca ctcattacaa cacttaacat 1920
   ttcagaagtt aaaagccagt tttatcgcta tccgtttatc tgtgttgtta agaacacaaa 1980
   tatttttgag tcggcgcatg tgcagttaat atacccagtc cctggcccgg gcgagcccag 2040
   agggcccaca atcaagccct gtcctccatg caaatgccca gcacctaacc tcttgggtgg 2100
   accatccgtc ttcatcttcc ctccaaagat caaggatgta ctcatgatct ccctgagccc 2160
   catagtcaca tgtgtggtgg tggatgtgag cgaggatgac ccagatgtcc agatcagctg 2220
   gtttgtgaac aacgtggaag tacacacagc tcagacacaa acccatagag aggattacaa 2280
   cagtactctc cgggtggtca gtgccctccc catccagcac caggactgga tgagtggcaa 2340
   ggagttcaaa tgcaaggtca acaacaaaga cctcccagcg cccatcgaga gaaccatctc 2400
   aaaacccaaa gggtcagtaa gagctccaca ggtatatgtc ttgcctccac cagaagaaga 2460
   gatgactaag aaacaggtca ctctgacctg catggtcaca gacttcatgc ctgaagacat 2520
   ttacgtggag tggaccaaca acgggaaaac agagctaaac tacaagaaca ctgaaccagt 2580
   cctggactct gatggttctt acttcatgta cagcaagctg agagtggaaa agaagaactg 2640
   ggtggaaaga aatagctact cctgttcagt ggtccacgag ggtctgcaca atcaccacac 2700
   gactaagagc ttctcccgga ctccgggtaa atga                             2734
<210> 28
   <211> 911
   <212> PRT
   <213> Artificial Sequence
<220>
   <223> Synthetic
<400> 28
   Pro Met Val Leu Leu Trp Tyr Leu Met Ser Leu Ser Phe Tyr Gly Ile
    1               5                  10                  15
   Leu Gln Ser His Ala Ser Glu Arg Cys Asp Asp Trp Gly Leu Asp Thr
               20                  25                  30
   Met Arg Gln Ile Gln Val Phe Glu Asp Glu Pro Ala Arg Ile Lys Cys
           35                  40                  45
   Pro Leu Phe Glu His Phe Leu Lys Tyr Asn Tyr Ser Thr Ala His Ser
       50                  55                  60
   Ser Gly Leu Thr Leu Ile Trp Tyr Trp Thr Arg Gln Asp Arg Asp Leu
   65                  70                  75                  80
   Glu Glu Pro Ile Asn Phe Arg Leu Pro Glu Asn Arg Ile Ser Lys Glu
                   85                  90                  95
   Lys Asp Val Leu Trp Phe Arg Pro Thr Leu Leu Asn Asp Thr Gly Asn
               100                 105                 110
   Tyr Thr Cys Met Leu Arg Asn Thr Thr Tyr Cys Ser Lys Val Ala Phe
           115                 120                 125
   Pro Leu Glu Val Val Gln Lys Asp Ser Cys Phe Asn Ser Ala Met Arg
       130                 135                 140
   Phe Pro Val His Lys Met Tyr Ile Glu His Gly Ile His Lys Ile Thr
   145                 150                 155                 160
   Cys Pro Asn Val Asp Gly Tyr Phe Pro Ser Ser Val Lys Pro Ser Val
                   165                 170                 175
   Thr Trp Tyr Lys Gly Cys Thr Glu Ile Val Asp Phe His Asn Val Leu
               180                 185                 190
   Pro Glu Gly Met Asn Leu Ser Phe Phe Ile Pro Leu Val Ser Asn Asn
           195                 200                 205
   Gly Asn Tyr Thr Cys Val Val Thr Tyr Pro Glu Asn Gly Arg Leu Phe
       210                 215                 220
   His Leu Thr Arg Thr Val Thr Val Lys Val Val Gly Ser Pro Lys Asp
   225                 230                 235                 240
   Ala Leu Pro Pro Gln Ile Tyr Ser Pro Asn Asp Arg Val Val Tyr Glu
                   245                 250                 255
   Lys Glu Pro Gly Glu Glu Leu Val Ile Pro Cys Lys Val Tyr Phe Ser
               260                 265                 270
   Phe Ile Met Asp Ser His Asn Glu Val Trp Trp Thr Ile Asp Gly Lys
           275                 280                 285
   Lys Pro Asp Asp Val Thr Val Asp Ile Thr Ile Asn Glu Ser Val Ser
       290                 295                 300
   Tyr Ser Ser Thr Glu Asp Glu Thr Arg Thr Gln Ile Leu Ser Ile Lys
   305                 310                 315                 320
   Lys Val Thr Pro Glu Asp Leu Arg Arg Asn Tyr Val Cys His Ala Arg
                   325                 330                 335
   Asn Thr Lys Gly Glu Ala Glu Gln Ala Ala Lys Val Lys Gln Lys Val
               340                 345                 350
   Ile Pro Pro Arg Tyr Thr Val Leu Glu Ile Asp Val Cys Thr Glu Tyr
           355                 360                 365
   Pro Asn Gln Ile Val Leu Phe Leu Ser Val Asn Glu Ile Asp Ile Arg
       370                 375                 380
   Lys Cys Pro Leu Thr Pro Asn Lys Met His Gly Asp Thr Ile Ile Trp
   385                 390                 395                 400
   Tyr Lys Asn Asp Ser Lys Thr Pro Ile Ser Ala Asp Arg Asp Ser Arg
                   405                 410                 415
   Ile His Gln Gln Asn Glu His Leu Trp Phe Val Pro Ala Lys Val Glu
               420                 425                 430
   Asp Ser Gly Tyr Tyr Tyr Cys Ile Val Arg Asn Ser Thr Tyr Cys Leu
           435                 440                 445
   Lys Thr Lys Val Thr Val Thr Val Leu Glu Asn Asp Pro Gly Leu Cys
       450                 455                 460
   Tyr Ser Thr Gln Ala Thr Phe Pro Gln Arg Leu His Ile Ala Gly Asp
   465                 470                 475                 480
   Gly Ser Leu Val Cys Pro Tyr Val Ser Tyr Phe Lys Asp Glu Asn Asn
                   485                 490                 495
   Glu Leu Pro Glu Val Gln Trp Tyr Lys Asn Cys Lys Pro Leu Leu Leu
               500                 505                 510
   Asp Asn Val Ser Phe Phe Gly Val Lys Asp Lys Leu Leu Val Arg Asn
           515                 520                 525
   Val Ala Glu Glu His Arg Gly Asp Tyr Ile Cys Arg Met Ser Tyr Thr
       530                 535                 540
   Phe Arg Gly Lys Gln Tyr Pro Val Thr Arg Val Ile Gln Phe Ile Thr
   545                 550                 555                 560
   Ile Asp Glu Asn Lys Arg Asp Arg Pro Val Ile Leu Ser Pro Arg Asn
                   565                 570                 575
   Glu Thr Ile Glu Ala Asp Pro Gly Ser Met Ile Gln Leu Ile Cys Asn
               580                 585                 590
   Val Thr Gly Gln Phe Ser Asp Leu Val Tyr Trp Lys Trp Asn Gly Ser
           595                 600                 605
   Glu Ile Glu Trp Asn Asp Pro Phe Leu Ala Glu Asp Tyr Gln Phe Val
       610                 615                 620
   Glu His Pro Ser Thr Lys Arg Lys Tyr Thr Leu Ile Thr Thr Leu Asn
   625                 630                 635                 640
   Ile Ser Glu Val Lys Ser Gln Phe Tyr Arg Tyr Pro Phe Ile Cys Val
                   645                 650                 655
   Val Lys Asn Thr Asn Ile Phe Glu Ser Ala His Val Gln Leu Ile Tyr
               660                 665                 670
   Pro Val Pro Gly Pro Gly Glu Pro Arg Gly Pro Thr Ile Lys Pro Cys
           675                 680                 685
   Pro Pro Cys Lys Cys Pro Ala Pro Asn Leu Leu Gly Gly Pro Ser Val
       690                 695                 700
   Phe Ile Phe Pro Pro Lys Ile Lys Asp Val Leu Met Ile Ser Leu Ser
   705                 710                 715                 720
   Pro Ile Val Thr Cys Val Val Val Asp Val Ser Glu Asp Asp Pro Asp
                   725                 730                 735
   Val Gln Ile Ser Trp Phe Val Asn Asn Val Glu Val His Thr Ala Gln
               740                 745                 750
   Thr Gln Thr His Arg Glu Asp Tyr Asn Ser Thr Leu Arg Val Val Ser
           755                 760                 765
   Ala Leu Pro Ile Gln His Gln Asp Trp Met Ser Gly Lys Glu Phe Lys
       770                 775                 780
   Cys Lys Val Asn Asn Lys Asp Leu Pro Ala Pro Ile Glu Arg Thr Ile
   785                 790                 795                 800
   Ser Lys Pro Lys Gly Ser Val Arg Ala Pro Gln Val Tyr Val Leu Pro
                   805                 810                 815
   Pro Pro Glu Glu Glu Met Thr Lys Lys Gln Val Thr Leu Thr Cys Met
               820                 825                 830
   Val Thr Asp Phe Met Pro Glu Asp Ile Tyr Val Glu Trp Thr Asn Asn
           835                 840                 845
   Gly Lys Thr Glu Leu Asn Tyr Lys Asn Thr Glu Pro Val Leu Asp Ser
       850                 855                 860
   Asp Gly Ser Tyr Phe Met Tyr Ser Lys Leu Arg Val Glu Lys Lys Asn
   865                 870                 875                 880
   Trp Val Glu Arg Asn Ser Tyr Ser Cys Ser Val Val His Glu Gly Leu
                   885                 890                 895
   His Asn His His Thr Thr Lys Ser Phe Ser Arg Thr Pro Gly Lys
               900                 905                 910